# EUROPÄISCHE PATENTSCHRIFT

(11) **EP 1 971 580 B1**
(45) Veröffentlichungstag und Bekanntmachung des Hinweises auf die Patenterteilung: **24.02.2010**
(21) Anmeldenummer: 06841650.2
(22) Anmeldetag: 29.12.2006
(51) Int. Cl.: C07D 241/08, C07D 401/06, C07D 403/06, C07D 409/06, C07D 409/12, C07D 413/06, C07D 417/06, C07D 417/12, C07F 5/02, C07F 7/10, C07F 7/18, C07F 9/6509, C07K 5/065, A01N 37/46

(54) **PIPERAZINVERBINDUNGEN MIT HERBIZIDER WIRKUNG**
PIPERAZINE COMPOUNDS WITH A HERBICIDAL ACTION
COMPOSES A BASE DE PIPERAZINE A ACTION HERBICIDE

(30) Priorität: 02.01.2006 EP 06000013
(43) Veröffentlichungstag der Anmeldung: 24.09.2008
(73) Patentinhaber: BASF SE, 67056 Ludwigshafen (DE)
(72) Erfinder: HUPE, Eike, 67067 Ludwigshafen (DE); ZAGAR, Cyrill, Kowloon/Hong Kong (CN); WITSCHEL, Matthias, 67098 Bad Dürkheim (DE); KÜHN, Toralf, 68199 Mannheim (DE); MOBERG, William Karl, 67454 Hassloch (DE); PARRA RAPADO, Liliana, 77654 Offenburg (DE); STELZER, Frank, 68309 Mannheim (DE); VESCOVI, Andrea, 68167 Mannheim (DE); PUHL, Michael, 68623 Lampertheim (DE); REINHARD, Robert, 67117 Limburgerhof (DE); SIEVERNICH, Bernd, 67454 Hassloch (DE); GROSSMANN, Klaus, 67141 Neuhofen (DE); EHRHARDT, Thomas, 67346 Speyer (DE); RACK, Michael, 69214 Eppelheim (DE)
(74) Vertreter: Reitstötter - Kinzebach
(86) Internationale Anmeldenummer: PCT/EP2006/070271
(87) Internationale Veröffentlichungsnummer: WO 2007/077201

(56) Entgegenhaltungen:
- RUSSELL R. KING, C. HAROLD LAWRENCE: "Herbicidal Properties of the Thaxtomin Group of Phytotoxins" J. AGRIC. FOOD CHEM., Bd. 49, 2001, Seiten 2298-2301, XP002370714 in der Anmeldung erwähnt
- BALBONI G., GUERRINI R., SALVADORI S., TOMATIS R., BRYANT S. D., BIANCHI C., ATTILA M., LAZARUS L.: "Opioid diketopiperazines. Synthesis and activity of a prototypic class of opioid antagonist" BIOLOGICAL CHEMISTRY, Bd. 378, Nr. 1, 1997, Seiten 19-29, XP008061086
- DATABASE CA [Online] CHEMICAL ABSTRACTS SERVICE, COLUMBUS, OHIO, US; LI, YONGQUAN ET AL: "Bio-manufacture of cyclo-dipeptide compounds as weedicides" XP002370723 gefunden im STN Database accession no. 2005:626272 & CN 1 557 961 CN (ZHEJIANG UNIVERSITY, PEOP. REP. CHINA) Januar 2004 (2004-01)

## Beschreibung

Die vorliegende Erfindung betrifft die Verwendung von Piperazinverbindungen der Formel I 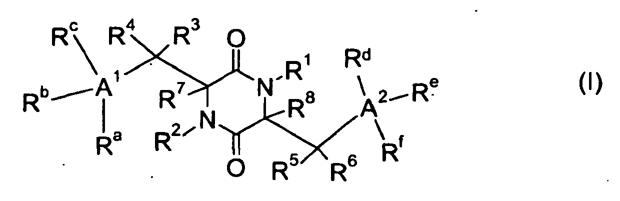 oder der landwirtschaftlich brauchbaren Salze von Piperazinverbindungen der Formel I als Herbizide, wobei in Formel I die Variablen die folgenden Bedeutungen haben:
R¹ und R² unabhängig voneinander:
Cyano, C₁-C₆-Alkyl, C₃-C₆-Cycloalkyl, C₃-C₆-Alkenyl, C₃-C₆-Cycloalkenyl, C₃-C₆-Alkinyl, C₃-C₆-Cycloalkinyl, Phenyl, Phenyl-(C₁-C₆)-Alkyl, Heterocyclyl, Heterocyclyl-(C₁-C₆)-Alkyl; Phenyl-[C₁-C₆-alkoxycarbonyl]-(C₁-C₆)-alkyl oder Phenylheterocyclyl-(C₁-C₆)-alkyl; oder
COR²¹, wobei
   R²¹ Wasserstoff, C₁-C₆-Alkyl, C₃-C₆-Cycloalkyl, C₂-C₆-Alkenyl, C₃-C₆-Cycloalkenyl, C₂-C₆-Alkinyl, C₃-C₆-Cycloalkinyl, Hydroxy, C₁-C₆-Alkoxy, C₃-C₆-Alkenyloxy, C₃-C₆-Alkinyloxy, Amino, C₁-C₆-Alkylamino, [Di-(C₁-C₆)-alkyl]amino, C₃-C₆-Alkenylamino, C₃-C₆-Alkinylamino, C₁-C₆-Alkylsulfonylaminb, N-(C₂-C₆-Alkenyl)-N-(C₁-C₆-alkyl)-amino, N-(C₂-C₆-Alkinyl)-N-(C₁-C₆-alkyl)-amino, N-(C₁-C₆-Alkoxy)-N-(C₁-C₆-alkyl)-amino, N-(C₂-C₆-Alkenyl)-N-(C₁-C₆-alkoxy)-amino, N-(C₂-C₆-Alkinyl)-N-(C₁-C₆-alkoxy)-amino, Phenyl, Phenylamino, Phenoxy, Naphthyl oder Heterocyclyl bedeutet; oder
NR²²R²³, wobei
   R²² und R²³ unabhängig voneinander Wasserstoff, C₁-C₆-Alkyl, C₃-C₆-Cycloalkyl, C₃-C₆-Alkenyl, C₃-C₆-Cycloalkenyl, C₃-C₆-Alkinyl, C₃-C₆-Cycloalkinyl oder C₁-C₆-Alkylcarbonyl bedeuten; oder R²⁴ C₁-C₆-Alkyl, C₃-C₆-Cycloalkyl, C₃-C₆-Alkenyl, C₃-C₆-Cycloalkenyl, C₃-C₆-Alkinyl, C₃-C₆-Cycloalkinyl, Phenyl oder Phenyl-(C₁-C₆)-Alkyl bedeutet; oder
SO₂R²⁵, wobei
   R²⁵ C₁-C₆-Alkyl oder Phenyl bedeutet;
   wobei die genannten aliphatischen, cyclischen oder aromatischen Teile der Substituenten von R¹ und R² partiell oder vollständig halogeniert sein können und/oder eine bis drei der folgenden Gruppen tragen können: Cyano, Hydroxy, C₁-C₄-Alkyl, C₁-C₄-Halogenalkyl, C₃-C₆-Cycloalkyl, C₁-C₄-Alkoxy, C₁-C₄-Alkylthio, [Di-(C₁-C₄)-alkyl]-amino, C₁-C₄-Alkylcarbonyl, Hydroxycarbonyl, C₁-C₄-Alkoxycarbonyl, Aminocarbonyl, C₁-C₄-Alkylaminocarbonyl, [Di-(C₁-C₄)-alkyl]aminocarbonyl oder C₁-C₄-Alkylcarbonyloxy;
und wobei R¹ zusätzlich Wasserstoff bedeuten kann;
- R³: ein Rest R²⁶, OR²⁷, SR²⁸, NR²⁹R³⁰ oder N(OR³¹)R³², wobei
R²⁶, R²⁷, R²⁸ R²⁹ und R³² unabhängig voneinander Wasserstoff, C₁-C₆-Alkyl, C₃-C₆-Cycloalkyl, C₃-C₆-Alkenyl, C₃-C₆-Alkinyl, C₃-C₆-Halogenalkinyl, Formyl, C₁-C₆-Alkylcarbonyl, C₃-C₆-Cycloalkylcarbonyl, C₂-C₆-Alkenylcarbonyl, C₂-C₆-Alkinylcarbonyl, C₁-C₆-Alkoxy-(C₁-C₆)-alkyl, C₁-C₆-Alkoxycarbonyl, C₂-C₆-Alkenyloxycarbonyl, C₃-C₆-Alkinyloxycarbonyl, C₁-C₆-Alkylaminocarbonyl, C₃-C₆-Alkenylaminocarbonyl, C₃-C₆-Alkinylaminocarbonyl, C₁-C₆-Alkylsulfonylaminocarbonyl, C₁-C₆-alkylaminocarbonyl, [Di-(C₁-C₆)-alkylamino]carbonyl, N-(C₃-C₆-Alkenyl)-N-(C₁-C₆-alkyl)-aminocarbonyl, N-(C₃-C₆-Alkinyl)-N-(C₁-C₆-alkyl)-aminocarbonyl, N-(C₁-C₆-Alkoxy)-N-(C₁-C₆-alkyl)-amino-carbonyl, N-(C₃-C₆-Alkenyl)-N-(C₁-C₆-alkoxy)-aminocarbonyl, N-(C₃-C₆-Alkinyl)-N-(C₁-C₆-alkoxy)-aminocarbonyl, Di-(C₁-C₆-alkyl)-aminothiocarbonyl, C₁-C₆-Alkylcarbonyl-C₁-C₆-alkyl, C₁-C₆-Alkoxyimino-C₁-C₆-alkyl, N-(C₁-C₆-Alkylamino)-imino-C₁-C₆-alkyl, N-(Di-C₁-C₆-alkylamino)-imino-C₁-C₆-alkyl oder [Tri-(C₁-C₄)-alkyl]silyl, wobei die genannten aliphatischen oder isocyclischen Teile der Substituenten partiell oder vollständig halogeniert sein können und/oder eine bis drei der folgenden Gruppen tragen können: Cyano, Hydroxy, C₁-C₄-Alkyl, C₁-C₄-Halogenalkyl, C₃-C₆-Cycloalkyl, C₁-C₄-Alkoxy, C₁-C₄-Alkylthio, [Di-(C₁-C₄)-alkyl]-amino, C₁-C₄-Alkylcarbonyl, Hydroxycarbonyl, C₁-C₄-Alkoxycarbonyl, Aminocarbonyl, C₁-C₄-Alkylaminocarbonyl, [Di-(C₁-C₄)-alkyl]aminocarbonyl oder C₁-C₄-Alkylcarbonyloxy; oder
Phenyl, Phenyl-C₁-C₆-alkyl, Phenylcarbonyl, Phenylcarbonyl-C₁-C₆-alkyl, Phenoxycarbonyl, Phenylaminocarbonyl, Phenylsulfonylaminocarbonyl, N-(C₁-C₆-Alkyl)-N-(phenyl)-aminocarbonyl, Phenyl-C₁-C₆-alkylcarbonyl, Heterocyclyl, Heterocyclyl-C₁-C₆-alkyl, Heterocyclylcarbonyl, Heterocyclylcarbonyl-C₁-C₆-alkyl, Heterocyclyloxycarbonyl, Heterocyclylaminocarbonyl, Heterocyclylsulfonylaminocarbonyl, N-(C₁-C₆-Alkyl)-N-(heterocyclyl)-aminocarbonyl, oder Heterocyclyl-C₁-C₆-alkylcarbonyl, wobei die Phenyl- oder Heterocyclyl-Teile der Substituenten partiell oder vollständig halogeniert sein können und/oder eine bis drei der folgenden Gruppen tragen können: Nitro, Cyano, C₁-C₄-Alkyl, C₁-C₄-Halogenalkyl, C₁-C₄-Alkoxy oder C₁-C₄-Halogenalkoxy; oder
S(O)ₙR³³ bedeuten, wobei
- n: 1 oder 2 bedeutet; und
R³³ C₁-C₆-Alkyl, C₁-C₆-Halogenalkyl oder Phenyl bedeutet, und wobei der Phenylsubstituent partiell oder vollständig halogeniert sein kann und/oder eine bis drei der folgenden Gruppen tragen kann: Nitro, Cyano, C₁-C₄-Alkyl, C₁-C₄-Halogenalkyl, C₁-C₄-Alkoxy oder C₁-C₄-Halogenalkoxy; und
R³⁰ und R³¹ unabhängig voneinander Wasserstoff, C₁-C₆-Alkyl, C₃-C₆-Cycloalkyl, C₂-C₆-Alkenyl, C₂-C₆-Alkinyl, wobei aliphatische oder isocyclische Teile der Substituenten partiell oder vollständig halogeniert sein können und/oder eine bis drei der folgenden Gruppen tragen können: Cyano, Hydroxy, C₁-C₄-Alkyl, C₁-C₄-Halogenalkyl, C₃-C₆-Cycloalkyl, C₁-C₄-Alkoxy, C₁-C₄-Alkylthio, [Di-(C₁-C₄)-alkyl]-amino, C₁-C₄-Alkylcarbonyl, Hydroxycarbonyl, C₁-C₄-Alkoxycarbonyl, Aminocarbonyl, C₁-C₄-Alkylaminocarbonyl, [Di-(C₁-C₄)-alkyl]aminocarbonyl oder C₁-C₄-Alkylcarbonyloxy,
Phenyl, Phenyl-C₁-C₆-alkyl, Heterocyclyl oder Heterocyclyl-C₁-C₆-alkyl bedeuten, wobei die Phenyl- oder Heterocyclyl-Teile der Substituenten partiell oder vollständig halogeniert sein können und/oder eine bis drei der folgenden Gruppen tragen können: Nitro, Cyano, C₁-C₄-Alkyl, C₁-C₄-Halogenalkyl, C₁-C₄-Alkoxy oder C₁-C₄-Halogenalkoxy;
R⁴, R⁵, R⁶ unabhängig voneinander Wasserstoff, C₁-C₆-Alkyl, C₁-C₆-Alkoxy,
wobei die genannten aliphatischen Teile der Substituenten von R⁴, R⁵ oder R⁶ partiell oder vollständig halogeniert sein können und/oder eine bis drei der folgenden Gruppen tragen können: Cyano, Hydroxy, C₁-C₄-Alkyl, C₁-C₄-Halogenalkyl, C₃-C₅-Cycloalkyl, C₁-C₄-Alkoxy, C₁-C₄-Alkylthio, [Di-(C₁-C₄)-alkyl]-amino, C₁-C₄-Alkylcarbonyl, Hydroxycarbonyl, C₁-C₄-Alkoxycarbonyl, Aminocarbonyl, C₁-C₄-Alkylaminocarbonyl, [Di-(C₁-C₄)-alkyl]aminocarbonyl oder C₁-C₄-Alkylcarbonyloxy;
R³ und R⁴ auch gemeinsam eine Ketogruppe bedeuten können;
R⁷, R⁸ unabhängig voneinander Wasserstoff, Hydroxy, C₁-C₆-Alkyl, das partiell oder vollständig halogeniert sein kann und/oder eine bis drei der folgenden Gruppen tragen kann: Cyano, Hydroxy, C₁-C₄-Alkyl, C₁-C₄-Halogenalkyl, C₃-C₆-Cycloalkyl, C₁-C₄-Alkoxy, C₁-C₄-Alkylthio, [Di-(C₁-C₄)-alkyl]-amino, C₁-C₄-Alkylcarbonyl, Hydroxycarbonyl, C₁-C₄-Alkoxycarbonyl, Aminocarbonyl, C₁-C₄-Alkylaminocarbonyl, [Di-(C₁-C₄)-alkyl]aminocarbonyl oder C₁-C₄-Alkylcarbonyloxy;
A¹, A² unabhängig voneinander Aryl oder Heteroaryl, mit der Ausnahme von Indolyl, bedeuten, wobei R^{a} in ortho-Position zur Verknüpfungsstelle von A¹ an ein C-Atom oder N-Atom von A¹ gebunden ist und wobei R^{a} eine der im Folgenden angegebenen Bedeutungen aufweist:
R^{a} Halogen, Cyano, Nitro, C₁-C₆-Alkyl, C₃-C₆-Cycloalkyl, C₂-C₆-Alkenyl, C₃-C₆-Cycloalkenyl, C₄-C₁₀-Alkadienyl, C₂-C₆-Alkinyl, [Tri-(C₁-C₆)-alkylsilyl]-(C₂-C₆)-alkinyl, C₃-C₆-Cycloalkinyl, C₁-C₆-Alkylthio, C₁-C₆-Alkylsulfinyl, Aryl, Phenyl-(C₁-C₆)-Alkyl, Phenyl-(C₂-C₆)-Alkenyl, Phenylsulfonyl-(C₁-C₆)-Alkyl, Heterocyclyl, Heterocyclyl-(C₁-C₆)-Alkyl oder Phenyl-[C₁-C₆-alkoxycarbonyl]-(C₁-C₆)-alkyl,
   Z¹P(O)(OR⁹)₂, Z²B(OR¹⁰)₂, wobei
   R⁹ und R¹⁰ jeweils Wasserstoff oder C₁-C₆-Alkyl bedeuten und R¹⁰ in Z²B(OR¹⁰)₂ zusammen eine C₂-C₄-Alkylenkette bilden können; oder
   Z³COR¹¹, wobei
   R¹¹ Wasserstoff, C₁-C₆-Alkyl, C₃-C₆-Cycloalkyl, C₂-C₆-Alkenyl, C₃-C₆-Cycloalkenyl, C₂-C₆-Alkinyl, C₃-C₆-Cycloalkinyl, Hydroxy, C₁-C₆-Alkoxy, C₃-C₆-Alkenyloxy, C₃-C₆-Alkinyloxy, Amino, C₁-C₆-Alkylamino, [Di-(C₁-C₆)-alkyl]amino, C₁-C₆-Alkoxyamino, [Di-(C₁-C₆)-Alkoxy]amino, C₁-C₆-Alkylsulfonylamino, C₁-C₆-Alkylaminosulfonylamino, [Di-(C₁-C₆)-alkylamino]sulfonylamino, C₃-C₆-Alkenylamino, C₃-C₆-Alkinylamino, N-(C₂-C₆-Alkenyl)-N-(C₁-C₆-alkyl)-amino, N-(C₂-C₆-Alkinyl)-N-(C₁-C₆-alkyl)-amino, N-(C₁-C₆-Alkoxy)-N-(C₁-C₆-alkyl)-amino, N-(C₂-C₆-Alkenyl)-N-(C₁-C₆-alkoxy)-amino, N-(C₂-C₆-Alkinyl)-N-(C₁-C₆-alkoxy)-amino, Phenyl, Phenoxy, Phenylamino, Naphthyl oder Heterocyclyl bedeutet; oder
   Z⁴NR¹²R¹³, wobei
   R¹² und R¹³ unabhängig voneinander Wasserstoff, C₁-C₆-Alkyl, C₃-C₆-Cycloalkyl, C₃-C₆-Alkenyl, C₃-C₆-Cycloalkenyl, C₃-C₆-Alkinyl, C₃-C₆-Cycloalkinyl, C₁-C₆-Alkylcarbonyl, C₃-C₆-Cycloalkylcarbonyl, [Di-(C₁-C₆)-alkylamino]carbonyl, C₁-C₆-Alkoxycarbonyl, C₁-C₆-Alkoxycarbonyl-(C₁-C₆)-alkyl, C₁-C₆-Alkylsulfonyl, C₁-C₆-Alkylaminosulfonyl, [Di-(C₁-C₆)-alkylamino]sulfonyl, Phenylcarbonyl, Phenylaminocarbonyl, Phenylsulfonyl, Phenylsulfonylaminocarbonyl oder Heterocyclylcarbonyl bedeuten; oder
   Z⁵CH=N-O-R¹⁴, wobei R¹⁴ Wasserstoff oder C₁-C₆-Alkyl bedeutet; oder
   Z⁶OR¹⁵, wobei
   R¹⁵ Wasserstoff, C₁-C₆-Alkyl, C₃-C₆-Cycloalkyl, C₃-C₆-Alkenyl, C₃-C₆-Cycloalkenyl, C₃-C₆-Alkinyl, C₃-C₆-Cycloalkinyl, C₁-C₆-Alkylcarbonyl, C₁-C₆-Alkoxycarbonyl-(C₁-C₆)-alkyl, [Di-(C₁-C₆)-Alkoxycarbonyl]-(C₁-C₆)-alkyl, Phenyl oder Phenyl-(C₁-C₆)-Alkyl bedeutet; oder
   Z⁷SO₂R¹⁶, wobei R¹⁶ C₁-C₆-Alkyl oder Phenyl bedeutet; und wobei
   Z¹, Z², Z³, Z⁴, Z⁵, Z⁶, Z⁷ unabhängig voneinander eine Bindung, -CH₂-, -CH₂-CH₂-, -O-CH(R¹⁷)-, -S-CH(R¹⁸)-, -S(O)-CH(R¹⁹)- oder -SO₂CH(R²⁰)- bedeutet, und wobei R¹⁷, R¹⁸, R¹⁹ und R²⁰ unabhängig voneinander Wasserstoff oder C₁-C₆-Alkyl bedeuten; und
   wobei die genannten aliphatischen, cyclischen oder aromatischen Teile des Substituenten R^{a} partiell oder vollständig halogeniert sein können und/oder eine bis drei der folgenden Gruppen tragen können: Cyano, Hydroxy, C₁-C₄-Alkyl, C₁-C₄-Halogenalkyl, C₃-C₆-Cycloalkyl, C₁-C₄-Alkoxy, C₁-C₄-Alkylthio, [Di-(C₁-C₄)-alkyl]-amino, C₁-C₄-Alkylcarbonyl, Hydroxycarbonyl, C₁-C₄-Alkoxycarbonyl, Aminocarbonyl, C₁-C₄-Alkylaminocarbonyl, [Di-(C₁-C₄)-alkyl]aminocarbonyl oder C₁-C₄-Alkylcarbonyloxy; und
   R^{b}, R^{c}, R^{d}, Re und R^{f} jeweils unabhängig voneinander für Wasserstoff stehen oder eine der für R^{a} angegebenen Bedeutungen aufweisen und
worin zwei an benachbarte Ringatome von A¹ gebundene Reste R^{a}, R^{b} oder R^{c} oder zwei an benachbarte Ringatome von A² gebundene Reste R^{d}, Re oder R^{f} auch für lineares C₃-C₆-Alkylen stehen können, das teilweise oder vollständig halogeniert sein kann und das eine bis drei der folgenden Gruppen tragen können: Cyano, Hydroxy, C₁-C₄-Alkyl, C₁-C₄-Halogenalkyl, C₃-C₆-Cycloalkyl, C₁-C₄-Alkoxy, C₁-C₄-Alkylthio, [Di-(C₁-C₄)-alkyl]-amino, C₁-C₄-Alkylcarbonyl, Hydroxycarbonyl, C₁-C₄-Alkoxycarbonyl, Aminocarbonyl, C₁-C₄-Alkylaminocarbonyl, [Di-(C₁-C₄)-alkyl]aminocarbonyl oder C₁-C₄-Alkylcarbonyloxy, worin eine CH₂-Gruppe in C₃-C₆-Alkylen durch eine Carbonylgruppe, Thiocarbonylgruppe oder Sulfonylgruppe ersetzt sein kann und worin eine oder zwei nicht benachbarte CH₂-Gruppen in C₃-C₆-Alkylen jeweils durch Sauerstoff, Schwefel oder eine Gruppe NR³⁴ ersetzt sein können, wobei R³⁴ eine für R¹² angegebenen bedeutungen aufweist.

Bei den von dem Pflanzenpathogen S. scabies produzierten Thaxtominen A und B (King R. R. et al., J. Agric. Food Chem. (1992) 40, 834-837) handelt es sich um Naturstoffe mit einem zentralen Piperazin-2,5-dion-Ring, der in der 3-Position einen 4-Nitroindol-3-ylmethyl-Rest und in der 2-Position einen gegebenenfalls durch OH substituierten Benzyl-Rest trägt. Aufgrund ihrer pflanzenschädigenden Wirkung wurde auch die Verwendungsmögfichkeit dieser Verbindungsklasse als Herbizide untersucht (King R. R. et al., J. Agric. Food Chem. (2001) 49, 2298-2301 ).

Die EP-A 181152 und EP-A 243122 beschreiben strukturell ähnliche Piperazinverbindungen und ihre Verwendung als Antagonisten des Platelet Activating Factor.

Die US 2003/0171379 A1 beschreibt die Verwendung von Mactanamid, einem fungistatischen Diketopiperazin der Formel A, 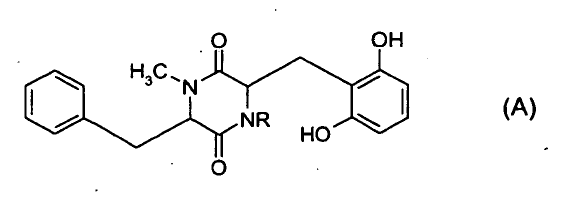 worin R für H oder Methyl steht, als entzündungshemmenden Wirkstoff in der Medizin.

Die WO 99/48889, WO 01/53290 und WO 2005/011699 beschreiben 2,5-Diketopiperazinverbindungen, die in der 3- bzw. 6-Position einen über eine Methylen- oder Methingruppe gebundenen 4-Imidazolyl-Rest und in der anderen 3- bzw. 6-Position einen Benzylrest aufweisen. Bei diesen Verbindungen handelt es sich um Antitumor-Wirkstoffe.

Die Verbindung 1,4-Diacetyl-3,6-di-(2-chlorphenyl)piperazin-2,5-dion ist bekannt (L. X. Wang, Y. Z. Shi, Z. M. Du, H. W. Hu, Chinese Chem. Lett. (1993) 4, 687-688).

Eine Aufgabe der vorliegenden Erfindung ist die Bereitstellung von Verbindungen mit herbizider Wirkung. Insbesondere sollen Verbindungen zur Verfügung gestellt werden, die eine hohe herbizide Wirkung, insbesondere bereits bei niedrigen Aufwandmengen, aufweisen und deren Verträglichkeit gegenüber Kulturpflanzen für eine kommerzielle Verwertung hinreichend ist.

Diese und weitere Aufgaben werden durch die eingangs definierten Verbindungen der Formel I und durch ihre landwirtschaftlich geeigneten Salze gelöst.

Dementsprechend betrifft die vorliegende Erfindung die Verwendung von Piperazinverbindungen der allgemeinen Formel I oder der landwirtschaftlich brauchbaren Salze von Piperazinverbindungen der Formel I als Herbizide, d.h. zur Bekämpfung von Schadpflanzen.

Die Erfindung betrifft auch Mittel, welche eine herbizid wirksame Menge mindestens einer Piperazinverbindung der Formel I oder eines landwirtschaftlich brauchbaren Salzes von I und für die Formulierung von Pflanzenschutzmitteln übliche Hilfsmittel enthalten.

Die Erfindung betrifft außerdem ein Verfahren zur Bekämpfung von unerwünschtem Pflanzenwuchs, bei dem man eine herbizid wirksame Menge mindestens einer Piperazinverbindung der Formel I oder eines landwirtschaftlich brauchbaren Salzes von I auf Pflanzen, deren Samen und/oder deren Lebensraum einwirken läßt.

Die Piperazinverbindungen der Formel I sind neu und ebenfalls Gegenstand der vorliegenden Erfindung,
- ausgenommen Verbindungen der Formel I, worin A¹ und A² Phenyl bedeuten, R¹ Methyl bedeutet, R² Wasserstoff oder Methyl bedeutet, R³, R⁴, R⁵, R⁶, R⁷, R⁸ Wasserstoff bedeuten, die Substituenten R^{a} und R^{b} jeweils für Hydroxy stehen, die in den beiden ortho-Position des Phenylrings A¹ angeordnet sind, und R^{c}, R^{d}, R^{e} und R^{f} Wasserstoff bedeuten,
- weiterhin ausgenommen Verbindungen der Formel I, worin A¹ für Phenyl steht und A² 4-Imidazolyl bedeutet oder A¹ für 4-Imidazolyl steht und A² Phenyl bedeutet, und
- weiterhin ausgenommen eine Verbindung der Formel I, worin A¹ für Phenyl steht, R^{a} Chlor bedeutet, R^{b} und R^{c} für Wasserstoff stehen, die Gruppe A²(R^{d}R^{e}R^{f}) für o-Chlorphenyl steht, R¹ und R² Methylcarbonyl bedeuten und R³ bis R⁸ jeweils Wasserstoff bedeuten.

Die Erfindung betrifft außerdem Verfahren und Zwischenprodukte zur Herstellung von Verbindungen der Formel I.

Weitere Ausführungsformen der vorliegenden Erfindung sind den Ansprüchen, der Beschreibung und den Beispielen zu entnehmen. Es versteht sich, dass die vorstehend genannten und die nachstehend noch zu erläuternden Merkmale des erfindungsgemäßen Gegenstandes nicht nur in der jeweils angegebenen Kombination, sondern auch in anderen Kombinationen verwendbar sind, ohne den Rahmen der Erfindung zu verlassen.

Die Verbindungen der Formel I können je nach Substitutionsmuster ein oder mehrere Chiralitätszentren enthalten und liegen dann als Enantiomeren- oder Diastereomerengemische vor. Gegenstand der Erfindung sind sowohl die reinen Enantiomeren oder Diastereomeren als auch deren Gemische.

Die Verbindungen der Formel I können auch in Form ihrer landwirtschaftlich brauchbaren Salze vorliegen, wobei es auf die Art des Salzes in der Regel nicht ankommt. Im Allgemeinen kommen die Salze derjenigen Kationen oder die Säureadditionssalze derjenigen Säuren in Betracht, deren Kationen, beziehungsweise Anionen, die herbizide Wirkung der Verbindungen I nicht negativ beeinträchtigen.

Es kommen als Kationen insbesondere Ionen der Alkalimetalle, vorzugsweise Lithium, Natrium oder Kalium, der Erdalkalimetalle, vorzugsweise Calcium oder Magnesium, und der Übergangsmetalle, vorzugsweise Mangan, Kupfer, Zink oder Eisen in Betracht. Ebenso kann als Kation Ammonium verwendet werden, wobei hier gewünschtenfalls ein bis vier Wasserstoffatome durch C₁-C₄-Alkyl, Hydroxy-C₁-C₄-alkyl, C₁-C₄-Alkoxy-C₁-C₄-alkyl, Hydroxy-C₁-C₄-alkoxy-C₁-C₄-alkyl, Phenyl oder Benzyl ersetzt sein können, vorzugsweise Ammonium, Dimethylammonium, Diisopropylammonium, Tetramethylammonium, Tetrabutylammonium, 2-(2-Hydroxyeth-1-oxy)eth-1-ylammonium, Di(2-hydroxyeth-1-yl)ammonium, Trimethylbenzylammonium. Des Weiteren kommen Phosphoniumionen, Sulfoniumionen, vorzugsweise Tri(C₁-C₄-alkyl)sulfonium oder Sulfoxoniumionen, vorzugsweise Tri(C₁-C₄-alkyl)sulfoxonium, in Betracht.

Anionen von brauchbaren Säureadditionsalzen sind in erster Linie Chlorid, Bromid, Fluorid, Hydrogensulfat, Sulfat, Dihydrogenphosphat, Hydrogenphosphat, Nitrat, Hydrogencarbonat, Carbonat, Hexafluorosilikat, Hexafluorophosphat, Benzoat sowie die Anionen von C₁-C₄-Alkansäuren, vorzugsweise Formiat, Acetat, Propionat oder Butyrat.

Die für die Substituenten der erfindungsgemäßen Verbindungen genannten organischen Molekülteile stellen Sammelbegriffe für individuelle Aufzählungen der einzelnen Gruppenmitglieder dar. Sämtliche Kohlenwasserstoffketten, wie
Alkyl-, Halogenalkyl-, sowie die Alkylteile in Cyanoalkyl-, Alkoxy-, Halogenalkoxy-, Alkylthio-, Halogenalkylthio-, Alkylsulfinyl-, Halogenalkylsulfinyl-, Alkylsulfonyl-, Halogenalkylsulfonyl-, N-Alkylaminosulfonyl, N,N-Dialkylaminosulfonyl, Dialkylamino-, N-Alkylsulfonylamino, N-Halogenalkylsulfonylamino, N-Alkyl-N-alkylsulfonylamino, N-Alkyl-N-halogenalkylsulfonylamino, Alkylcarbonyl-, Halogenalkylcarbonyl-, Alkoxycarbonyl-, Halogenalkoxycarbonyl, Alkylcarbonyloky-, Alkylaminocarbonyl-, Dialkylaminocarbonyl-, Dialkylaminothiocarbonyl, Alkoxyalkyl-, Dialkoxyalkyl-, Alkylthioalkyl-, Dialkylaminoalkyl-, Dialkylhydrazinoalkyl-, Alkyliminooxyalkyl-, Alkylcarbonylalkyl, Alkoxyiminoalkyl, N-(Alkylamino)-iminoalkyl, N-(Dialkylamino)-iminoalkyl, Alkoxycarbonylalkyl-, Dialkylaminocarbonylalkyl-, Phenylalkenylcarbonyl, Heterocyclylalkenylcarbonyl, N-Alkoxy-N-alkylaminocarbonyl-, N-Alkyl-N-phenylaminocarbonyl-, N-Alkyl-N-heterocyclylaminocarbonyl-, Phenylalkyl-, Heterocyclylalkyl-, Phenylcarbonylalkyl-, Heterocyclylcarbonylalkyl-, Dialkylaminoalkoxycarbonyl-, Alkoxyalkoxycarbonyl-, Alkenylcarbonyl-, Alkenyloxycarbonyl-, Alkenylaminocarbonyl-, N-Alkenyl-N-alkylaminocarbonyl-, N-Alkenyl-N-alkoxyaminocarbonyl-, Alkinylcarbonyl-, Alkinyloxycarbonyl-, Alkinylaminocarbonyl-, N-Alkinyl-N-alkylaminocarbonyl-, N-Alkinyl-N-alkoxyaminocarbonyl-, Alkenyl-, Alkinyl-, Halogenalkenyl-, Halogenalkinyl- und Alkoxyalkoxy-Teile
können geradkettig oder verzweigt sein. Das Präfix Cₙ-Cₘ- gibt die jeweilige Kohlenstoffzahl der Kohlenwasserstoffeinheit an. Sofern nicht anders angegeben tragen halogenierte Substituenten vorzugsweise ein bis fünf gleiche oder verschiedene Halogenatome, insbesondere Fluoratome oder Chloratome.

Die Bedeutung Halogen steht jeweils für Fluor, Chlor, Brom oder Iod.

Ferner bedeuten beispielsweise:
Alkyl sowie die Alkylteile beispielsweise in Alkoxy, Alkylthio, Alkylsulfinyl und Alkylsulfonyl, Alkylcarbonyl, Alkylamino, Alkylsilyl, Phenylalkyl, Phenylsulfonylalkyl, Heterocyclylalkyl : gesättigte, geradkettige oder verzweigte Kohlenwasserstoffreste mit einem oder mehr C-Atomen, z.B. 1 bis 2, 1 bis 4, oder 1 bis 6 Kohlenstoffatomen, z.B. C₁-C₆-Alkyl wie Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl. In einer erfindungsgemäßen Ausführungsform steht Alkyl für kleine Alkylgruppen wie C₁-C₄-Alkyl. In einer anderen erfindungsgemäßen Ausführungsform steht Alkyl für größere Alkylgruppen wie C₅-C₆-Alkyl.
Halogenalkyl: einen Alkylrest wie vorstehend genannt, dessen Wasserstoffatome partiell oder vollständig durch Halogenatome wie Fluor, Chlor, Brom und/oder Iod substituiert sind, z.B. Chlormethyl, Dichlormethyl, Trichlormethyl, Fluormethyl, Difluormethyl, Trifluormethyl, Chlorfluormethyl, Dichlorfluormethyl, Chlordifluormethyl, 2-Fluorethyl, 2-Chlorethyl, 2-Bromethyl, 2-lodethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl, 2-Fluorpropyl, 3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifluorpropyl, 3,3,3-Trichlorpropyl, 2,2,3,3,3-Pentafluorpropyl, Heptafluorpropyl, 1-(Fluormethyl)-2-fluorethyl, 1-(Chlormethyl)-2-chlorethyl, 1-(Brommethyl)-2-bromethyl, 4-Fluorbutyl, 4-Chlorbutyl, 4-Brombutyl und Nonafluorbutyl.
Cycloalkyl sowie die Cycloalkylteile beispielsweise in Cycloalkoxy oder Cycloalkylcarbonyl: monocyclische, gesättigte Kohlenwasserstoffgruppen mit drei oder mehr C-Atomen, z.B. 3 bis 6 Kohlenstoffringgliedem, wie Cyclopropyl, Cyclobutyl, Cyclopentyl und Cyclohexyl.
Alkenyl sowie Alkenylteile beispielsweise in Phenyl-(C₂-C₆)-Alkenyl oder Alkenylamino: einfach ungesättigte, geradkettige oder verzweigte Kohlenwasserstoffreste mit zwei oder mehr C-Atomen, z. B. 2 bis 4, 2 bis 6 oder 3 bis 6 Kohlenstoffatomen und einer Doppelbindung in einer beliebigen Position, z.B. C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-penteriyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl, 1-Ethyl-2-methyl-2-propenyl.

In einer erfindungsgemäßen Ausführungsform werden Alkenylgruppen wie C₂-C₆-Alkenyl verwendet. In einer anderen erfindungsgemäßen Ausführungsform werden Alkenylgruppen wie C₃-C₆-Alkenyl eingesetzt.

Cycloalkenyl sowie Cycloalkenylteile: monocyclische, einfach ungesättigte Kohlenwasserstoffgruppen mit drei oder mehr C-Atomen, z. B. 3 bis 6, vorzugsweise 5 bis 6 Kohlenstoffringgliedern, wie Cyclopenten-1-yl, Cyclopenten-3-yl, Cyclohexen-1-yl, Cyclohexen-3-yl, Cyclohexen-4-yl.

Alkinyl sowie Alkinylteile beispielsweise in [Tri-(C₁-C₆)-alkylsilyl-(C₂-C₆)-alkinyl oder Alkinylamino: geradkettige oder verzweigte Kohlenwasserstoffgruppen mit zwei oder mehr C-Atomen, z. B. 2 bis 4, 2 bis 6, oder 3 bis 6 Kohlenstoffatomen und einer oder zwei Dreifachbindungen in beliebiger, jedoch nicht benachbarter Position, z. B. C₂-C₆-Alkinyl wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-penfinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl, 1-Ethyl-1-methyl-2-propinyl.

Cycloalkinyl sowie Cyctoalkinylteile: monocyclische Kohlenwasserstoffgruppen mit drei der mehr C-Atomen, z. B. 3 bis 6, vorzugsweise 5 bis 6 Kohlenstoffringgliedem und einer Dreifachbindung, wie Cyclohexin-1-yl, Cyclohexin-3-yl, Cyclohexin-4-yl.

C₄-C₁₀-Alkadienyl: zweifach ungesättigte, geradkettige oder verzweigte Kohlenwasserstoffreste mit vier oder mehr C-Atomen und zwei Doppelbindungen in einer beliebigen, jedoch nicht benachbarten Position, z. B. 4 bis 10 Kohlenstoffatomen und zwei Doppelbindungen in einer beliebigen, jedoch nicht benachbarten Position, z. B. 1,3-Butadienyl, 1-Methyl-1,3-butadienyl, 2-Methyl-1,3-butadienyl, Penta-1,3-dien-1-yl, Hexa-1,4-dien-1-yl, Hexa-1,4-dien-3-yl, Hexa-1,4-dien-6-yl, Hexa-1,5-dien-1-yl, Hexa-1,5-dien-3-yl, Hexa-1,5-dien-4-yl, Hepta-1,4-dien-1-yl, Hepta-1,4-dien-3-yl, Hepta-1,4-dien-6-yl, Hepta-1,4-dien-7-yl, Hepta-1,5-dien-1-yl, Hepta-1,5-dien-3-yl, Hepta-1,5-dien-4-yl, Hepta-1,5-dien-7-yl, Hepta-1,6-dien-1-yl, Hepta-1,6-dien-3-yl, Hepta-1,6-dien-4-yl, Hepta-1,6-dien-5-yl, Hepta-1,6-dien-2-yl, Octa-1,4-dien-1-yl, Octa-1,4-dien-2-yl, Octa-1,4-dien-3-yl, Octa-1,4-dien-6-yl, Octa-1,4-dien-7-yl, Octa-1,5-dien-1-yl, Octa-1,5-dien-3-yl, Octa-1,5-dien-4-yl, Octa-1,5-dien-7-yl, Octa-1,6-dien-1-yl, Octa-1,6-dien-3-yl, Octa-1,6-dien-4-yl, Octa-1,6-dien-5-yl, Octa-1,6-dien-2-yl, Deca-1,4-dienyl, Deca-1,5-dienyl, Deca-1,6-dienyl, Deca-1,7-dienyl, Deca-1,8-dienyl, Deca-2,5-dienyl, Deca-2,6-dienyl, Deca-2,7-dienyl, Deca-2,8-dienyl.

Alkoxy oder Alkoxyteile beispielsweise in Phenylalkoxy, Alkoxyamino, Alkoxycarbonyl: Alkyl, wie vorstehend definiert, das über ein O-Atom gebunden ist: z. B. Methoxy, Ethoxy, n-Propoxy, 1-Methylethoxy, Butoxy, 1-Methylpropoxy, 2-Methylpropoxy oder 1,1-Dimethylethoxy, Pentoxy, 1-Methylbutoxy, 2-Methylbutoxy, 3-Methylbutoxy, 1,1-Dimethylpropoxy, 1,2-Dimethylpropoxy, 2,2-Dimethylpropoxy, 1-Ethylpropoxy, Hexoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy oder 1-Ethyl-2-methylpropoxy.

In einer erfindungsgemäßen Ausführungsform werden kleine Alkoxygruppen wie C₁-C₄-Alkoxy verwendet. In einer anderen erfindungsgemäßen Ausführungsform werden größere Alkoxygruppen wie C₅-C₆-Alkoxy eingesetzt.

Alkenyloxy: Alkenyl wie vorstehend genannt, das über ein Sauerstoffatom gebunden ist, z. B. C₃-C₆-Alkenyloxy wie 1-Propenyloxy, 2-Propenyloxy, 1-Methylethenyloxy, 1-Butenyloxy, 2-Butenyloxy, 3-Butenyloxy, 1-Methyl-1-propenyloxy, 2-Methyl-1-propenyloxy, 1-Methyl-2-propenyloxy, 2-Methyl-2-propenyloxy, 1-Pentenyloxy, 2-Pentenyloxy, 3-Pentenyloxy, 4-Pentenyloxy, 1-Methyl-1-butenyloxy, 2-Methyl-1-butenyloxy, 3-Methyl-1-butenyloxy, 1-Methyl-2-butenyloxy, 2-Methyl-2-butenyloxy, 3-Methyl-2-butenyloxy, 1-Methyl-3-butenyloxy, 2-Methyl-3-butenyloxy, 3-Methyl-3-butenyloxy, 1,1-Dimethyl-2-propenyloxy, 1,2-Dimethyl-1-propenyloxy, 1,2-Dimethyl-2-propenyloxy, 1-Ethyl-1-propenyloxy, 1-Ethyl-2-propenyloxy, 1-Hexenyloxy, 2-Hexenyloxy, 3-Hexenyloxy, 4-Hexenyloxy, 5-Hexenyloxy, 1-Methyt-1-pentenyloxy, 2-Methyl-1-pentenyloxy, 3-Methyl-1-pentenyloxy, 4-Methyl-1-pentenyloxy, 1-Methyl-2-pentenyloxy, 2-Methyl-2-pentenyloxy, 3-Methyl-2-pentenyloxy, 4-Methyl-2-pentenyloxy, 1-Methyl-3-pentenyloxy, 2-Methyl-3-pentenyloxy, 3-Methyl-3-pentenyloxy, 4-Methyl-3-pentenyloxy, 1-Methyl-4-pentenyloxy, 2-Methyl-4-pentenyloxy, 3-Methyl-4-pentenyloxy, 4-Methyl-4-pentenyloxy, 1,1-Dimethyl-2-butenyloxy, 1,1-Dimethyl-3-butenyloxy, 1,2-Dimethyl-1-butenyloxy, 1,2-Dimethyl-2-butenyloxy, 1,2-Dimethyl-3-butenyloxy, 1,3-Dimethyl-1-butenyloxy, 1,3-Dimethyl-2-butenyloxy, 1,3-Dimethyl-3-butenyloxy, 2,2-Dimethyl-3-butenyloxy, 2,3-Dimethyl-1-butenyloxy, 2,3-Dimethyl-2-butenyloxy, 2,3-Dimethyl-3-butenyloxy, 3,3-Dimethyl-1-butenyloxy, 3,3-Dimethyl-2-butenyloxy, 1-Ethyl-1-butenyloxy, 1-Ethyl-2-butenyloxy, 1-Ethyl-3-butenyloxy, 2-Ethyl-1-butenyloxy, 2-Ethyl-2-butenyloxy, 2-Ethyl-3-butenyloxy, 1,1,2-Trimethyl-2-propenyloxy, 1-Ethyl-1-methyl-2-propenyloxy, 1-Ethyl-2-methyl-1propenyloxy und 1-Ethyl-2-methyl-2-propenyloxy. In einer erfindungsgemäßen Ausführungsform werden kleine Alkenyloxygruppen wie C₃-C₄-Alkenyloxy verwendet. In einer anderen erfindungsgemäßen Ausführungsform werden größere Alkenyloxygruppen wie C₅-C₆-Alkenyloxy eingesetzt.

Alkinyloxy: Alkinyl wie vorstehend genannt, das über ein Sauerstoffatom gebunden ist, z. B. C₃-C₆-Alkinyloxy wie 2-Propinyloxy, 2-Butinyloxy, 3-Butinyloxy, 1-Methyl-2-propinyloxy, 2-Pentinyloxy, 3-Pentinyloxy, 4-Pentinyloxy, 1-Methyl-2-butinyloxy, 1-Methyl-3-butinyloxy, 2-Methyl-3-butinyloxy, 1-Ethyl-2-propinyloxy, 2-Hexinyloxy, 3-Hexinyloxy, 4-Hexinyloxy, 5-Hexinyloxy, 1-Methyl-2-pentinyloxy, 1-Methyl-3-pentinyloxy. In einer erfndungsgemäßen Ausführungsform werden kleine Alkinyloxygruppen wie C₃-C₄-Alkinyloxy verwendet. In einer anderen erfindungsgemäßen Ausführungsform werden größere Alkinyloxygruppen wie C₅-C₆-Alkinyloxy eingesetzt.

Alkylthio: Alkyl, wie vorstehend definiert, das über ein S-Atom gebunden ist.

Alkylsulfinyl: Alkyl, wie vorstehend definiert, das über eine SO-Gruppe gebunden ist.

Alkylsulfonyl: Alkyl, wie vorstehend definiert, das über eine S(O)₂-Gruppe gebunden ist.

Alkylcarbonyl: Alkyl, wie vorstehend definiert, das über eine (C=O)-Gruppe gebunden ist, z. B. Methylcarbonyl, Ethylcarbonyl, Propylcarbonyl, 1-Methylethylcarbonyl, Butylcarbonyl, 1-Methylpropylcarbonyl, 2-Methylpropylcarbonyl oder 1,1-Dimethylethylcarbonyl, Pentylcarbonyl, 1-Methylbutylcarbonyl, 2-Methylbutylcarbonyl, 3-Methylbutylcarbonyl, 2,2-Dimethylpropylcarbonyl, 1-Ethylpropylcarbonyl, Hexylcarbonyl, 1,1-Dimethylpropylcarbonyl, 1,2-Dimethylpropylcarbonyl, 1-Methylpentylcarbonyl, 2-Methylpentylcarbonyl, 3-Methylpentylcarbonyl, 4-Methylpentylcarbonyl, 1,1-Dimethylbutylcarbonyl, 1,2-Dimethylbutylcarbonyl, 1,3-Dimethylbutylcarbonyl, 2,2,-Dimethylbutylcarbonyl, 2,3-Dimethylbutylcarbonyl, 3,3-Dimethylbutylcarbonyl, 1-Ethylbutylcarbonyl, 2-Ethylbutylcarbönyl, 1,1,2-Trimethylpropylcarbonyl, 1,2,2-Trimethylpropylcarbonyl, 1-Ethyl-1-methylpropylcarbonyl oder 1-Ethyl-2-methylpropylcarbonyl.

Alkenylcarbonyl: Alkenyl, wie vorstehend definiert, das über eine (C=O)-Gruppe gebunden ist, z.B. 1-Ethenylcarbonyl.

Alkinylcarbonyl: Alkinyl, wie vorstehend definiert, das über eine (C=O)-Gruppe gebunden ist, z.B. 1-Propinylcarbonyl.

Heterocyclyl: ein mono- oder bicyclischer, gesättigter, partiell ungesättigter oder aromatischer heterocyclischer Ring mit drei oder mehr, z.B. 3 bis 10 Ring-Atomen, z. B. ein monocyclischer 3-, 4-, 5-, 6-oder 7-gliedriger heterocyclischer Ring, der ein bis vier gleiche oder verschiedene Heteroatome, ausgewählt aus der Gruppe Sauerstoff, Schwefel oder Stickstoff enthält, und über C oder N gebunden sein kann, z. B.
C-gebundene 3-4-gliedrige, gesättigte oder ungesättigt Ringe wie 2-Oxiranyl, 2-Oxetanyl, 3-Oxetanyl, 2-Aziridinyl, 3-Thiethanyl, 1-Azetidinyl, 2-Azetidinyl.
C-gebundene, 5-gliedrige, gesättigte Ringe wie Tetrahydrofuran-2-yl, Tetrahydrofuran-3-yl, Tetrahydrothien-2-yl, Tetrahydrothien-3-yl, Tetrahydropyrrol-2-yl, Tetrahydropyrrol-3-yl, Tetrahydropyrazol-3-yl, Tetrahydro-pyrazol-4-yl, Tetrahydroisoxazol-3-yl, Tetrahydroisoxazol-4-yl, Tetrahydroisoxazol-5-yl, 1,2-Oxathiolan-3-yl, 1,2-Oxathiolan-4-yl, 1,2-Oxathiolan-5-yl, Tetrahydroisothiazol-3-yl, Tetrahydroisothiazol-4-yl, Tetrahydroisothiazol-5-yl, 1,2-Dithiolan-3-yl, 1,2-Dithiolan-4-yl, Tetrahydroimidazol-2-yl, Tetrahydroimidazol-4-yl, Tetrahydrooxazol-2-yl, Tetrahydrooxazol-4-yl, Tetrahydrooxazol-5-yl, Tetrahydrothiazol-2-yl, Tetrahydrothiazol-4-yl, Tetrahydrothiazol-5-yl, 1,3-Dioxolan-2-yl, 1,3-Dioxolan-4-yl, 1,3-Oxathiolan-2-yl, 1,3-Oxathiolan-4-yl, 1,3-Oxathiolan-5-yl, 1,3-Dithiolan-2-yl, 1,3-Dithiolan-4-yl, 1,3,2-Dioxathiolan-4-yl.
C-gebundene, 6-gliedrige, gesättigte Ringe wie:
Tetrahydropyran-2-yl, Tetrahydropyran-3-yl, Tetrahydropyran-4-yl, Piperidin-2-yl, Piperidin-3-yl, Piperidin-4-yl, Tetrahydrothiopyran-2-yl, Tetrahydrothiopyran-3-yl, Tetrahydrothiopyran-4-yl, 1,3-Dioxan-2-yl, 1,3-Dioxan-4-yl, 1,3-Dioxan-5-yl, 1,4-Dioxan-2-yl, 1,3-Dithian-2-yl, 1,3-Dithian-4-yl, 1,3-Dithian-5-yl, 1,4-Dithian-2-yl, 1,3-Oxathian-2-yl, 1,3-Oxathian-4-yl, 1,3-Oxathian-5-yl, 1,3-Oxathian-6-yl, 1,4-Oxathian-2-yl, 1,4-Oxathian-3-yl, 1,2-Dithian-3-yl, 1,2-Dithian-4-yl, Hexahydropyrimidin-2-yl, Hexahydropyrimidin-4-yl, Hexahydropyrimidin-5-yl, Hexahydropyrazin-2-yl, Hexahydropyridazin-3-yl, Hexahydropyridazin-4-yl, Tetrahydro-1,3-oxazin-2-yl, Tetrahydro-1,3-oxazin-4-yl, Tetrahydro-1,3-oxazin-5-yl, Tetrahydro-1,3-oxazin-6-yl, Tetrahydro-1,3-thiazin-2-yl, Tetrahydro-1,3-thiazin-4-yl, Tetrahydro-1,3-thiazin-5-yl, Tetrahydro-1,3-thiazin-6-yl, Tetrahydro-1,4-thiazin-2-yl, Tetrahydro-1,4-thiazin-3-yl, Tetrahydro-1,4-oxazin-2-yl, Tetrahydro-1,4-oxazin-3-yl, Tetrahydro-1,2-oxazin-3-yl, Tetrahydro-1,2-oxazin-4-yl, Tetrahydro-1,2-oxazin-5-yl, Tetrahydro-1,2-oxazin-6-yl.
N-gebundene, 5-gliedrige, gesättigte Ringe wie:
Tetrahydropyrrol-1-yl, Tetrahydropyrazol-1-yl, Tetrahydroisoxazol-2-yl, Tetrahydroisothiazol-2-yl, Tetrahydroimidazol-1-yl, Tetrahydrooxazol-3-yl, Tetrahydrothiazol-3-yl.
N-gebundene, 6-gliedrige, gesättigte Ringe wie:
Piperidin-1-yl, Hexahydropyrimidin-1-yl, Hexahydropyrazin-1-yl, Hexahydropyridazin-1-yl, Tetrahydro-1,3-oxazin-3-yl, Tetrahydro-1,3-thiazin-3-yl, Tetrahydro-1,4-thiazin-4-yl, Tetrahydro-1,4-oxazin-4-yl, Tetrahydro-1,2-oxazin-2-yl.
C-gebundene, 5-gliedrige, partiell ungesättigte Ringe wie:
2,3-Dihydrofuran-2-yl, 2,3-Dihydrofuran-3-yl, 2,5-Dihydrofuran-2-yl, 2,5-Dihydrofuran-3-yl, 4,5-Dihydrofuran-2-yl, 4,5-Dihydrofuran-3-yl, 2,3-Dihydro-thien-2-yl, 2,3-Dihydrothien-3-yl, 2,5-Dihydrothien-2-yl, 2,5-Dihydrothien-3-yl, 4,5-Dihydrothien-2-yl, 4,5-Dihydrothien-3-yl, 2,3-Dihydro-1H-pyrrol-2-yl, 2,3-Dihydro-1H-pyrrol-3-yl, 2,5-Dihydro-1H-pyrrol-2-yl, 2,5-Dihydro-1H-pyrrol-3-yl, 4,5-Dihydro-1H-pyrrol-2-yl, 4,5-Dihydro-1H-pyrroi-3-yl, 3,4-Dihydro-2H-pyrrol-2-yl, 3,4-Dihydro-2H-pyrrol-3-yl, 3,4-Dihydro-5H-pyrrol-2-yl, 3,4-Dihydro-5H-pyrrol-3-yl, 4,5-Dihydro-1H-pyrazol-3-yl, 4,5-Dihydro-1H-pyrazol-4-yl, 4,5-Dihydro-1H-pyrazol-5-yl, 2,5-Dihydro-1H-pyrazol-3-yl, 2,5-Dihydro-1H-pyrazol-4-yl, 2,5-Dihydro-1H-pyrazol-5-yl, 4,5-Dihydroisoxazol-3-yl, 4,5-Dihydroisoxazol-4-yl, 4,5-Dihydroisoxazol-5-yl, 2,5-Dihydroisoxazol-3-yl, 2;5-Dihydroisoxazol-4-yl, 2,5-Dihydroisoxazol-5-yl, 2,3-Dihydroisoxazol-3-yl, 2,3-Dihydroisoxazol-4-yl, 2,3-Dihydroisoxazol-5-yl, 4,5-Dihydroisothiazol-3-yl, 4,5-Dihydroisothiazol-4-yl, 4,5-Dihydroisothiazol-5-yl, 2,5-Dihydroisothiazol-3-yl, 2,5-Dihydroisothiazol-4-yl, 2,5-Dihydroisothiazol-5-yl, 2,3-Dihydroisothiazol-3-yl, 2,3-Dihydroisothiazol-4-yl, 2,3-Dihydroisothiazol-5-yl, Δ ³-1,2-Dithiol-3-yl, Δ ³-1,2-Dithiol-4-yl, Δ ³-1,2-Dithiol-5-yl, 4,5-Dihydro-1H-imidazol-2-yl, 4,5-Dihydro-1H-imidazol-4-yl, 4,5-Dihydro-1H-imidazol-5-yl, 2,5-Dihydro-1H-imidazol-2-yl, 2,5-Dihydro-1H-imidazol-4-yl, 2,5-Dihydro-1H-imidazol-5-yl, 2,3-Dihydro-1H-imidazol-2-yl, 2,3-Dihydro-1H-imidazol-4-yl, 4,5-Dihydro-oxazol-2-yl, 4,5-Dihydrooxazol-4-yl, 4,5-Dihydrooxazol-5-yl, 2,5-Dihydro-oxazol-2-yl, 2,5-Dihydrooxazol-4-yl, 2,5-Dihydrooxazol-5-yl, 2,3-Dihydrooxazol-2-yl, 2,3-Dihydrooxazol-4-yl, 2,3-Dihydrooxazol-5-yl, 4,5-Dihydro-thiazol-2-yl, 4,5-Dihydrothiazol-4-yl, 4,5-Dihydrothiazol-5-yl, 2,5-Dihydro-thiazol-2-yl, 2,5-Dihydrothiazol-4-yl, 2,5-Dihydrothiazol-5-yl, 2,3-Dihydro-thiazol-2-yl, 2,3-Dihydrothiazol-4-yl, 2,3-Dihydrothiazol-5-yl, 1,3-Dioxol-2-yl, 1,3-Dioxol-4-yl, 1,3-Dithiol-2-yl, 1,3-Dithiol-4-yl, 1,3-Oxathiol-2-yl, 1,3-Oxathiol-4-yl, 1,3-Oxathiol-5-yl, 1,2,3-Δ ²-Oxadiazolin-4-yl, 1,2,3-Δ ²-Oxadiazolin-5-yl, 1,2,4-Δ ⁴-Oxadiazolin-3-yl, 1,2,4-Δ ⁴-Oxadiazolin-5-yl, 1,2,4-Δ ²-Oxadiazolin-3-yl, 1,2,4-Δ ²-Oxadiazolin-5-yl, 1,2,4-Δ ³-Oxadiazolin-3-yl, 1,2,4-Δ ³-Oxadiazolin-5-yl, 1,3,4-Δ ²-Oxadiazolin-2-yl, 1,3,4-Δ ²-Oxadiazolin-5-yl, 1,3,4-Δ ³-Oxadiazolin-2-yl, 1,3,4-Oxadiazolin-2-yl, 1,2,4-Δ ⁴-Thiadiazolin-3-yl, 1,2,4-Δ ⁴-Thiadiazolin-5-yl, 1,2,4-Δ ³-Thiadiazolin-3-yl, 1,2,4-A ³-Thiadiazolin-5-yl, 1,2,4-Δ ²-Thiadiazolin-3-yl, 1,2,4-Δ ²-Thiadiazolin-5-yl, 1,3,4-Δ ²-Thiadiazolin-2-yl, 1,3,4-Δ ²-Thiadiazolin-5-yl, 1.3,4-Δ ³-Thiadiazolin-2-yl, 1,3,4-Thiadiazolin-2-yl, 1,2,3-Δ ²-Triazolin-4-yl, 1,2,3-Δ ²-Triazolin-5-yl, 1,2,4-Δ ²-Triazolin-3-yl, 1,2,4-Δ ²-Triazolin-5-yl, 1,2,4-Δ ³-Triazolin-3-yl, 1,2,4-Δ ³-Triazolin-5-yl, 1,2,4-Δ ¹-Triazolin-2-yl, 1,2,4-Triazolin-3-yl, 3H-1,2,4-Dithiazol-5-yl, 2H-1,3,4-Dithiazol-5-yl, 2H-1,3,4-Oxathiazol-5-yl.
C-gebundene, 6-gliedrige, partiell ungesättigte Ringe wie:
2H-3,4-Dihydropyran-6-yl, 2H-3,4-Dihydropyran-5-yl, 2H-3,4-Dihydropyran-4-yl, 2H-3,4-Dihydropyran-3-yl, 2H-3,4-Dihydropyran-2-yl, 2H-3,4-Dihydrothiopyran-6-yl, 2H-3,4-Dihydrothiopyran-5-yl, 2H-3,4-Dihydrothiopyran-4-yl, 2H-3,4-Dihydrothiopyran-3-yl, 2H-3,4-Dihydrothiopyran-2-yl, 1,2,3,4-Tetrahydropyridin-6-yl, 1,2,3,4-Tetrahydropyridin-5-yl, 1,2,3,4-Tetrahydropyridin-4-yl, 1,2,3,4-Tetrahydropyridin-3-yl, 1,2,3,4-Tetrahydropyridin-2-yl, 2H-5,6-Dihydropyran-2-yl, 2H-5,6-Dihydropyran-3-yl, 2H-5,6-Dihydropyran-4-yl, 2H-5,6-Dihydropyran-5-yl, 2H-5,6-Dihydropyran-6-yl, 2H-5,6-Dihydrothiopyran-2-yl, 2H-5,6-Dihydro-thiopyran-3-yl, 2H-5,6-Dihydrothiopyran-4-yl, 2H-5,6-Dihydrothiopyran-5-yl, 2H-5,6-Dihydrothiopyran-6-yl, 1,2,5,6-Tetrahydropyridin-2-yl, 1,2,5,6-Tetrahydropyridin-3-yl, 1,2,5,6-Tetrahydropyridin-4-yl, 1,2,5,6-Tetrahydro-pyridin-5-yl, 1,2,5,6-Tetrahydropyridin-6-yl, 2,3,4,5-Tetrahydropyridin-2-yl, 2,3,4,5-Tetrahydropyridin-3-yl, 2,3,4,5-Tetrahydropyridin-4-yl, 2,3,4,5-Tetrahydropyridin-5-yl, 2,3,4,5-Tetrahydropyridin-6-yl, 4H-Pyran-2-yl, 4H-Pyran-3-yl-, 4H-Pyran-4-yl, 4H-Thiopyran-2-yl, 4H-Thiopyran-3-yl, 4H-Thiopyran-4-yl, 1,4-Dihydropyridin-2-yl, 1,4-Dihydropyridin-3-yl, 1,4-Dihydropyridin-4-yl, 2H-Pyran-2-yl, 2H-Pyran-3-yl, 2H-Pyran-4-yl, 2H-Pyran-5-yl, 2H-Pyran-6-yl, 2H-Thiopyran-2-yl, 2H-Thiopyran-3-yl, 2H-Thiopyran-4-yl, 2H-Thiopyran-5-yl, 2H-Thiopyran-6-yl, 1,2-Dihydropyridin-2-yl, 1,2-Dihydro-pyridin-3-yl, 1,2-Dihydropyridin-4-yl, 1,2-Dihydropyridin-5-yl, 1,2-Dihydro-pyridin-6-yl, 3,4-Dihydropyridin-2-yl, 3,4-Dihydropyridin-3-yl, 3,4-Dihydropyridin-4-yl, 3,4-Dihydropyridin-5-yl, 3,4-Dihydropyridin-6-yl, 2,5-Dihydro-pyridin-2-yl, 2,5-Dihydropyridin-3-yl, 2,5-Dihydropyridin-4-yl, 2,5-Dihydro-pyridin-5-yl, 2,5-Dihydropyridin-6-yl, 2,3-Dihydropyridin-2-yl, 2,3-Dihydro-pyridin-3-yl, 2,3-Dihydropyridin-4-yl, 2,3-Dihydropyridin-5-yl, 2,3-Dihydro-pyridin-6-yl, 2H-5,6-Dihydro-1,2-oxazin-3-yl, 2H-5,6-Dihydro-1,2-oxazin-4-yl, 2H-5,6-Dihydro-1,2-oxazin-5-yl, 2H-5,6-Dihydro-1,2-oxazin-6-yl, 2H-5,6-Dihydro-1,2-thiazin-3-yl, 2H-5,6-Dihydro-1,2-thiazin-4-yl, 2H-5,6-Dihydro-1,2-thiazin-5-yl, 2H-5,6-Dihydro-1,2-thiazin-6-yl, 4H-5,6-Dihydro-1,2-oxazin-3-yl, 4H-5,6-Dihydro-1,2-oxazin-4-yl, 4H-5,6-Dihydro-1,2-oxazin-5-yl, 4H-5,6-Dihydro-1,2-oxazin-6-yl, 4H-5,6-Dihydro-1,2-thiazin-3-yl, 4H-5,6-Dihydro-1,2-thiazin-4-yl, 4H-5,6-Dihydro-1,2-thiazin-5-yl, 4H-5,6-Dihydro-1,2-thiazin-6-yl, 2H-3,6-Dihydro-1,2-oxazin-3-yl, 2H-3,6-Dihydro-1,2-oxazin-4-yl, 2H-3,6-Dihydro-1,2-oxazin-5-yl, 2H-3,6-Dihydro-1,2-oxazin-6-yl, 2H-3,6-Dihydro-1,2-thiazin-3-yl, 2H-3,6-Dihydro-1,2-thiazin-4-yl, 2H-3,6-Di-hydro-1,2-thiazin-5-yl, 2H-3,6-Dihydro-1,2-thiazin-6-y!, 2H-3,4-Dihydro-1,2-oxazin-3-yl, 2H-3,4-Dihydro-1,2-oxazin-4-yl, 2H-3,4-Dihydro-1,2-oxazin-5-yl, 2H-3,4-Dihydro-1,2-oxazin-6-yl, 2H-3,4-Dihydro-1,2-thiazin-3-yl, 2H-3,4-Dihydro-1,2-thiazin-4-yl, 2H-3,4-Dihydro-1,2-thiazin-5-yl, 2H-3,4-Dihydro-1,2-thiazin-6-yl, 2,3,4,5-Tetrahydropyridazin-3-yl, 2,3,4,5-Tetrahydropyridazin-4-yl, 2,3,4,5-Tetrahydropyridazin-5-yl, 2,3,4,5-Tetrahydropyridazin-6-yl, 3,4,5,6-Tetrahydropyridazin-3-yl, 3,4,5,6-Tetrahydropyhdazin-4-yl, 1,2,5,6-Tetrahydropyridazin-3-yl, 1,2,5,6-Tetrahydropyridazin-4-yl, 1,2,5,6-Tetrahydropyridazin-5-yl, 1,2,5,6-Tetrahydropyridazin-6-yl, 1,2,3,6-Tetrahydropyridazin-3-yl, 1,2,3,6-Tetrahydropyridazin-4-yl, 4H-5,6-Dihydro-1,3-oxazin-2-yl, 4H-5,6-Dihydro-1,3-oxazin-4-yl, 4H-5,6-Dihydro-1,3-oxazin-5-yl, 4H-5,6-Dihydro-1,3-oxazin-6-yl, 4H-5,6-Dihydro-1,3-thiazin-2-yl, 4H-5,6-Dihydro-1,3-thiazin-4-yl, 4H-5,6-Dihydro-1,3-thiazin-5-yl, 4H-5,6-Dihydro-1,3-thiazin-6-yl, 3,4,5-6-Tetrahydropyrimidin-2-yl, 3,4,5,6-Tetrahydropyrimidin-4-yl, 3,4,5,6-Tetrahydropyrimidin-5-yl, 3,4,5,6-Tetrahydropyrimidin-6-yl, 1,2,3,4-Tetrahydropyrazin-2-yl, 1,2,3,4-Tetrahydropyrazin-5-yl, 1,2,3,4-Tetrahydropyrimidin-2-yl, 1,2,3,4-Tetrahydropyrimidin-4-yl, 1,2,3,4-Tetrahydropyrimidin-5-yl, 1,2,3,4-Tetrahydropyrimidin-6-yl, 2,3-Dihydro-1,4-thiazin-2-yl, 2,3-Dihydro-1,4-thiazin-3-yl, 2,3-Dihydro-1,4-thiazin-5-yl, 2,3-Dihydro-1,4-thiazin-6-yl, 2H-1,2-Oxazin-3-yl, 2H-1,2-Oxazin-4-yl, 2H-1,2-Oxazin-5-yl, 2H-1,2-Oxazin-6-yl, 2H-1,2-Thiazin-3-yl, 2H-1,2-Thiazin-4-yl, 2H-1,2-Thiazin-5-yl, 2H-1,2-Thiazin-6-yl, 4H-1,2-Oxazin-3-yl, 4H-1,2-Oxazin-4-yl, 4H-1,2-Oxazin-5-yl, 4H-1,2-Oxazin-6-yl, 4H-1,2-Thiazin-3-yl, 4H-1,2-Thiazin-4-yl, 4H-1,2-Thiazin-5-yl, 4H-1,2-Thiazin-6-yl, 6H-1,2-Oxazin-3-yl, 6H-1,2-Oxazin-4-yl, 6H-1,2-Oxazin-5-yl, 6H-1,2-Oxazin-6-yl, 6H-1,2-Thiazin-3-yl, 6H-1,2-Thiazin-4-yl, 6H-1,2-Thiazin-5-yl, 6H-1,2-Thiazin-6-yl, 2H-1,3-Oxazin-2-yl, 2H-1,3-Oxazin-4-yl, 2H-1,3-Oxazin-5-yl, 2H-1,3-Oxazin-6-yl, 2H-1,3-Thiazin-2-yl, 2H-1,3-Thiazin-4-yl, 2H-1,3-Thiazin-5-yl, 2H-1,3-Thiazin-6-yl, 4H-1,3-Oxazin-2-yl, 4H-1,3-Oxazin-4-yl, 4H-1,3-Oxazin-5-yl, 4H-1,3-Oxazin-6-yl, 4H-1,3-Thiazin-2-yl, 4H-1,3-Thiazin-4-yl, 4H-1,3-Thiazin-5-yl, 4H-1,3-Thiazin-6-yl, 6H-1,3-Oxazin-2-yl, 6H-1,3-Oxazin-4-yl, 6H-1,3-Oxazin-5-yl, 6H-1,3-Oxazin-6-yl, 6H-1,3-Thiazin-2-yl, 6H-1,3-Oxazin-4-yl, 6H-1,3-Oxazin-5-yl, 6H-1,3-Thiazin-6-yl, 2H-1,4-Oxazin-2-yl, 2H-1,4-Oxazin-3-yl, 2H-1,4-Oxazin-5-yl, 2H-1,4-Oxazin-6-yl, 2H-1,4-Thiazin-2-yl, 2H-1,4-Thiazin-3-yl, 2H-1,4-Thiazin-5-yl, 2H-1,4-Thiazin-6-yl, 4H-1,4-Oxazin-2-yl, 4H-1,4-Oxazin-3-yl, 4H-1,4-Thiazin-2-yl, 4H-1,4-Thiazin-3-yl, 1,4-Dihydropyridazin-3-yl, 1,4-Dihydropyridazin-4-yl, 1,4-Dihydropyridazin-5-yl, 1,4-Dihydropyridazin-6-yl, 1,4-Dihydropyrazin-2-yl, 1,2-Dihydropyrazin-2-yl, 1,2-Dihydropyrazin-3-yl, 1,2-Dihydropyrazin-5-yl, 1,2-Dihydropyrazin-6-yl, 1,4-Dihydropyrimidin-2-yl, 1,4-Dihydropyrimidin-4-yl, 1,4-Dihydropyrimidin-5-yl, 1,4-Dihydropyrimidin-6-yl, 3,4-Dihydropyrimidin-2-yl, 3,4-Dihydropyrimidin-4-yl, 3,4-Dihydropyrimidin-5-yl oder 3,4-Dihydropyrimidin-6-yl.
N-gebundene, 5-gliedrige, partiell ungesättigte Ringe wie:
2,3-Dihydro-1H-pyrrol-1-yl, 2,5-Dihydro-1H-pyrrol-1-yl, 4,5-Dihydro-1H-pyrazol-1-yl, 2,5-Dihydro-1H-pyrazol-1-yl, 2,3-Dihydro-1H-pyrazol-1-yl, 2,5-Dihydroisoxazol-2-yl, 2,3-Dihydroisoxazol-2-yl, 2,5-Dihydroisothiazol-2-yl, 2,3-Dihydroisoxazol-2-yl, 4,5-Dihydro-1H-imidazol-1-yl, 2,5-Dihydro-1H-imidazol-1-yl, 2,3-Dihydro-1H-imidazol-1-yl, 2,3-Dihydrooxazol-3-yl, 2,3-Dihydrothiazol-3-yl, 1,2,4-Δ ⁴-Oxadiazolin-2-yl, 1,2,4-Δ ²-Oxadiazolin-4-yl, 1,2,4-Δ ³-Oxadiazolin-2-yl, 1,3,4-Δ ²-Oxadiazolin-4-yl, 1,2,4-Δ ⁵-Thiadiazolin-2-yl, 1,2,4-Δ ³-Thiadiazolin-2-yl, 1,2,4-Δ ²-Thiadiazolin-4-yl, 1,3,4-Δ ²-Thiadiazolin-4-yl, 1,2,3-Δ ²-Triazolin-1-yl, 1,2,4-Δ ²-Triazolin-1-yl, 1,2,4-Δ ²-Triazolin-4-yl, 1,2,4-Δ ³-Triazolin-1-yl, 1,2,4-Δ ¹-Triazolin-4-yl.
N-gebundene, 6-gliedrige, partiell ungesättigte Ringe wie:
1,2,3,4-Tetrahydropyridin-1-yl, 1,2,5,6-Tetrahydropyhdin-1-yl, 1,4-Dihydropyridin-1-yl, 1,2-Dihydropyridin-1-yl, 2H-5,6-Dihydro-1,2-oxazin-2-yl, 2H-5,6-Dihydro-1,2-thiazin-2-yl, 2H-3,6-Dihydro-1,2-oxazin-2-yl, 2H-3,6-Dihydro-1,2-thiazin-2-yl, 2H-3,4-Dihydro-1,2-oxazin-2-yl, 2H-3,4-Dihydro-1,2-thiazin-2-yl, 2,3,4,5-Tetrahydropyridazin-2-yl, 1,2,5,6-Tetrahydropyridazin-1-yl, 1,2,5,6-Tetrahydropyridazin-2-yl, 1,2,3,6-Tetrahydropyridazin-1-yl, 3,4,5,6-Tetrahydropyrimidin-3-yl, 1,2,3,4-Tetrahydropyrazin-1-yl, 1,2,3,4-Tetrahydropyrimidin-1-yl, 1,2,3,4-Tetrahydropyrimidin-3-yl, 2,3-Dihdro-1,4-thiazin-4-yl, 2H-1,2-Oxazin-2-yl, 2H-1,2-Thiazin-2-yl, 4H-1,4-Oxazin-4-yl, 4H-1,4-Thiazin-4-yl, 1,4-Dihydropyridazin-1-yl, 1,4-Dihydropyrazin-1-yl, 1,2-Dihydropyrazin-1-yl, 1,4-Dihydropyrimidin-1-yl oder 3,4-Dihydropyrimidin-3-yl.
C-gebundene, 5-gliedrige, heteroaromatische Ringe mit in der Regel 1, 2, 3 oder 4 Stickstoffatomen oder einem unter Sauerstoff und Schwefel ausgewählten Heteroatom und gegebenenfalls mit 1, 2 oder 3 Stickstoffatomen als Ringglieder wie:
2-Furyl, 3-Furyl, 2-Thienyl, 3-Thienyl, Pyrrol-2-yl, Pyrrol-3-yl, Pyrazol-3-yl, Pyrazol-4-yl, Isoxazol-3-yl, Isoxazol-4-yl, Isoxazot-5-yl, Isothiazol-3-yl, Isothiazol-4-yl, Isothiazol-5-yl, Imidazol-2-yl, Imidazol-4-yl, Oxazol-2-yl, Oxazol-4-yl, Oxazol-5-yl, Thiazol-2-yl, Thiazol-4-yl, Thiazol-5-yl, 1,2,3-Oxadiazol-4-yl, 1,2,3-Oxadiazol-5-yl, 1,2,4-Oxadiazol-3-yl, 1,2,4,-Oxadiazol-5-yl, 1,3,4-Oxadiazol-2-yl, 1,2,3-Thiadiazol-4-yl, 1,2,3-Thiadiazol-5-yl, 1,2,4-Thiadiazol-3-yl, 1,2,4-Thiadiazol-5-yl, 1,3,4-Thiadiazolyl-2-yl, 1,2,3-Triazol-4-yl, 1,2,4-Triazol-3-yl, Tetrazol-5-yl.
C-gebundene, 6-gliedrige, heteroaromatische Ringe mit in der Regel 1, 2, 3 oder 4 Stickstoffatomen als Ringglieder wie :
Pyridin-2-yl, Pyridin-3-yl, Pyridin-4-yl, Pyridazin-3-yl, Pyridazin-4-yl, Pyrimidin-2-yl, Pyrimidin-4-yl, Pyrimidin-5-yl, Pyrazin-2-yl, 1,3,5-Triazin-2-yl, 1,2,4-Triazin-3-yl, 1,2,4-Triazin-5-yl, 1,2,4-Triazin-6-yl, 1,2,4,5-Tetrazin-3-yl.
N-gebundene, 5-gliedrige, heteroaromatische Ringe mit in der Regel 1, 2, 3 oder 4 Stickstoffatomen als Ringglieder wie:
Pyrrol-1-yl, Pyrazol-1-yl, Imidazol-1-yl, 1,2,3-Triazol-1-yl, 1,2,4-Triazol-1-yl, Tetrazol-1-yl.
oder ein bicyclischer Heterocyclus, der einen der vorgenannten 5- oder 6-gliedrigen heterocyclischen Ringe und einen weiteren, daran ankondensierten, gesättigten, ungesättigten oder aromatischen Carbocyclus, beispielsweise einen Benzol-, Cyclohexan, Cyclohexen oder Cyclohexadien-Ring, oder einen weiteren, daran ankondensierten 5-oder 6-gliedrigen heterocyclischen Ring aufweist, wobei letzterer ebenfalls gesättigt, ungesättig oder aromatisch sein kann.

Ein Schwefelatom in den genannten Heterocyclen kann zu S=O oder S(=O)₂ oxidiert sein.

Dementsprechend steht Hetaryl bzw. Heteroaryl für einen 5- oder 6-gliedrigen heteroaromatischen Rest, der 1, 2, 3 oder 4 gleiche oder verschiedene Heteroatome, ausgewählt aus der Gruppe Sauerstoff, Schwefel oder Stickstoff, als Ringglieder aufweist, der über C oder N gebunden sein kann, und der mit einem weiteren ankondensierten Benzolring oder einem 5- bis 6-gliedrigen Heteroaromaten ein bicyclisches Ringsystem ausbilden kann. Beispiele für Hetaryl sind die vorgenannten C-gebundenen, 5- und 6-gliedrigen, heteroaromatischen Ringe, die vorgenannten N-gebundenen, 5-gliedrigen, heteroaromatischen Ringe, und bicyclische heteroaramatische Reste wie Chinolinyl, Isochinolinyl, Chinazolinyl, Chinoxalinyl, Indolyl, Benzothienyl, Benzofuryl, Benzoxazolyl, Benzthiazolyl, Benzimidazolyl, Benzpyrazolyl, Benzotriazol, Indolizinyl, 1,2,4-Triazolo[1,5-a]pyrimidinyl, 1,2,4-Triazolo[4,3-a]pyridinyl, Pyrazolo[3,4-b]pyridinyl, 1,2,4-Triazolo[1,5-a]pyridinyl, Imidazo[1,2-a]pyridyl, Imidazo[3,4-a]pyrimidinyl, und dergleichen.

Aryl: ein- oder mehrkerniger aromatischer Carbocyclus, z. B. ein- bis zweikerniger oder ein- bis dreikerniger aromatischer Carbocyclus mit 6 bis 14 Ringgliedern, wie z. B. Phenyl, Naphthyl oder Anthracenyl.

Arylalkyl: ein über eine Alkylengruppe, insbesondere über eine Methylen-, 1,1-Ethylen- oder 1,2-Ethylengruppe gebundener Arylrest, z.B. Benzyl, 1-Phenylethyl und 2-Phenylethyl.

Heterocyclylalkyl sowie Hetarylalkyl: ein über eine Alkylengruppe, insbesondere über eine Methylen-, 1,1-Ethylen- oder 1,2-Ethylengruppe gebundener Heterocyclyl- bzw. Hetarylrest.

In einer besonderen Ausführungsform haben die Variablen der Verbindungen der Formel I folgende Bedeutungen, wobei diese sowohl für sich allein betrachtet als auch in Kombination miteinander besondere Ausgestaltungen der Verbindungen der Formel I darstellen:
- R¹: Wasserstoff, Amino, Cyano, C₁-C₆-Alkyl, C₃-C₆-Alkenyl, C₃-C₆-Alkinyl, Phenyl-(C₁-C₆)-alkyl, Heterocyclyl-(C₁-C₆)-alkyl oder COR²¹, wobei R²¹ die zuvor genannten Bedeutunge aufweist und insbesondere für C₁-C₆-Alkyl, C₁-C₆-Alkoxy, Phenyl, Phenylamino oder Heterocyclyl steht; wobei die genannten aliphatischen, cyclischen oder aromatischen Teile der Substituenten partiell oder vollständig halogeniert sein können. Besonders bevorzugt hat R¹ die Bedeutungen Wasserstoff oder C₁-C₆-Alkyl, insbesondere Methyl.
- R²: Amino, Cyano, C₁-C₆-Alkyl, C₃-C₆-Alkenyl, C₃-C₆-Alkinyl, Phenyl-(C₁-C₆)-alkyl, Heterocyclyl-(C₁-C₆)-alkyl oder COR²¹, wobei R²¹ die zuvor genannten Bedeutungen aufweist und insbesondere für C₁-C₆-Alkyl, C₁-C₆-Alkoxy, Phenyl, Phenylamino oder Heterocyclyl steht; wobei die genannten aliphatischen, cyclischen oder aromatischen Teile der Substituenten partiell oder vollständig halogeniert sein können. Besonders bevorzugt hat R² die Bedeutung C₁-C₆-Alkyl, insbesondere Methyl.
- R³: ein Rest R²⁶ oder eine Gruppe OR²⁷, wobei R²⁶ und R²⁷ die zuvor genannten Bedeutungen aufweisen und insbesondere unabhängig voneinander Wasserstoff, C₁-C₆-Alkyl, C₁-C₆-Alkylcarbonyl, Phenyl-C₁-C₆-alkyl oder Phenylcarbonyl bedeuten , wobei die genannten aliphatischen oder aromatischen Teile der Substituenten partiell oder vollständig halogeniert sein können, oder SO₂R³¹ bedeuten, wobei R³¹ C₁-C₆-Alkyl oder Phenyl bedeutet, und wobei der Phenylsubstituent in R³¹ partiell oder vollständig halogeniert sein kann und/oder eine bis drei C₁-C₆-Alkyl-Gruppen tragen kann. Besonders bevorzugt hat R³ die Bedeutungen Wasserstoff, C₁-C₆-Alkyl, Phenyl-C₁-C₆-alkoxy oder C₁-C₆-Alkylsulfonyl.
R⁴, R⁵ und/oder R⁶ Wasserstoff.
R³ und R⁴ können auch zusammen eine Ketogruppe bedeuten. Vorzugsweise stehen dann R⁵ und/oder R⁶ für Wasserstoff.
R⁷ und R⁸ bedeuten vorzugsweise unabhängig voneinander Wasserstoff oder Methyl, insbesondere Wasserstoff.
A¹, A² stehen unabhängig voneinander für Aryl oder Heteroaryl, ausgewählt aus der Gruppe Phenyl, Naphthyl, Furyl, Thienyl, Pyrrolyl, Pyrazolyl, Thiazolyl, Isothiazolyl, Oxazolyl, Isoxazolyl, Triazolyl, Tetrazolyl, Pyridinyl, Pyridazinyl, Pyrimidinyl, Pyrazinyl, Triazinyl oder Tetrazinyl, insbesondere ausgewählt aus der Gruppe Phenyl, Furyl, Thienyl, Triazolyl, Tetrazolyl oder Pyridinyl. Besonders bevorzugt hat A¹ die Bedeutung Phenyl oder Pyridinyl, insbesondere Phenyl. Besonders bevorzugt hat A² die Bedeutung Phenyl oder Thienyl, insbesondere Phenyl. Erfindungsgemäß weist A¹ einen bis drei von Wasserstoff verschiedene Substituenten R^{a}, R^{b}, R^{c} auf, wobei R^{a} in ortho-Position zur Verknüpfungsstelle von A¹ an ein N- oder ein C-Atom von A¹ gebunden ist, wobei R^{a} bevorzugt eine der folgenden Bedeutungen aufweist:
- Halogen, Cyano, Nitro, C₁-C₆-Alkyl, C₃-C₆-Cycloalkyl, C₂-C₆-Alkenyl, C₂-C₆-Alkinyl, [Tri-(C₁-C₆)-alkylsilyl]-(C₂-C₆)-alkinyl, C₁-C₆-Alkylthio, C₁-C₆-Alkylsulfinyl, Aryl, Heterocyclyl, insbesondere 5- oder 6-gliedriges Heterocyclyl, wobei Aryl und Heterocyclyl unsubstituiert sind oder einen oder 2 Reste aufweisen können, die unter C₁-C₄-Alkyl, C₁-C₄-Halolkyl, C₁-C₄-Alkoxy, C₁-C₄-Haloalkoxy, CN, Phenyl und Halogen ausgewählt sind,
- Z¹P(O)(OR⁹)₂, wobei Z¹ eine Bindung oder -CH₂- bedeutet und R⁹ jeweils Wasserstoff oder C₁-C₆-Alkyl bedeuten;
- Z³COR¹¹, wobei Z³ eine Bindung bedeutet und R¹¹ die zuvorgenannten Bedeutungen hat und insbesondere Wasserstoff, C₁-C₆-Alkyl, Hydroxy, C₁-C₆-Alkoxy, C₃-C₆-Alkenyloxy, C₃-C₆-Alkinyloxy, Amino, C₁-C₆-Alkylamino, [Di-(C₁-C₆)-alkyl]amino, C₁-C₆-Alkoxyamino, N-C₁-C₆-Alkoxy-N-C₁-C₆-alkylamino, [Di-(C₁-C₆)-Alkoxy]amino, C₁-C₆-Alkylsulfonylamino, C₁-C₆-Alkylaminosulfonylamino, [Di-(C₁-C₆)-alkylamino]sulfonylamino, Phenyl, Phenoxy, Phenylamino, Naphthyl oder Heterocyclyl, speziell C-gebundenes 5- oder 6-gliedrige Heteroaryl, bedeutet;
- Z⁴NR¹²R¹³, wobei Z⁴ eine Bindung oder -CH₂- bedeutet und R¹² und R¹³ die zuvorgenannten Bedeutungen haben und insbesondere unabhängig voneinander Wasserstoff, C₁-C₆-Alkyl, C₃-C₆-Cycloalkyl. C₃-C₆-Alkenyl, C₃-C₆-Alkinyl, C₁-C₆-Alkylcarbonyl, [Di-(C₁-C₆)-alkylamino]carbonyl, C₁-C₆-Alkoxycarbonyl, C₁-C₆-Alkylsulfonyl, C₁-C₆-Alkylaminosulfonyl, [Di-(C₁-C₆)-alkylamino]sulfonyl, C₃-C₆-Cycloalkylcarbonyl, Phenylcarbonyl, Phenylaminocarbonyl, Phenylsulfonyl, Phenylsulfonylaminocarbonyl,oder Heterocyclylcarbonyl, speziell C-gebundenes 5- oder 6-gliedriges Heteroarylcarbonyl, bedeuten;
- Z⁵CH=N-O-R¹⁴, wobei Z⁵ eine Bindung bedeutet und R¹⁴ Wasserstoff oder C₁-C₆-Alkyl bedeutet; oder
- Z⁶OR¹⁵, wobei Z⁶ eine Bindung oder -CH₂- bedeutet und R¹⁵ die zuvorgenannten Bedeutungen hat und insbesondere C₃-C₆-Cycloalkyl, C₃-C₆-Alkenyl, C₃-C₆-Alkinyl, C₁-C₆-Alkylcarbonyl, C₁-C₆-Alkoxycarbonyl-(C₁-C₆)-alkyl, Di-(C₁-C₆-Alkoxycarbonyl)-C₁-C₆-alkyl, Phenylcarbonyl, Phenyl oder Phenyl-(C₁-C₆)-Alkyl bedeutet, R¹⁵ auch Wassestoff oder C₁-C₆-Alkyl bedeuten kann; oder
- Z⁷SO₂R¹⁶, wobei Z⁷ eine Bindung oder CH₂ bedeutet und R¹⁶ C₁-C₆-Alkyl oder Phenyl;
wobei die genannten aliphatischen, cyclischen oder aromatischen Teile der Substituenten R^{a} partiell oder vollständig halogeniert sein können.
R^{b}, R^{c}, R^{d}, R^{e} und R^{f} stehen vorzugsweise für Wasserstoff oder haben unabhängig voneinander eine der für R^{a} als bevorzugt genannten Bedeutungen.
Sofern R^{a} an ein Stickstoffatom gebunden ist, ist R^{a} vorzugsweise von Halogen, C₁-C₆-Alkylthio, C₁-C₆-Alkylsulfinyl, Z¹P(O)(OR⁹)₂, worin Z¹ für eine Bindung steht, verschieden. In einer bevorzugten Ausführungsform der Erfindung ist R^{a} an ein C-Atom gebunden.

Insbesondere weist R^{a} eine der folgenden Bedeutungen auf:
- Halogen, Cyano, Nitro, C₁-C₆-Alkylthio, C₁-C₆-Alkylsulfinyl, Aryl, Heterocyclyl,
wobei die zwei letzgenannten Reste unsubstituiert sind oder einen oder 2 Reste aufweisen können, die unter C₁-C₄-Alkyl, C₁-C₄-Halolkyl, C₁-C₄-Alkoxy, C₁-C₄-Haloalkoxy, CN, Phenyl und Halogen ausgewählt sind,
- C₂-C₆-Alkenyl, C₂-C₆-Alkinyl,
- Z¹P(O)(OR⁹)₂, wobei Z¹ eine Bindung oder -CH₂- bedeutet und R⁹ jeweils Wasserstoff oder C₁-C₆-Alkyl bedeuten; oder
- Z³COR¹¹, wobei Z³ eine Bindung bedeutet und R¹¹ Wasserstoff, C₁-C₆-Alkyl, Hydroxy, C₁-C₆-Alkoxy, C₃-C₆-Alkenyloxy, C₃-C₆-Alkinyloxy, Amino, C₁-C₆-Alkylamino, [Di-(C₁-C₆)-alkyl]amino, C₁-C₆-Alkoxyamino, N-C₁-C₆-Alkoxy-N-C₁-C₆-alkylamino, C₁-C₆-Alkylsulfonylamino, C₁-C₆-Alkylaminosulfonylamino, [Di-(C₁-C₆)-alkylamino]sulfonylamino, Phenyl, Phenoxy, Phenylamino, Naphthyl oder Heterocyclyl, speziell C-gebundenes 5- oder 6-gliedriges Heteroaryl, bedeutet; oder
- Z⁴NR¹²R¹³, wobei Z⁴ eine Bindung oder -CH₂- bedeutet und R¹² und R¹³ unabhängig voneinander Wasserstoff, C₁-C₆-Alkyl, C₃-C₆-Cycloalkyl, C₃-C₆-Alkenyl, C₃-C₆-Alkinyl, C₁-C₆-Alkylcarbonyl, [Di-(C₁-C₆)-alkylamino]carbonyl, C₁-C₆-Alkoxycarbonyl, C₁-C₆-Alkylsulfonyl, Phenylcarbonyl, Phenylsulfonyl, oder Heterocyclylcarbonyl, speziell C-gebundenes 5- oder 6-gliedriges Heteroarylcarbonyl, bedeuten; oder
- Z⁵CH=N-O-R¹⁴, wobei Z⁵ eine Bindung bedeutet und R¹⁴ Wasserstoff oder C₁-C₆-Alkyl bedeutet; oder
- Z⁶OR¹⁵, wobei Z⁶ eine Bindung oder -CH₂- bedeutet und R¹⁵ Wasserstoff, C₁-C₆-Alkyl, C₃-C₆-Cycloalkyl, C₃-C₆-Alkenyl, C₃-C₆-Alkinyl, C₁-C₆-Alkylcarbonyl, C₁-C₆-Alkoxycarbonyl-(C₁-C₆)-alkyl, Phenyl oder Phenyl-(C₁-C₆)-Alkyl bedeutet; oder
- Z⁷SO₂R¹⁶, wobei Z⁷ eine Bindung oder CH₂ bedeutet und R¹⁶C₁-C₆-Alkyl oder Phenyl;
und wobei die genannten aliphatischen, cyclischen oder aromatischen Teile der Substituenten R^{a} partiell oder vollständig halogeniert sein können.

Ganz speziell bevorzugt steht R^{a} für einen Rest, der ausgewählt ist unter Halogen, Cyano, Nitro, C₂-C₄-Alkenyl und C₂-C₄-Alkinyl, NH-C(O)-C₁-C₆-Alkyl, NH-S(O)₂-C₁-C₆-Alkyl und 5 gliedrigem Heteroaryl, z.B. Oxazolyl, Thiazolyl, Isoxazolyl, Isothiazolyl, wobei die vorgeanntne Heteroarylreste einen oder 2 Reste aufweisen können, die unter C₁-C₂-Alkyl, C₁-C₂-Haloalkyl und Halogen ausgewählt sind, und der insbesondere in einer der ortho-Postionen von A¹ gebunden ist.

R^{b}, R^{c}, R^{d}, R^{e} und R^{f} stehen insbesondere für Wasserstoff oder haben unabhängig voneinander eine der für R^{a} als besonders bevorzugt genannten Bedeutungen oder stehen für: C₁-C₆-Alkyl, C₁-C₆-Halogenalkyl, C₃-C₆-Cycloalkyl, das partiell oder vollständig halogeniert sein kann, C₂-C₆-Alkenyl, das partiell oder vollständig halogeniert sein kann, [Tri-(C₁-C₆)-alkylsilyl]-(C₂-C₆)-alkinyl, oder eine Gruppe Z⁶OR¹⁵, worin Z⁶ eine Bindung bedeutet und R¹⁵ Wasserstoff, C₁-C₆-Alkyl oder Halogenalkyl bedeutet.

Insbesondere sind die Reste R^{b}, R^{c}, R^{d}, R^{e} und R^{f} unabhängig voneiander ausgewählt unter Wasserstoff, Halogen, C₁-C₄-Alkyl, C₂-C₄-Alkenyl, C₂-C₄-Alkinyl, C₁-C₄-Alkoxy, C₁-C₄-Halogenalkyl und C₁-C₄-Halogenalkoxy, wobei zwei an benachbarte C-Atome von A¹ oder A² gebundene Gruppen R^{b}, R^{c}, R^{d}, R^{e} oder R^{f} auch für eine Gruppe O-CH₂-O stehen können.

Insbesondere ist R^{b} ein von Wasserstoff verschiedener Rest. Vorzugsweise ist R^{b} ein in der ortho-Position von A¹, gebundener Rest, d.h. wenn R^{a} ebenfalls in der ortho-Position gebunden ist, befindet sich R^{b} in der zweiten ortho-Position.

Sofern einer oder beide Reste R^{b}, R^{c} für einen von Wasserstoff verschiedenen Substituenten stehen, sind sie insbesondere ausgewählt unter den als bevorzugt angegebenen Substituenten, und speziell unter Halogen, C₁-C₄-Alkyl, C₁-C₄-Alkoxy, C₁-C₄-Halogenalkyl, C₂-C₄-Alkenyl, C₂-C₄-Alkinyl oder C₁-C₄-Halogenalkoxy oder R^{b} und R^{c} stehen gemeinsam für eine Gruppe O-CH₂-O.

Insbesondere ist A² unsubstituiert oder einer oder zwei der Substituenten R^{d}, R^{e} bzw. R^{f} stehen für einen von Wasserstoff verschiedenen Substituenten. Sofern 1 oder 2 der Substituenten R^{d}, R^{e} und R^{f} von Wasserstoff verschieden sind , sind sie insbesondere ausgewählt unter Halogen, C₁-C₄-Alkyl, C₁-C₄-Alkoxy, C₁-C₄-Halogenalkyl und C₁-C₄-Halogenalkoxy.

Die Bedeutung, die ein Substituent im Rahmen der Erfindung annehmen kann, ist vollkommen unabhängig von der Bedeutung, die ein anderer Substituent im Rahmen der Erfindung annehmen kann.

Ein spezieller Gegenstand der Erfindung sind Piperazinverbindungen der allgemeinen Formel I, worin A¹ und A² Phenyl bedeuten, R¹ für Methyl steht und R² Wasserstoff, C₁-C₆-Alkyl, C₂-C₆-Alkenyl, C₂-C₆-Alkinyl, Aryl oder C₁-C₆-Alkoxy, insbesondere Wasserstoff oder Methyl bedeutet, die Substituenten R^{a} und R^{b} unabhängig voneinander jeweils für Wasserstoff, Hydroxy, C₁-C₆-Alkyl, C₂-C₆-Alkenyl, C₂-C₆-Alkinyl oder C₁-C₆-Alkoxy stehen, die in den beiden ortho-Positionen des Phenylrings A¹ angeordnet sind, R³, R⁴, R⁵, R⁶, R⁷, R⁸ Wasserstoff bedeuten und R^{c}, R^{d}, R^{e} und R^{f} ebenfalls Wasserstoff bedeuten. Hierunter bevorzugt sind solche Verbindungen, worin R^{a} für C₂-C₆-Alkenyl oder C₂-C₆-Alkinyl steht.

Ein weiterer Gegenstand der Erfindung sind Piperazinverbindungen der Formel I, die von den Verbindungen des zuvor genannten speziellen Gegenstands verschieden sind, d.h. Verbindungen der allgemeinen Formel I, ausgenommen solche Verbindungen der Formel I, worin R¹ für Methyl steht und R² für Wasserstoff, C₁-C₆-Alkyl, C₂-C₆-Alkenyl, C₂-C₆-Alkinyl, Aryl oder C₁-C₆-Alkoxy, insbesondere für Wasserstoff oder Methyl steht, die Substituenten R^{a} und R^{b} unabhängig voneinander jeweils für Wasserstoff, Hydroxy, C₁-C₆-Alkyl, C₂-C₆-Alkenyl, C₂-C₆-Alkinyl oder C₁-C₆-Alkoxy stehen, die in den beiden ortho-Positionen des Phenylrings A¹ angeordnet sind, R³, R⁴, R⁵, R⁶, R⁷, R⁸ Wasserstoff bedeuten und R^{c}, R^{d}, R^{e} und R^{f} ebenfalls Wasserstoff bedeuten.

Ein weiterer bevorzugter Gegenstand der Erfindung sind diejenigen Verbindungen der Formel I (S,S), die an den gekennzeichneten Positionen (1) und (2) jeweils S-Konfiguration haben.

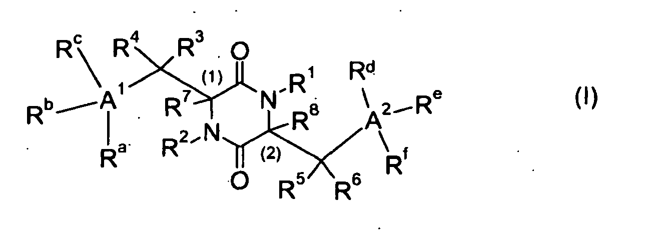

Bevorzugt sind die Verbindungen der Formel 1.1, bei denen R³, R⁴, R⁵, R⁶, R⁷ und R⁸ Wasserstoff sind und R² CH₃ ist, besonders bevorzugt die Verbindungen (S,S)-1.1, die an den gekennzeichneten Positionen (1) und (2) jeweils S-Konfiguration haben.

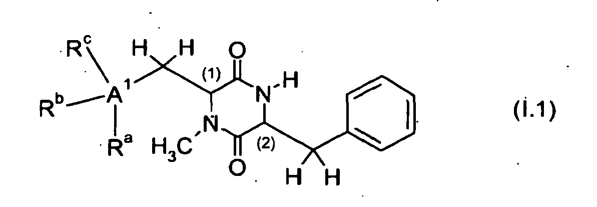

Die Piperazinverbindungen der Formel I können nach Standardmethoden der Synthese organischer Verbindungen auf verschiedene Art und Weise herstellt werden, beispielsweise nach den im Folgenden näher erläuterten Verfahren:

### Verfahren A

Die Verbindungen der Formel I können beispielsweise in Analogie zu literaturbekannten Verfahren durch Cyclisierung entsprechender Dipeptid-Vorstufen der Formel II hergestellt werden, beispielsweise in Analogie zu der von T. Kawasaki et al., Org. Lett. 2(19) (2000), 3027-3029, Igor L. Rodionov et al., Tetrahedron 58(42) (2002), 8515-8523 oder A. L. Johnson et al., Tetrahedron 60 (2004), 961-965 beschriebenen Methode. Die Cyclisierung von Dipeptiden der Formel II zu den erfindungsgemäßen Verbindungen wird im Folgenden auch als Verfahren A bezeichnet und ist in dem nachfolgenden Schema skizziert.

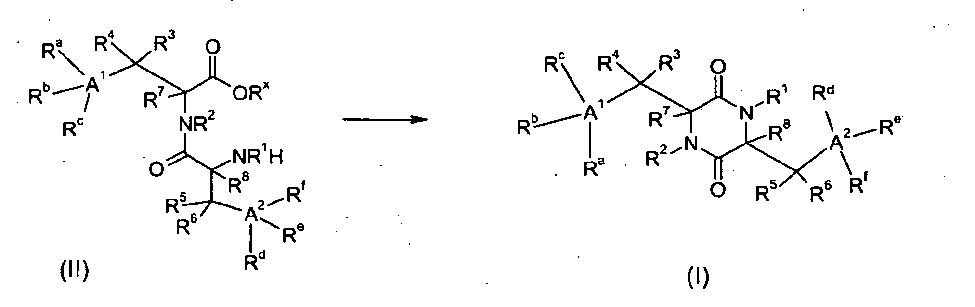

In Formel II haben die Variablen A¹, A², R¹ - R⁸, R^{a}, R^{b}, R^{c}, R^{d}, R^{e} und R^{f} die für Formel I angegebene Bedeutung. Die Gruppe OR^{x} steht für eine geeignete, über Sauerstoff gebundene Abgangsgruppe. R^{x} ist hierbei z.B. C₁-C₆-Alkyl, insbesondere Methyl, Ethyl oder Phenyl-C₁-C₆-alkyl, z.B. Benzyl. Dipeptide der allgemeinen Formel II sind neu und ebenfalls Gegenstand der vorliegenden Erfindung.

Die Cyclisierung kann beispielsweise durch Umsetzung eines Dipeptids der Formel II, entweder in Gegenwart von Säure oder Base (saure oder basische Cyclisierung) oder durch Erhitzen der Reaktionsmischung (thermische Cycilsierung) bewirkt werden.

Die Basen oder Säuren werden entweder in äquimolaren Mengen zum Dipeptid II oder im Überschuss eingesetzt. In einer besonderen Ausführungsform des erfindungsgemäßen Verfahrens werden die Basen oder Säuren in einem Überschuß bezogen auf das Dipeptid verwendet.

Die Umsetzung des Dipeptids II in Gegenwart einer Base erfolgt üblicherweise bei Temperaturen im Bereich von 0°C bis zum Siedepunkt der Reaktionsmischung, bevorzugt von 10°C bis 50°C, insbesondere bevorzugt von 15°C bis 35°C. In der Regel wird die Umsetzung in einem Lösungsmittel vorgenommen, bevorzugt in einem inerten organischen Lösungsmittel.

Geeignete inerte organische Lösungsmittel umfassen aliphatische Kohlenwasserstoffe wie Pentan, Hexan, Cyclohexan und Gemische von C₅-C₈-Alkanen, aromatische Kohlenwasserstoffe wie Toluol, o-, m- und p-Xylol, halogenierte Kohlenwasserstoffe wie Dichlormethan, Dichlorethan, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol, tert.-Butanol, Wasser sowie Dimethylsulfoxid, Dimethylformamid und Dimethylacetamid sowie Morpholin und N-Methylmorpholin. Es können auch Gemische der genannten Lösungsmittel verwendet werden.

In einer bevorzugten Ausgestaltungsform der Erfindung wird die Reaktion in einem Tetrahydrofuran- Wasser Gemisch durchgeführt, beispielsweise mit einem Mischungsverhältnis von 1 : 10 bis 10 : 1 (Volumenteile).

Als Basen kommen allgemein anorganische Verbindungen wie Alkalimetall- und Erdalkalimetallhydroxide wie Lithiumhydroxid, Natriumhydroxid, Kaliumhydroxid oder Calciumhydroxid, wässrige Lösung von Ammoniak, Alkalimetall- oder Erdalkalimetalloxide wie Lithiumoxid, Natriumoxid, Calciumoxid und Magnesiumoxid, Alkalimetall- und Erdalkalimetallhydride wie Lithiumhydrid, Natriumhydrid, Kaliumhydrid und Calciumhydrid, Alkalimetallamide wie Lithiumamid, beispielsweise Lithiumdiisopropylamid, Natriumamid und Kaliumamid, Alkalimetall- und Erdalkalimetallcarbonate wie Lithiumcarbonat, Kaliumcarbonat, Cäsiumcarbonat und Calciumcarbonat sowie Alkalimetallhydrogencarbonate wie Natriumhydrogencarboriat, metallorganische Verbindungen, insbesondere Alkalimetallalkyle wie Methyllithium, Butyllithium und Phenyllithium, Alkylmagnesiumhalogenide wie Methylmagnesiumchlorid sowie Alkalimetall- und Erdalkalimetallalkoholate wie Natriummethanolat, Natriumethanolat, Kaliumethanolat, Kalium- tert.-Butanolat, Kalium-tert.-Pentariolat und Dimethoxymagnesium, außerdem organische Basen, z.B. tertiäre Amine wie Trimethylamin, Triethylamin, Diisopropylethylamin 2-Hydroxypyridin und N-Methylpiperidin, Pyridin, substituierte Pyridine wie Collidin, Lutidin und 4-Dimethylaminopyridin sowie bicyclische Amine in Betracht. Selbstverständlich kann auch eine Mischung verschiedener Basen verwendet werden.

In einer Ausführungsform des erfindungsgemäßen Verfahrens wird die Reaktion von II in Gegenwart von Basen durchgeführt, bevorzugt in Gegenwart der Basen Kaliumtert.-Butanolat, 2-Hydroxypyridin oder einer wässrigen Lösung von Ammoniak oder einer Mischung dieser Basen. Bevorzugt wird nur eine dieser Basen verwendet. In einer besonders bevorzugten Ausführungsform wird die Reaktion in Gegenwart einer wässrigen Lösung von Ammoniak, die beispielsweise von 10 bis 50 w/v %ig sein kann, durchgeführt.

Die Umsetzung von II in Gegenwart einer Säure erfolgt üblicherweise bei Temperaturen im Bereich von 10°C bis zum Siedepunkt der Reaktionsmischung, bevorzugt von 50°C bis zum Siedepunkt, insbesondere bevorzugt beim Siedepunkt unter Rückfluss. In der Regel wird die Umsetzung in einem Lösungsmittel vorgenommen, bevorzugt in einem inerten organischen Lösungsmittel.

Als Lösungsmittel kommen prinzipiell diejenigen in Betracht, die auch für die basische Cyclisierung verwendet werden können, insbesondere Alkohole. In einer bevorzugten Ausführungsform wird die Reaktion in n-Butanol durchgeführt.

Als Säuren für die Cyclisierung von II kommen grundsätzlich sowohl Brönstedt- als auch Lewis-Säuren in Betracht. Insbesondere können anorganische Säuren, z.B. Halogenwasserstoffsäuren wie Fluorwasserstoffsäure, Salzsäure, Bromwasserstoffsäure, anorganische Oxosäuren wie Schwefelsäure und Perchlorsäure, weiterhin anorganische Lewis-Säuren wie Bortrifluorid, Aluminiumtrichlorid, Eisen-III-chlorid, Zinn-IV-chlorid, Titan-IV-chlorid und Zink-II-chlorid, sowie organische Säuren, beispielsweise Carbonsäuren und Hydroxycarbonsäuren wie Ameisensäure, Essigsäure, Propionsäure, Oxalsäure, Zitronensäure und Trifluoressigsäure, sowie organische Sulfonsäuren wie Toluolsulfonsäure, Benzolsulfonsäure, Camphersulfonsäure und dergleichen, Verwendung finden. Selbstverständlich kann auch eine Mischung verschiedener Säuren eingesetzt werden..

In einer Ausführungsform des erfindungsgemäßen Verfahrens wird die Reaktion in Gegenwart von organischen Säuren durchgeführt, beispielsweise in Gegenwart von Carbonsäuren wie Ameisensäure, Essigsäure oder Trifluoressigsäure oder einer Mischung dieser Säuren. Bevorzugt wird nur eine dieser Säuren verwendet. In einer bevorzugten Ausführungsform wird die Reaktion in Essigsäure durchgeführt.

Eine besonders bevorzugte Ausführungsform der sauren Cyclisierung wird in Gegenwart von n-Butanol, N-Methyl-Morpholin und Essigsäure unter Rückflussbedingungen durchgeführt.

In einer weiteren Ausführungsform der Erfindung wird die Umsetzung ausschließlich durch Erhitzen der Reaktionsmischung durchgeführt (thermische Cyclisierung). Die Umsetzung erfolgt hierbei üblicherweise bei Temperaturen im Bereich von 10°C bis zum Siedepunkt der Reaktionsmischung, bevorzugt von 50°C bis zum Siedepunkt der Reaktionsmischung, insbesondere bevorzugt beim Siedepunkt der Reaktionsmischung unter Rückfluss. In der Regel wird die Umsetzung in einem Lösungsmittel vorgenommen, bevorzugt in einem inerten organischen Lösungsmittel.

Als Lösungsmittel kommen prinzipiell diejenigen in Betracht, die bei der basischen Cycilsierung verwendet werden. Bevorzugt werden polar aprotische Lösungsmittel, z.B. Dimethylsulfoxid oder Dimethylformamid oder deren Mischungen. In einer bevorzugten Ausführungsform wird die Reaktion in Dimethylsulfoxid durchgeführt.

Die Reaktionsgemische erhalten nach einem der erfindungsgemäßen Verfahren A können z.B. in üblicher Weise aufgearbeitet werden. Das kann z.B. durch Mischen mit Wasser, Trennung der Phasen und gegebenenfalls chromatographische Reinigung der Rohprodukte erfolgen. Die Zwischen- und Endprodukte fallen z.T. in Form zäher Öle an, die in der Regel unter vermindertem Druck und bei mäßig erhöhter Temperatur von flüchtigen Anteilen befreit oder gereinigt werden können. Sofern die Zwischen- und Endprodukte als Feststoffe erhalten werden, kann die Reinigung auch durch Umkristallisieren oder Digerieren erfolgen.

### Verfahren B

Die Verbindungen der Formel I mit R¹ ≠ Wasserstoff können gemäß einem weiteren erfidnungsgemäßen Verfahren (Verfahren B) auch dadurch hergestellt werden, dass man eine Piperazinverbindung der Formel I, worin R¹ für Wasserstoff steht, mit einem Alkylierungsmittel oder Acylierungsmittel, das den von Wasserstoff verschiedenen Rest R¹ enthält, umsetzt. Derartige Umsetzungen können in Analogie zu literaturbekannten Verfahren erfolgen, beispielsweise nach den von I.O. Donkor et al., Bioorg. Med. Chem. Lett. 11 (19) (2001), 2647-2649, B.B. Snider et al., Tetrahedron 57 (16) (2001), 3301-3307, I. Yasuhiro et al., J. Am. Chem. Soc. 124(47) (2002), 14017-14019, oder M. Falomi et al., Europ. J. Org. Chem. (8) (2000), 1669-1675 beschriebenen Methoden.

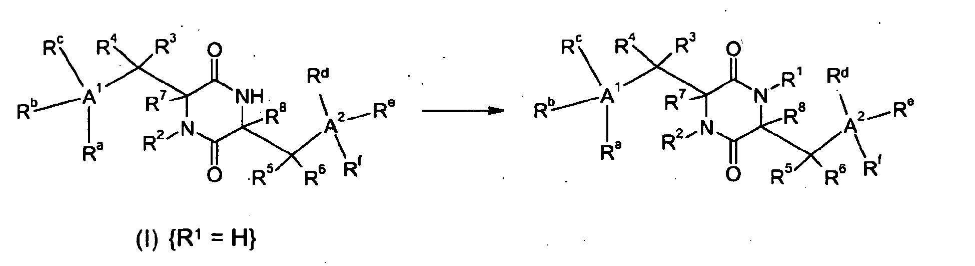

Gemäß Verfahren B wird eine Piperazinverbindung der Formel I mit R¹ = Wasserstoff mit einem geeigneten Alkylierungsmittel, im Folgenden Verbindung X¹-R¹, oder Acylierungsmittel, im Folgenden Verbindung X²-R¹, umgesetzt, wobei man eine Piperazinverbindung der Formel I mit R¹ * Wasserstoff erhält.

In den Alkylierungsmitteln X¹-R¹ kann X¹ Halogen oder O-SO₂-R^{m} mit R^{m} in der Bedeutung von C₁-C₄-Alkyl oder Aryl, welche gegebenenfalls durch Halogen, C₁-C₄-Alkyl oder Halo-C₁-C₄-alkyl substituiert sind, bedeuten. In Acylierungsmitteln X²-R¹ kann X² Halogen, insbesondere Cl bedeuten. Dabei ist R¹ * Wasserstoff und hat die oben angegebene Bedeutung und steht insbesondere für C₁-C₆-Alkyl, C₃-C₆-Cycloalkyl, C₃-C₆-Alkenyl, C₃-C₆-Cycloalkenyl, C₃-C₆-Alkinyl, C₃-C₆-Cycloalkinyl, Phenyl-(C₁-C₆)-Alkyl, Heterocyclyl, Heterocyclyl-(C₁-C₆)-Alkyl; Phenyl-[C₁-C₆-alkoxycarbonyl]-(C₁-C₆)-alkyl oder Phenylheterocydyl-(C₁-C₆)-alkyl; oder COR²¹ oder SO₂R²⁵, wobei die genannten aliphatischen, cyclischen oder aromatischen Teile von R¹ partiell oder vollständig halogeniert sein können und/oder eine bis drei der folgenden Gruppen tragen können: Cyano, Hydroxy, C₁-C₄-Alkyl, C₁-C₄-Halogenalkyl, C₃-C₆-Cycloalkyl, C₁-C₄-Alkoxy, C₁-C₄-Alkylthio, [Di-(C₁-C₄)-alkyl]-amino, C₁-C₄-Alkylcarbonyl, Hydroxycarbonyl, C₁-C₄-Alkoxycarbonyl, Aminocarbonyl, C₁-C₄-Alkylaminocarbonyl, [Di-(C₁-C₄)-alkyl]aminocarbonyl oder C₁-C₄-Alkylcarbonyloxy.

Die Umsetzung erfolgt üblicherweise bei Temperaturen im Bereich von -78°C bis zum Siedepunkt der Reaktionsmischung, bevorzugt von -50°C bis 65°C, insbesondere bevorzugt von -30°C bis 65°C. In der Regel wird die Umsetzung in einem Lösungsmittel vorgenommen, bevorzugt in einem inerten organischen Lösungsmittel.

Geeignete Lösungsmittel sind die unter Verfahren A zitierten Verbindungen, unter anderem Toluol, Dichlormethan, Tetrahydrofuran oder Dimethylformamid oder deren Mischungen.

In einer bevorzugten Ausführungsform der Erfindung wird die Reaktion in Tetrahydrofuran durchgeführt.

In einer bevorzugten Ausführungsform wird die Verbindung I mit R¹ = H mit dem Alkylierungs- bzw. Acylierungsmittel in Gegenwart einer Base durchgeführt. Geeignete Basen sind die unter Verfahren A zitierten Verbindungen. Die Basen werden im Allgemeinen äquimolar eingesetzt. Sie können auch im Überschuss oder selbst als Lösungsmittel verwendet werden. In einer bevorzugten Ausführungsform des erfindungsgemäßen Verfahrens wird die Base in äquimolarer Menge oder im Wesentlichen äquimolarer Menge zugesetzt. In einer weiteren bevorzugten Ausführungsform wird Natriumhydrid als Base verwendet.

Die Aufarbeitung erfolgt in der Regel in Analogie zu der unter Verfahren A beschriebenen Vorgehensweise.

### Verfahren C

In Analogie zu der unter Verfahren B geschilderten Weise können Verbindungen I, worin R² für Wasserstoff steht, mit Alkylierungsmitteln R²-X¹ oder Acyclierungsmitteln R²-X² umgesetzt werden, wobei man Verbindungen der Formel I mit R² = Wasserstoff erhält (Verfahren C). Die Reaktionsbedingungen des erfindungsgemäßen Verfahrens C entsprechen denen des Verfahrens B.

### Verfahren D

Die Verbindungen der Formel I können gemäß dem im folgenden Schema skizzierten Verfahren durch Umwandlung des Substituenten R^{a} hergestellt werden, beispielsweise in Analogie zu den von J. Tsuji, Top. Organomet. Chem. (14) (2005), 332 pp., oder J. Tsuji, Organic Synthesis with Palladium Compounds (1980), 207 pp. und Organikum, 21. Auflage, 2001, Wiley und dort zitierte Literatur, beschriebenen Methoden.

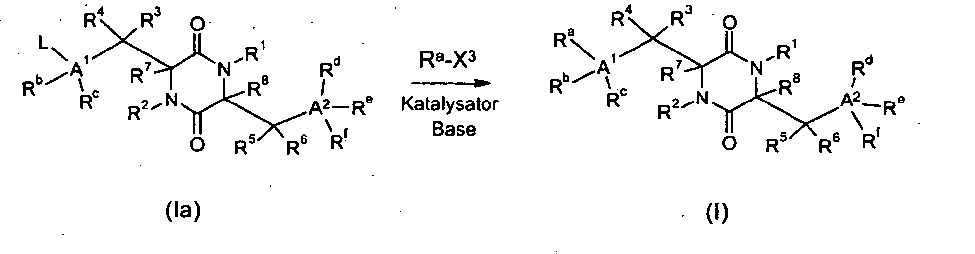

Hierzu wird eine Piperazinverbindung der Formel Ia, die anstelle des Substituenten R^{a} eine geeignete Abgangsgruppe L aufweist, durch Umsetzung mit einem Kupplungspartner, der eine Gruppe R^{a} enthält (Verbindung R^{a}-X³), in ein anderes Piperazinderivat der Formel I überführt.

Die Umsetzung erfolgt üblicherweise in Gegenwart eines Katalysators, bevorzugt in Gegenwart eines Übergangsmetallkatalysators. In der Regel findet die Reaktion in Gegenwart einer Base statt.

Diese Reaktionssequenz ist im Folgenden am Beispiel des Substituenten R^{a} dargestellt und kann selbstredend in analoger Weise für die Umwandlung der Substituenten R^{b} und R^{c} herangezogen werden.

Als Abgangsgruppe L kommen z.B. Halogen, insbesondere Chlor, Brom oder Iod, oder S(O)ₙR^{k}, mit n = 0, 1, 2 und R^{k} in der Bedeutung von C₁-C₆-Alkyl, Halo-(C₁-C₆)-alkyl oder gegebenenfalls halogeniertem oder mit C₁-C₄-Alkyl substituiertem Aryl in Betracht.

Als Kupplungspartner X³-R^{a} kommen insbesondere solche Verbindungen in Betracht, worin X³ im Falle von R^{a} in der Bedeutung von C₁-C₆-Alkyl, C₂-C₆-Alkenyl, Aryl oder Heteroaryl für eine der folgenden Gruppen steht:
- Zn-R^{l} mit R^{l} in der Bedeutung von Halogen, C₁-C₆-Alkyl, C₂-C₆-Alkenyl, Aryl oder Heteroaryl ;
- B(OR^{m})₂, mit R^{m} in der Bedeutung von H oder C₁-C₆-Alkyl, wobei zwei Alkylsubstituenten zusammen eine C₂-C₄-Alkylenkette bilden können; oder
- SnRⁿ₃, mit Rⁿ in der Bedeutung von C₁-C₆-Alkyl oder Aryl bedeutet.

Sofern R^{a} für C₂-C₆-Alkinyl steht, kann X³ auch Wasserstoff bedeuten.

Zur Herstellung der Verbindung I, worin Rₐ für CN steht, kann man die Verbindung Ia, worin L für Brom oder Iod steht, auch mit Kupfercyanid in Analogie zu bekannten Verfahren umsetzen (siehe beispielsweise Organikum, 21. Auflage, 2001, Wiley, S. 404 und dort zitierte Literatur).

Nach einer der bevorzugten Ausführungsformen sind dabei L oder R^{a} in den Verbindungen der Formel I in ortho-Position zur Verknüpfungsstelle von A¹ an ein C-Atom von A¹ gebunden.

Diese Umsetzung erfolgt üblicherweise bei Temperaturen im Bereich von -78°C bis zum Siedepunkt der Reaktionsmischung, bevorzugt von -30°C bis 65°C, insbesondere bevorzugt bei Temperaturen von 30°C bis 65°C. In der Regel wird die Umsetzung in einem inerten organischen Lösungsmittel in Gegenwart einer Base durchgeführt..

Geeignete Lösungsmittel sind die unter Verfahren A zitierten Verbindungen. In einer Ausführungsform des erfindungsgemäßen Verfahrens wird Tetrahydrofuran mit einer katalytischen Menge Wasser verwendet; in einer anderen Ausführungsform wird nur Tetrahydrofuran eingesetzt.

Geeignete Basen sind die unter Verfahren A zitierten Verbindungen.

Die Basen werden im Allgemeinen äquimolar eingesetzt. Sie können auch im Überschuß oder selbst als Lösungsmittel verwendet werden.

In einer bevorzugten Ausführungsform des erfindungsgemäßen Verfahrens wird die Base in äquimolarer Menge zugesetzt. In einer weiteren bevorzugten Ausführungsform werden Triethylamin oder Cäsiumcarbonat als Base verwendet, besonders bevorzugt Cäsiumcarbonat.

Als Katalysatoren für das erfindungsgemäße Verfahren sind prinzipiell Verbindungen der Übergangsmetalle Ni, Fe, Pd, oder Cu geeignet. Es ist möglich organische oder anorganische Verbindungen einzusetzen. Beispielhaft seien genannt: Pd(PPh₃)₂Cl₂, Pd(OAc)₂, PdCl₂, oder Na₂PdCl₄. Ph steht hierbei für Phenyl.

Die verschiedenen Katalysatoren können sowohl einzeln als auch als Gemische eingesetzt werden. In einer bevorzugten Ausführungsform der Erfindung wird Pd(PPh₃)₂Cl₂ verwendet.

Die Aufarbeitung kann analog der in Verfahren A beschriebenen Vorgehensweise erfolgen.

### Verfahren E

Piperazinverbindungen der Formel I, worin eine der Gruppen R^{a}, R^{b} oder R^{c} für COOH steht, können außerdem aus Piperazinverbindung der Formel I, worin R^{a}, R^{b} oder R^{c} für COOR^{z} mit R^{z} in der Bedeutung Alkyl, z.B. CH₃, steht, durch Verseifung der Estergruppe hergestellt werden. Die Verseifung gelingt z.B. durch Umsetzung mit (H₃C)₃SnOH, beispielsweise nach K. C. Nicolaou et al., Angew. Chem. Int. Ed. Engl. (44) (2005), 1378. Die so erhaltene Carbonsäure kann dann nach Standardmethoden der organische Synthese, gegebenenfalls nach Überführung in das Säurechlorid, durch Umsetzung mit einem Amin HNR^{u}R^{v} oder einem Alkohol HOR^{w} in den entsprechenden Ester oder das Amid überführt werden, Organikum, Autorenkollektiv, Leipzig 1993, 19. Auflage, S. 424-429. Diese Reaktionssequenz ist im Folgenden am Beispiel des Substituenten R^{a} dargestellt und kann selbstredend in analoger Weise für die Umwandlung der Substituenten R^{b} und R^{c} herangezogen werden.

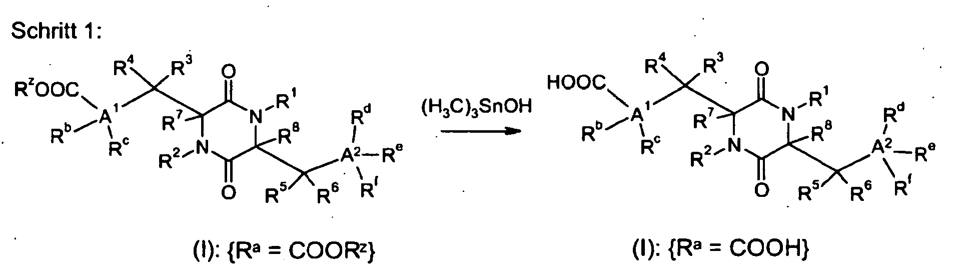

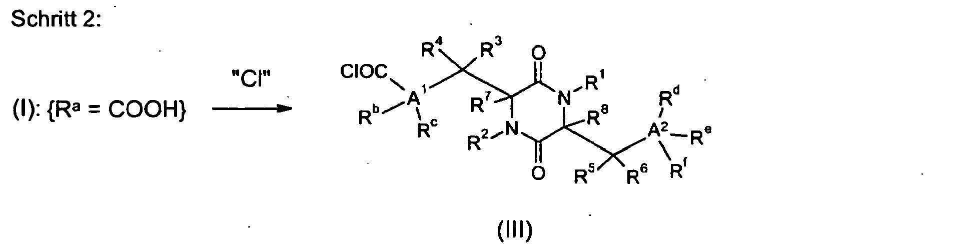

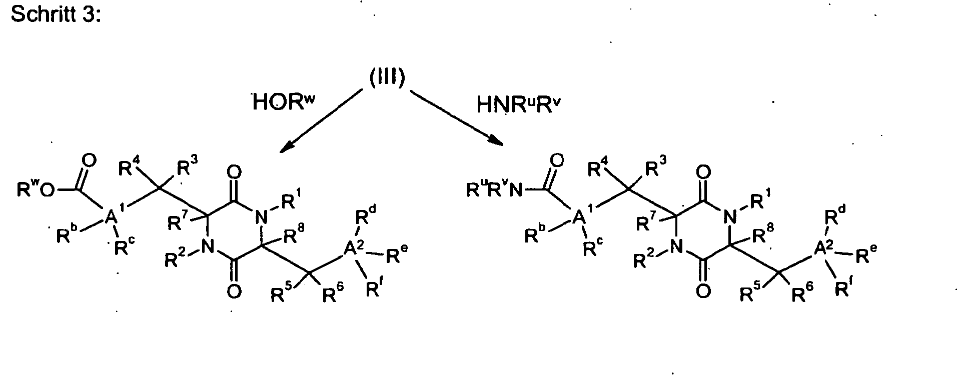

In diesem Schema haben die Variablen A¹, A², R¹-R⁸, R^{b}, R^{c}, R^{d}, R^{e} und R^{f} die zuvor genannten Bedeutungen. R^{u} und R^{v} stehen unabhängig voneinander für Wasserstoff, C₁-C₆-Alkyl, C₂-C₆-Alkenyl, C₂-C₆-Alkinyl, C₁-C₆-Alkoxy, C₁-C₆-Alkylsulfonyl, C₁-C₆-Alkylaminosulfonyl, [Di-(C₁-C₆)-alkylamino]sulfonyl oder ggf. subsituiertes Phenyl. R^{w} steht für C₁-C₆-Alkyl, C₃-C₆-Alkenyl oder C₃-C₆-Alkinyl.

In einem ersten Schritt wird die Estergruppe in der Piperazinverbindung I {R^{a} = COOR^{z}} verseift. Die Verseifung gelingt z.B. durch Umsetzung mit (H₃C)₃SnOH, wobei man die freie Säure von I erhält {R^{a} = COOH}. Die Reaktion zur freien Säure erfolgt üblicherweise mit einem Überschuss an (H₃C)₃SnOH. In der Regel wird die Reaktion in einem inerten organischen Lösungsmittel durchgeführt. Zu den geeigneten Lösungsmitteln zählt insbesondere Dichlorethan. Im Allgemeinen erfolgt die Umsetzung bei erhöhter Temperatur, beispielsweise bei ca. 80°C.

In einem zweiten Schritt wird die Säure I {R^{a} = COOH} in ihr Säurechlorid der Formel III überführt. Die Umsetzung zum Säurechlorid erfolgt üblicherweise bei Temperaturen von 10°C bis 50°C, bevorzugt bei Raumtemperatur, beispielsweise bei 25°C. In der Regel wird die Reaktion in einem inerten organischen Lösungsmittel durchgeführt. Zu den geeigneten Lösungsmitteln zählt insbesondere Dichlormethan. In einer bevorzugten Ausführungsfrom erfolgt die Umsetzung in Dichlormethan und katalytischen Mengen Dimethylformamid. Geeignet sind für die Chlorierung eine Vielzahl von Reagenzien, besipielsweise Oxalylchlorid oder Thionylchlorid. Bevorzugt werden im Wesentlichen äquimolare Mengen des Chlorierungsreagenzes, insbesondere Oxalylchlorid verwendet.

Die Umsetzung mit einem Amin NHR^{u}R^{v} in der Folgereaktion erfolgt üblicherweise durch Zugabe eines Überschusses des jeweiligen Amins. Die Reaktion kann in einem Temperaturbereich von 0°C bis 40°C, bevorzugt bei Raumtemperatur, beispielsweise bei 25°C ausgeführt werden.

Die Umsetzung mit einem Alkohol HOR^{w} in der Folgereaktion erfolgt üblicherweise durch Zugabe eines Überschusses sowohl des jeweiligen Alkohols als auch von Triethylamin.

Die Reaktion kann in einem Temperaturbereich von 0°C bis 40°C, bevorzugt bei Raumtemperatur, beispielsweise bei 25°C ausgeführt werden.

Die Aufarbeitung kann in Analogie zu der bei Verfahren A beschriebenen Vorgehensweise erfolgen.

### Verfahren F

Die Verbindungen der Formel I können gemäß der im Folgenden gezeigten Synthese durch Kupplung von Piperazinverbindungen der allgemeinen Formel IV mit Verbindung gen V hergestellt werden. Die Kupplung von IV mit V gelingt in Analogie zu literaturbekannten Verfahren, beispielsweise nach G. Porzi et al., Tetrahedron Asymmetry 9 (19), (1998), 3411-3420, oder C. I. Harding et al., Tetrahedron 60 (35), (2004), 7679-7692, oder C. J. Chang et al., J. Chem. Soc. Perk. T. 1 (24), (1994), 3587-3593.

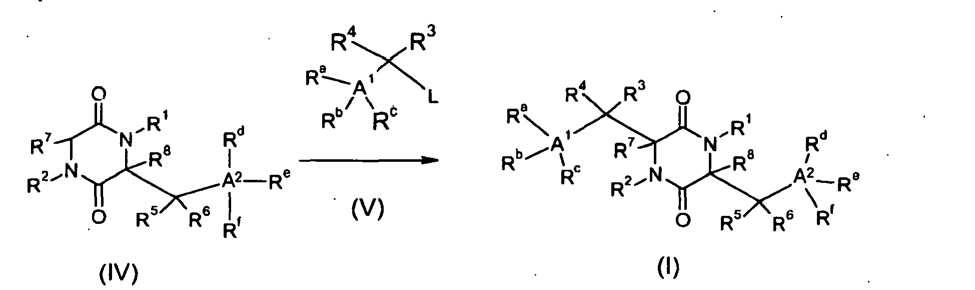

In dem Schema haben A¹, A², R¹ - R⁸, R^{a}, R^{b}, R^{c} R^{d}, R^{e} und R^{f} die zuvor angebenen Bedeutungen. L steht für eine geeignete Abgangsgruppe, wie Halogen oder OSO₂R^{m}, mit R^{m} in der Bedeutung, von C₁-C₄-Alkyl, Aryl, oder ein- bis dreifach durch C₁-C₄-Alkyl substituiertes Aryl.

In der Regel erfolgt die Reaktion bei Temperaturen im Bereich von -78°C bis zum Siedepunkt der Reaktionsmischung, bevorzugt im Bereich von -78°C bis 40°C, insbesondere bevorzugt im Bereich von -78°C bis 30°C.

In der Regel wird die Reaktion in einem inerten organischen Lösungsmittel in Gegenwart einer Base durchgeführt. Geeignete Lösungsmittel sind die unter Verfahren A zitierten Verbindungen. In einer bevorzugen Ausführungsform des erfindungsgemäßen Verfahrens wird Tetrahydrofuran eingesetzt.

Geeignete Basen sind die unter Verfahren A zitierten Verbindungen. In einer weiteren, bevorzugten Ausführungsform wird Lithiumdiisopropylamid, besonders bevorzugt in im Wesentlichen äquimolarer Menge, insbesondere äquimolar als Base verwendet.

Verbindungen der Formel V sind zum Teil kommerziell erhältlich oder durch literaturbeschriebene Transformationen der entsprechenden kommerziell erhältlichen Vorprodukte herstellbar.

Die Aufarbeitung kann in Analogie zu der bei Verfahren A beschriebenen Vorgehensweise erfolgen.

Die für die Herstellung der Verbindungen der Formel I benötigen Vorstufen und Zwischenprodukte sind teilweise kommerziell erhältlich, aus der Literatur bekannt oder können nach literaturbekannten Verfahren hergestellt werden.

Die Dipeptid-Verbindungen der Formel II können beispielsweise aus N-geschützten Dipeptiden der allgemeinen Formel VI in Analogie zu literaturbekannten Verfahren hergestellt werden, beispielsweise nach Glenn L. Stahl et al., J. Org. Chem. 43(11), (1978), 2285-6 oder A. K. Ghosh et al., Org. Lett. 3(4), (2001), 635-638.

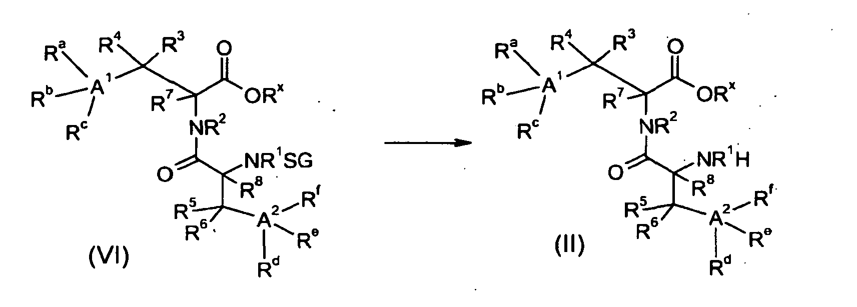

In den Formeln II und VI haben die Variablen A¹, A², R¹ - R⁸, R^{a}, R^{b}, R^{c}, R^{d}, R^{e} und R^{f} die für Formel I angegebene Bedeutung, SG bedeutet eine Stickstoff Schutzgruppe wie Boc (= tert-Butoxycarbonyl) und OR^{x} steht für eine über ein Sauerstoffatom gebundene Abgangsgruppe. Selbstverständlich gelten die bevorzugten Bedeutungen für die Verbindungen der Formel I sinngemäß jeweils für die Verbindungen der Formel II oder IV. Bezüglich der Abgangsgruppe OR^{x} gilt das zuvor für die Dipeptide der Formel II Gesagte.

So kann beispielsweise ein Dipeptid der Formel VI, worin SG für Boc steht und OR^{x} eine geeignete Abgangsgruppe, bei der R^{x} z.B. C₁-C₆-Alkyl, insbesondere Methyl, Ethyl oder Benzyl ist, in Gegenwart einer Säure zu einer Verbindung der Formel II umgesetzt werden.

Die Umsetzung erfolgt üblicherweise bei Temperaturen im Bereich von -30°C und dem Siedepunkt der Reaktionsmischung, bevorzugt von 0°C bis 50°C, insbesondere bevorzugt von 20°C bis 35°C.

Die Reaktion kann in einem Lösungsmittel stattfinden, inbesondere in einem inerten organischen Lösungsmittel. Als Lösungsmittel kommen prinzipiell die bei der basischen Cyclisierung zitierten Verbindungen in Betracht, insbesondere Tetrahydrofuran oder Dichlormethan oder deren Mischungen. In einer bevorzugten Ausführungsform wird die Reaktion in Dichlormethan durchgeführt.

Als Säuren finden die bei Verfahren A zitierten Säuren Verwendung.

In einer Ausführungsform des erfindungsgemäßen Verfahrens wird die Reaktion in Gegenwart von organischen Säuren durchgeführt, beispielsweise in Gegenwart starker organischer Säuren, wie Ameisensäure, Essigsäure oder Trifluoressigsäure oder deren Mischungen. In einer bevorzugten Ausführungsform wird die Reaktion in Gegenwart von Trifluoressigsäure durchgeführt.

Die Aufarbeitung kann in Analogie zu der bei Verfahren A beschriebenen Vorgehensweise erfolgen.

Die geschützten Dipeptide der Formel VI können in Analogie zu literaturbekannten Verfahren hergestellt werden, beispielsweise nach Wilford L. Mendelson et al., Int. J.Peptide & Protein Research 35(3), (1990), 249-257. Ein typischer Zugang ist die Amidierung einer Boc-geschützen Aminosäure VIII mit einem Aminosäureester der Formel VII wie in dem folgenden Schema dargestellt:

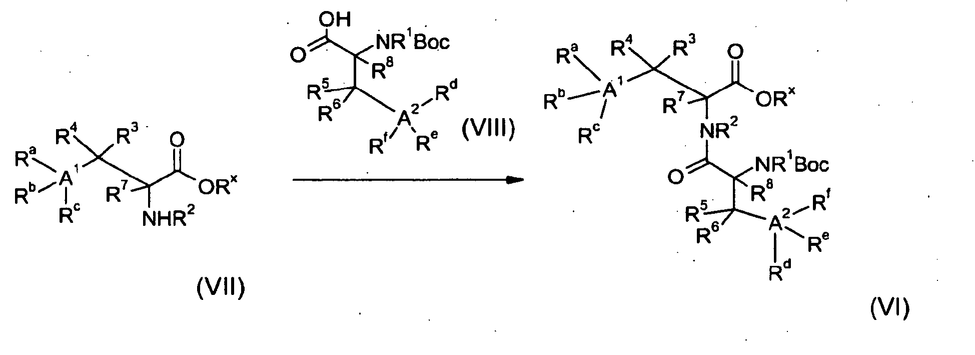

In diesem Schema haben die Variablen die zuvor genannten Bedeutungen. Anstelle von Boc können auch andere Amino-Schutzgruppen eingesetzt werden.

In der Regel erfolgt die Umsetzung von VII mit VIII bei Temperaturen in einem Bereich von -30°C bis zum Siedepunkt der Reaktionsmischung, bevorzugt von 0°C bis 50°C, insbesondere bevorzugt von 20°C bis 35°C. Die Reaktion kann in einem Lösungsmittel, bevorzugt in einem inerten organischen Lösungsmittel ausgeführt werden. Geeignet sind die bei Verfahren A im Zusammenhang mit der basischen Cyclisierung genannten Lösungsmittel.

Im Allgemeinen erfordert die Umsetzung die Anwesenheit eines Aktivierungsreagenzes. Geeignete Aktivierungsreagenzien sind Kondensationsmittel wie z.B. polystyrol- oder nicht polystyrolgebundenes Dicyclohexylcarbodiimid (DCC), Diisopropylcarbodiimid, 1-Ethyl-3-(dimethylaminopropyl)carbodiimid (EDAC), Carbonyldiimidazol, Chlorkohlensäureester wie Methylchloroformiat, Ethylchloroformiat, Isoropylchloroformiat, Isobutylchloroformiat, sec-Butylchloroformiat oder Allylchloroformiat, Pivaloylchlorid, Polyphosphorsäure, Propanphosphonsäureanhydrid, Bis(2-oxo-3-oxazolidinyl)-phosphorylchlorid (BOPCI) oder Sulfonylchloride wie Methansulfonylchlorid, Toluolsulfonylchlorid oder Benzolsulfonylchlorid. Nach einer Ausführungsform werden als Aktivierungsreagenz EDAC oder DCC Bevorzugt.

Vorzugsweise erfolgt die Umsetzung von VII mit VIII in Gegenwart einer Base.Geeignete Basen sind die unter Verfahren A zitierten Verbindungen. In einer Ausführungsform werden als Base Triethylamin oder N-Ethyldiisopropylamin oder deren Mischungen, besonders bevorzugt N-Ethyldiisopropylamin verwendet.

Die Aufarbeitung kann in Analogie zu der bei Verfahren A beschriebenen Vorgehensweise erfolgen.

Die Verbindungen der Formel VII können ihrerseits durch Entschützen entsprechender geschützten Aminosäureverbindungen IX in Analogie zu literaturbekannten Verfahren hergestellt werden, beispielsweise nach Glenn L. Stahl et al., J. Org. Chem. 43(11), (1978), 2285-6. oder A. K. Ghosh et al., Org. Lett. 3(4), (2001), 635-638. Die Herstellung von VII aus einer Boc-geschützten Aminosäureverbindung IX ist im folgenden Schema dargestellt. Anstelle der Boc-Gruppe können auch andere Amino-Schutzgruppen eingesetzt werden.

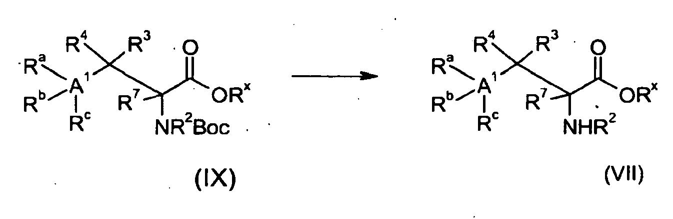

Die Umsetzung einer Verbindung der Formel IX zur Verbindung VII erfolgt typischerweise in Gegenwart einer Säure bei Temperaturen in einem Berich von -30°C bis zum Siedepunkt der Reaktionsmischung, bevorzugt von 0°C bis 50°C, insbesondere bevorzugt von 20°C bis 35°C. Die Reaktion kann in einem Lösungsmittel durchgeführt werden, bevorzugt in einem inerten organischen Lösungsmittel.

Als Lösungsmittel kommen prinzipiell die unter der basischen Cyclisierung zitierten Verbindungen in Betracht, insbesondere Tetrahydrofuran oder Dichlormethan oder deren Mischungen. In einer bevorzugten Ausführungsform wird die Reaktion in Dichlormethan durchgeführt.

Als Säuren und saure Katalysatoren finden die bei Verfahren A zitierten Stoffe Verwendung.

In einer Ausführungsform des erfindungsgemäßen Verfahrens wird die Reaktion in Gegenwart von organischen Säuren durchgeführt, beispielsweise in Gegenwart von starken organischen Säuren, wie Ameisensäure, Essigsäure oder Trifluoressigsäure oder deren Mischungen. In einer bevorzugten Ausführungsform wird die Reaktion in Gegenwart von Trifluoressigsäure durchgeführt.

Die Aufarbeitung kann in Analogie zu der bei Verfahren A beschriebenen Vorgehensweise erfolgen.

Die Verbindungen der Formel IX können entsprechend der im folgenden Schema dargestellten Umsetzung hergestellt werden. Die Umsetzung von Verbindung V mit der geschützten Aminosäureverbindung X kann in Analogie zu literaturbekannten Verfahren erfolgen, beispielsweise nach I. Ojima et al., J. Am. Chem. Soc., 109(21), (1987), 6537-6538 oder J. M. McIntosh et al., Tetrahedron 48(30), (1992), 6219-6224.

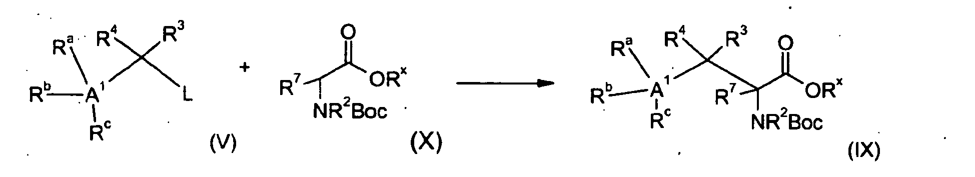

In diesem Schema haben die Variablen die zuvor genannten Bedeutungen. L steht für eine Abgangsgruppe, z.B eine der bei Verfahren F genannten Abgangsgruppen. Anstelle von Boc können auch andere Amino-Schutzgruppen eingesetzt werden.

Die Umsetzung von V mit X von erfolgt in der Regel in Gegenwart von Base. Geeignete Basen sind die unter Verfahren A zitierten Verbindungen. In einer weiteren bevorzugten Ausführungsform wird Lithiumdiisopropylamid, besonders bevorzugt in im Wesentlichen äquimolarer Menge, insbesondere äquimolar als Base verwendet.

Üblicherweise wird die Reaktion bei Temperaturen im Bereich von - 78°C bis zum Siedepunkt der Reaktionsmischung, bevorzugt von - 78°C bis zum Siedepunkt, insbesondere bevorzugt von - 78°C bis 30°C durchgeführt.

Die Reaktion kann in einem Lösungsmittel durchgeführt werden, bevorzugt in einem inerten organischen Lösungsmittel. Als Lösungsmittel kommen prinzipiell die unter der basischen Cyclisierung genannten Lösungsmittel in Betracht, insbesondere Dichlormethan oder Tetrahydrofuran oder deren Mischungen. In einer bevorzugten Ausführungsform wird die Reaktion in Tetrahydrofuran durchgeführt.

Die Aufarbeitung kann in Analogie zu der bei Verfahren A beschriebenen Vorgehensweise erfolgen.

Verbindungen der Formel V sind zum Teil kommerziell erhältlich oder durch literaturbeschriebene Transformationen der entsprechenden kommerziell erhältlichen Vorprodukte herstellbar.

Aminosäurederivate der Formel VIII, X oder das unten beschriebene Derivat XV sind ebenfalls zum Teil kommerziell erhältlich oder durch literaturbeschriebene Transformationen der entsprechenden kommerziell erhältlichen Vorprodukten herstellbar.

Die Verbindungen der Formel IV mit R¹ * Wasserstoff können dadurch hergestellt werden, dass man eine Piperazinverbindung der Formel IV, worin R¹ für Wasserstoff steht, mit einem Alkylierungsmittel oder Acylierungsmittel, das den von Wasserstoff verschiedenen Rest R¹ enthält, umsetzt. In analoger Weise können Verbindungen IV mit R² ≠. Wasserstoff dadurch hergestellt werden, dass man eine Piperazinverbindung der Formel IV, worin R² für Wasserstoff steht, mit einem Alkylierungsmittel oder Acylierungsmittel, das den von Wasserstoff verschiedenen Rest R² enthält, umsetzt. Derartige Umsetzungen können in Analogie zu literaturbekannten Verfahren erfolgen, beispielsweise nach den von I.O. Donkor et al., Bioorg. Med. Chem. Lett. 11 (19) (2001), 2647-2649, B.B. Snider, et al., Tetrahedron 57 (16) (2001), 3301-3307, I. Yasuhiro et al., J. Am. Chem. Soc. 124(47) (2002), 14017-14019, oder M. Falorni et al., Europ. J. Org. Chem. (8) (2000), 1669-1675 beschriebenen Methoden.

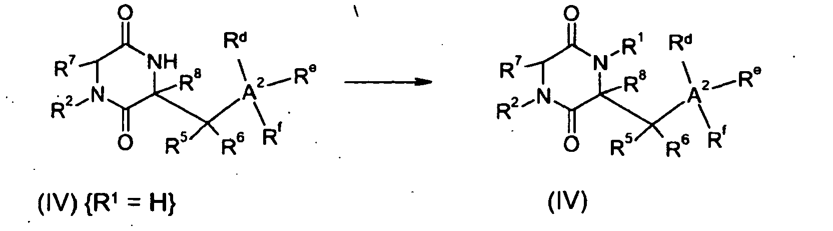

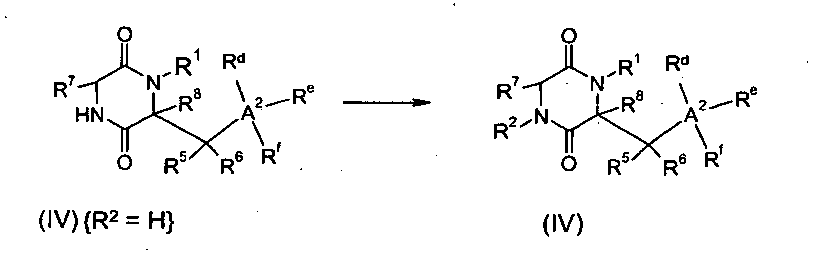

Bezüglich der Alkylierungsmittel bzw. Acyclierungsmittel gilt das bei den Verfahren B und C Gesagte in gleicher Weise. Bezüglich der Reaktionsbedingungen dieser Umsetzungen gilt ebenfalls das zuvor bei den Verfahren B und C Gesagte.
Die Verbindungen der Formel IV können auch durch intramolekulare Cyclisierung von Verbindungen der allgemeinen Formel XIII in Analogie zu weiteren literaturbekannten Verfahren hergestellt werden, beispielsweise nach T. Kawasaki et al., Org. Lett. 2(19) (2000), 3027-3029, 3027-3029, Igor L. Rodionov et al., Tetrahedron 58(42) (2002), 8515-8523 oder A. L. Johnson et al., Tetrahedron 60 (2004), 961-965.

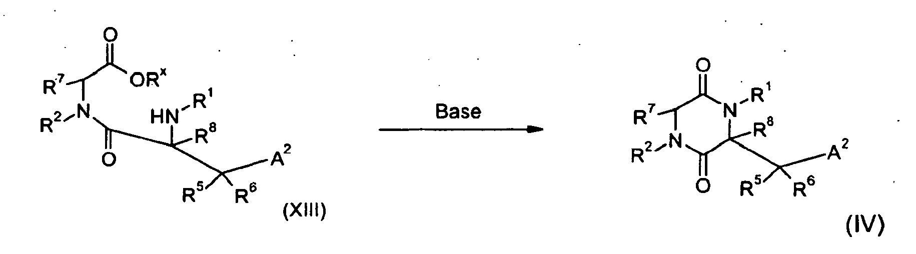

Dabei ist OR^{x} eine geeignete Abgangsgruppe, R^{x} ist hierbei z.B. C₁-C₆-Alkyl, insbesondere Methyl, Ethyl oder Benzyl. 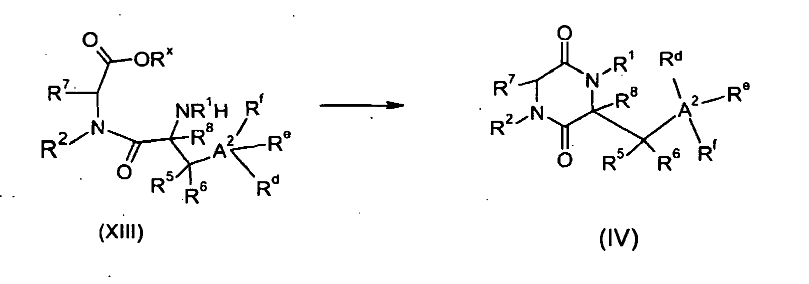 In Formel XIII haben die Variablen R^{x}, A², R¹, R², R⁵, R⁶, R⁷, R⁸, R^{d}, R^{e} und R^{f} die für Formel II angegebene Bedeutung. Die Gruppe OR^{x} steht für eine geeignete, über Sauerstoff gebundene Abgangsgruppe. R^{x} ist hierbei z.B. C₁-C₆-Alkyl, insbesondere Methyl, Ethyl oder Phenyl-C₁-C₆-alkyl, z.B. Benzyl.

Die Cyclisierung der Verbindungen der Formel XIII kann in Gegenwart einer Base erfolgen. Die Reaktion erfolgt dann in der Regel bei Temperaturen im Bereich von 0°C bis zum Siedepunkt der Reaktionsmischung, bevorzugt von 10°C bis 50°C, insbesondere bevorzugt von 15°C bis 35°C. Die Umsetzung kann in einem Lösungsmittel durchgeführt werden, bevorzugt in einem inerten organischen Lösungsmittel.

Als Lösungsmittel kommen prinzipiell die unter der themischen Cyclisierung zitierten Verbindungen in Betracht, insbesondere ein Tetrahydrofuran- Wasser Gemisch mit einem Mischungsverhältnis von 1 : 10 bis 10 : 1.

Geeignete Basen sind die bei der basischen Cyclisierung gemäß Verfahren A genannten Basen, insbesondere Kalium- tert.-Butanolat, 2-Hydroxypyridin oder eine wässrige Lösung von Ammoniak oder einer Mischung dieser Basen. Bevorzugt wird nur eine dieser Basen verwendet. In einer besonders bevorzugten Ausführungsform wird die Reaktion in Gegenwart einer wässrigen Lösung von Ammoniak, die beispielsweise von 10 bis 50 w/v %ig sein kann, durchgeführt.

Die Verbindungen der Formel XIII können ihrerseits nach der im folgenden Schema dargestellten Synthese in Analogie zu literaturbekannten Verfahren hergestellt werden, beispielsweise nach Wilford L. Mendelson et al., Int. J.Peptide & Protein Research 35(3), (1990), 249-257, Glenn L. Stahl et al., J. Org. Chem. 43(11), (1978), 2285-2286 oder A. K. Ghosh et al., Org. Lett. 3(4), (2001), 635-638.

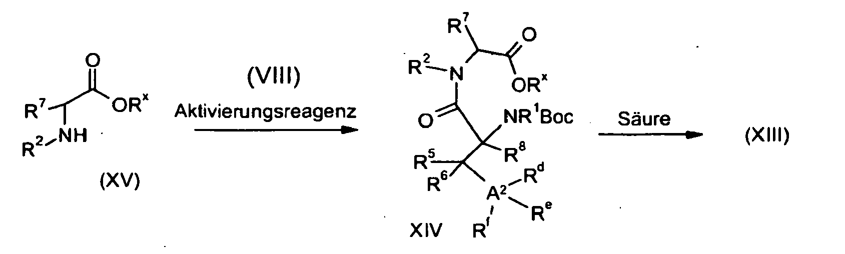

In dem Schema haben die Variablen R^{x}, A², R¹, R², R⁵, R⁶, R⁷, R⁸, R^{d}, R^{e} und R^{f} die für Formel II bwz. XIII angegebenen Bedeutungen. Die Synthese umfasst in einem ersten Schritt die Kupplung von Aminosäureverbindungen XV mit Boc-geschützten Aminosäuren VIII in Gegenwart eines Aktivierungsreagenz.

Die Umsetzung einer Verbindung der Formel XV mit einer Verbindung der Formel VIII erfolg üblicherweise bei Temperaturen im Bereich von -30°C bis zum Siedepunkt der Reaktionsmischung, bevorzugt von 0°C bis 50°C, insbesondere bevorzugt von 20°C bis 35°C. Die Reaktion kann in einem Lösungsmittel durchgeführt werden, bevorzugt in einem inerten organischen Lösungsmittel. Wegen weiterer Details wird diesbezüglich auf die Herstellung von Verbindung VI durch Amidierung der Aminosäureverbindung VIII mit der Verbindung VII verwiesen.

Im Allgemeinen erfordert die Umsetzung die Anwesenheit eines Aktivierungsreagenzes. Geeignete Aktivierungsreagenzien sind Kondensationsmittel wie z.B. polystyrol- oder nicht polystyrolgebundenes Dicyclohexylcarbodiimid (DCC), Diisopropytcarbodiimid, 1-Ethyl-3-(dimethylaminopropyl)carbodiimid (EDAC), Carbonyldiimidazol, Chlorkohlensäureester wie Methylchloroformiat, Ethylchloroformiat, Isoropylchloroformiat, Isobutylchloroformiat, sec-Butylchloroformiat oder Allylchloroformiat, Pivaloylchlorid, Polyphosphorsäure, Propanphosphonsäureanhydrid, Bis(2-oxo-3-oxazolidinyl)-phosphorylchlorid (BOPCI) oder Sulfonylchloride wie Methansulfonylchlorid, Toluolsulfonylchlorid oder Benzolsulfonylchlorid. Nach einer Ausführungsform werden als Aktivierungsreagenz EDAC oder DCC bevorzugt.

Vorzugsweise erfolgt die Umsetzung von XV mit VIII in Gegenwart einer Base.

Geeignete Basen sind die unter Verfahren A zitierten Verbindungen. In einer Ausführungsform werden als Base Triethylamin oder N-Ethyldlisopropylamin oder deren Mischungen, besonders bevorzugt N-Ethyldiisopropylamin verwendet.

Die Aufarbeitung kann in Analogie zu der bei Verfahren A beschriebenen Vorgehensweise erfolgen.

Das Entschützen der Verbindung XIV zur Verbindung XIII erfolgt typischerweise durch Behandlung mit einer Säure. Die Umsetzung erfolgt üblicherweise bei Temperaturen im Bereich von -30°C bis zum Siedepunkt der Reaktionsmischung, bevorzugt von 0°C bis 50°C, insbesondere bevorzugt von 20°C bis 35°C. Die Reaktion kann in einem Lösungsmittel, bevorzugt in einem inerten organischen Lösungsmittel ausgefürt werden.

Als Lösungsmittel kommen prinzipiell die unter Verfahren A im Zusammenhang mit der basischen Cyclisierung genannten Lösungsmittel in Betracht, insbesondere Tetrahydrofuran oder Dichlormethan oder deren Mischungen. In einer bevorzugten Ausführungsform wird die Reaktion in Dichlormethan durchgeführt.

Als Säure finden die bei Verfahren A genannten Säuren Verwendung. Wegen weiterer Details wird auch auf die Entschützung von VI zur Verbindung II verwiesen. Die dort genannten Reaktionsbedingungen eignen sich auch zum Entschützen von Verbindung XIV. In einer Ausführungsform des erfindungsgemäßen Verfahrens wird die Reaktion in Gegenwart von organischen Säuren, insbesondere starker organischer Säuren durchgeführt, beispielsweise in Gegenwart von Ameisensäure, Essigsäure oder Trifluoressigsäure oder deren Mischungen. In einer bevorzugten Ausführungsform wird die Reaktion in Gegenwart von Trifluoressigsäure durchgeführt.

Die Aufarbeitung kann in Analogie zu der bei Verfahren A beschriebenen Vorgehensweise erfolgen.

### Herstellungsbeispiele:

### Beispiel 1

### 3-Benzyl-6-(2-ethinyl-benzyl)-1,4-dimethyl-piperazin-2,5-dion

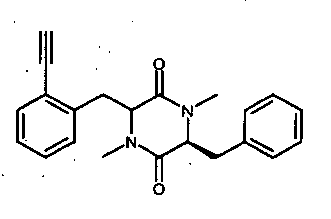

### 1.1) 2-(tert-Butoxycarbonyl-methyl-amino)-3-(2-iodphenyl)-propionsäureethylester

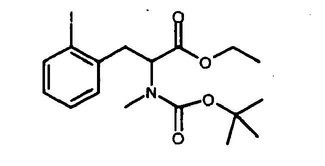

Zu (tert-Butoxycarbonyl-methyl-amino)-essigsäureethylester (7.4 g, 34 mmol) in Tetrahydrofuran (abs., 50 mL) wurde bei - 78 °C langsam Lithiumdiisopropylamid-Lösung (2 M in THF/n-Heptan, 17 mL, 34 mmol) zugetropft. Es wurde für 2 h bei dieser Temperatur nachgerührt. Anschließend wurde 1-Brommethyl-2-iod-benzol (10.0 g, 46 mmol) langsam zugetropft und für 1 h bei -78 °C nachgerührt. Die Reaktionslösung wurde langsam (12 h) auf Raumtemperatur erwärmt und anschließend am Rotationsverdampfer eingeengt. Der Rückstand wurde in Essigsäureethylester aufgenommen, gewaschen, getrocknet und eingeengt. Der so erhaltene Rückstand wurde anschließend säulenchromatographisch (SiO₂, Hexan/ Essigsäureethylester) gereinigt. Es wurden so 6.4 g (43 %) der Zielverbindung erhalten.
M+Na (m/z): 456.

### 1.2) 3-(2-Iodphenyl)-2-methylamino-propionsäureethylester

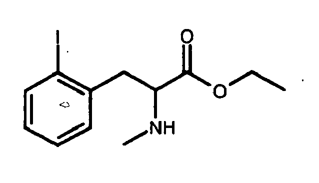

Zu 2-(tert-Butoxycarbonyl-methyl-amino)-3-(2-iodo-phenyl)-propionsäureethylester (6.4 g, 15 mmol) in CH₂Cl₂ (35 mL) wurde Trifluoressigsäure (25 mL) gegeben. Es wurde für 2 h bei Raumtemperatur nachgerührt. Die Reaktionslösung wurde am Rotationsverdampfer eingeengt, der Rückstand in Essigsäureethylester aufgenommen, gewaschen (NaHCO₃ sat.), getrocknet und eingeengt. Der so erhaltene Rückstand (3.9 g, 79 %) wurde als Rohprodukt weiter umgesetzt.
M+1 (m/z): 334.

### 1.3) 2-{[2-(tert-Butoxycarbonyl-methyl-amino)-3-phenyl-propionyl]-methyl-amino}3-(2-iodphenyl)-propionsäureethylester

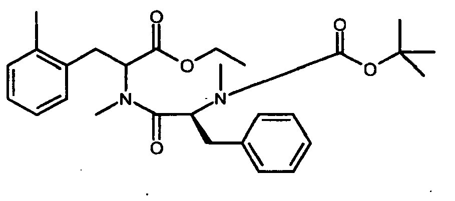

3-(2-Iodo-phenyl)-2-methylamino-propionsäureethylester (3.94 g, 12 mmol), 2-(tert-Butoxycarbonyl-methyl-amino)-3-phenyl-propionsäure (3.7 g, 13 mmol), N-Ethyldiisopropyl-amin (7.8 g, 61 mmol) und EDAC (4.6 g, 24 mmol) wurden in THF (abs., 50 mL) für 16 h gerührt. Die Reaktionslösung wurde am Rotationsverdampfer eingeengt. Der Rückstand wurde in Essigsäureethylester aufgenommen, gewaschen, getrocknet und eingeengt. Nach säulenchromatographischer Reinigung (SiO₂, Hexan/Essigsäureethylester) wurden 4.0 g (56 %) der Zielverbindung erhalten.
M+Na (m/z): 617.

### 1.4) 3-(2-Iodphenyl)-2-[methyl-(2-methylamino-3-phenyl-propionyl)-amino]-propionsäureethylester

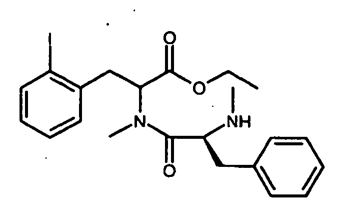

Zu 2-{[2-(tert-Butoxycarbonyl-methyl-amino)-3-phenyl-propionyl]-methyl-amino}3-(2-iodo-phenyl)-propionsäureethylester (2.5 g, 4.2 mmol) in CH₂Cl₂ (10 mL) wurde Trifluoressigsäure (10 mL) gegeben. Es wurde für 2 h bei Raumtemperatur nachgerührt und anschließend am Rotationsverdampfer eingeengt. Der Rückstand wurde säulenchromatographisch gereinigt (SiO₂, Hexan/ Essigsäureethylester).
M+1 (m/z): 495.

### 1.5) 3-Benzyl-6-(2-iodbenzyl)-1,4-dimethyl-piperazin-2,5-dion

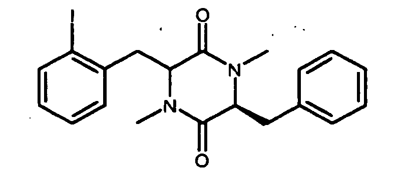

3-(2-Iodphenyl)-2-[methyl-(2-methylamino-3-phenyl-propionyl)-amino]-propionsäureethylester (2.1 g, 4.2 mmol) wurde in THF (30 mL) aufgenommen und mit NH₄OH (25 w/v % in H₂O, 20 mL) versetzt. Es wurde für 12 h bei Raumtemperatur nachgerührt und anschließend am Rotationsverdampfer eingeengt. Der so erhaltene Rückstand wurde säulenchromatographisch (SiO₂, Hexan/Essigsäureethylester) gereinigt. Es wurden so 0.4 g (21 %) eines unpolaren Isomers und 0.6 g (31 %) eines polaren Isomers erhalten.
M+1 (m/z): 449.

### 1.6) 3-Benzyl-1,4-dimethyl-6-(2-trimethylsilanylethinyl-benzyl)-piperazin-2,5-dion

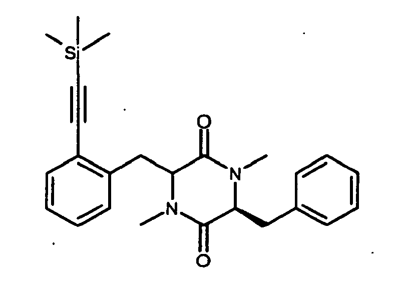

3-Benzyl-6-(2-iodbenzyl)-1,4-dimethyl-piperazine-2,5-dion (polares Isomer aus 1.5, 100 mg, 0.22 mmol) wurde zusammen mit Diisopropylamid (0.5 mL), Trimethylsilylacetylen (40 mg, 0.4 mmol), Pd(PPh₃)₄ (60 mg, 0.05 mmol) und Cul (10 mg, 0.05 mmol) in DMF (abs., 5 mL) unter Argon für 12 h bei 80 °C gerührt. Nach Abkühlen und Zugabe von H₂O wurde mit Methyl-tert.-Butylether extrahiert, die organischen Phasen getrocknet und eingeengt. Nach säulenchromatographischer Reinigung wurden 21 mg (23 %) der Zielverbindung als farbloses Öl erhalten.
M+1 (m/z): 419.

### 1.7) 3-Benzyl-6-(2-ethinyl-benzyl)-1,4-dimethyl-piperazin-2,5-dion

Zu 3-Benzyl-1,4-dimethyl-6-(2-trimethylsilanylethinyl-benzyl)-piperazin-2,5-dion (140 mg, 0.22 mmol) in THF (abs., 5 mL) wurde bei 0 °C Tetrabutylammoniumfluorid (1 mM in THF, 0.5 mL, 0.5 mmol) zugetropft. Es wurde für 1 h bei dieser Temperatur nachgerührt. Nach Zugabe von NH₄Cl-Lösung (gesättigt, aq.) wurde mit Essigsäureethylester extrahiert, die organischen Phasen getrocknet und eingeengt. Nach säulenchromatographischer Reinigung wurden 50 mg (65 %) der Zielverbindung als gelbes Öl erhalten.
M+1 (m/z): 346.
¹H-NMR (CDCl₃): δ = 2.41 (d, 2H), 2.72 (s, 3H), 2.87 (s, 3H), 3.00 (dm, 1H), 3.12 (dm, 1H), 3.31 (s, 1H), 4.16 (m, 2H), 6.94 (dm, 1H), 7.14-7.31 (brm, 5H), 7.35 (m, 2H), 7.45 (m, 1H).

In analoger Weise wurden weitere Verbindungen der Formel I hergestellt, wobei die Strukturelemente der folgenden Tabelle II als Legende für die nachfolgenden Tabellen A.1 bis A.4 stehen.

**Tabelle II**

| | | | | |
|---|---|---|---|---|
| 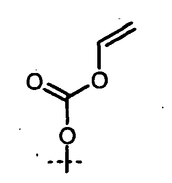 | 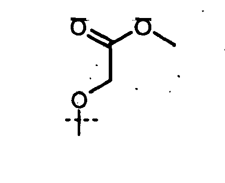 | 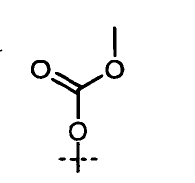 | 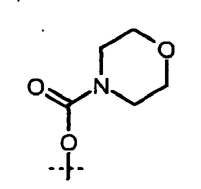 | 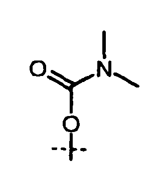 |
| 1) | 2) | 3) | 4) | 5) |
| 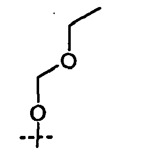 6) | 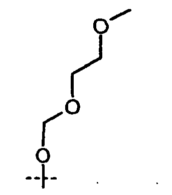 | 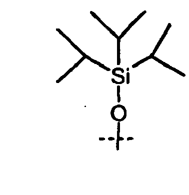 | 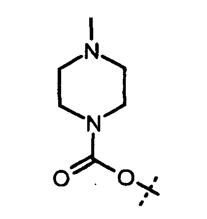 | 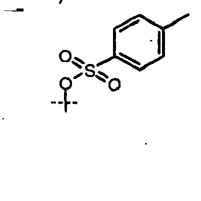 |
| | 7) | 8) | 9) | 10) |
| 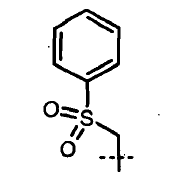 | 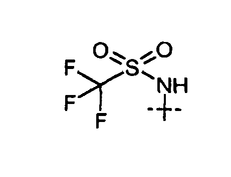 | 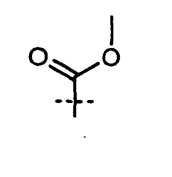 | 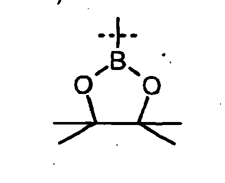 | 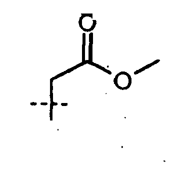 |
| 11) | 12) | 13) | 14) | 15) |
| 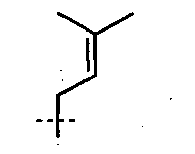 | 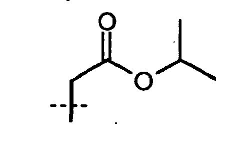 | 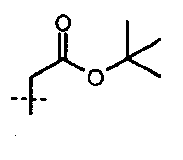 | 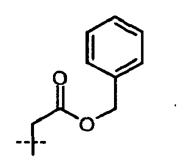 | 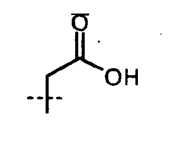 |
| 16) | 17) | 18) | 19) | 20) |
| 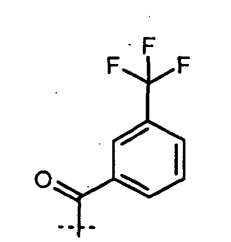 | 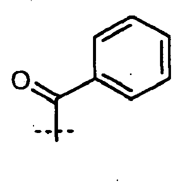 | 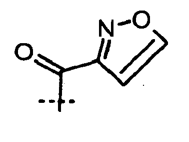 | 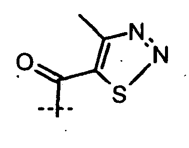 | 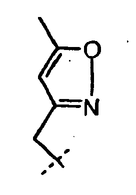 |
| 21) | 22) | 23) | 24) | 25) |
| 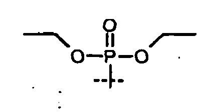 | 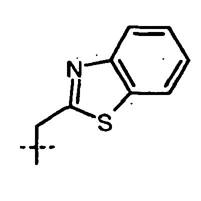 | 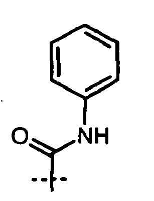 | 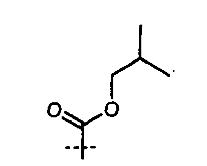 | 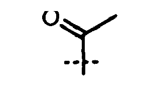 |
| 26) | 27) | 28) | 29) | 30) |
| 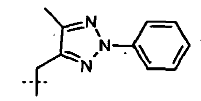 | 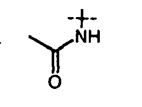 | 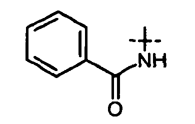 | 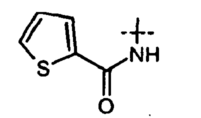 | 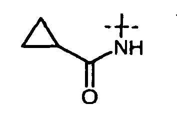 |
| 31) | 32) | 33) | 34) | 35) |
| 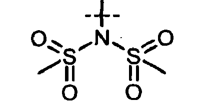 | 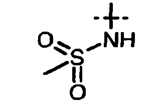 | 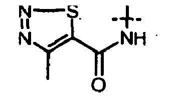 | 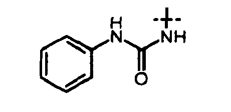 | 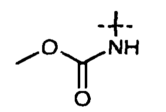 |
| 36) | 37) | 38) | 39) | 40) |
| 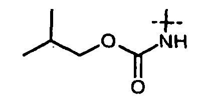 | 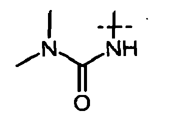 | 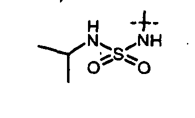 | 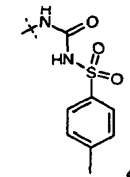 | 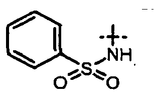 |
| 41) | 42) | 43) | 44) | 45) |
| 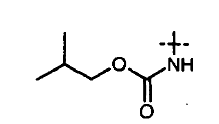 | 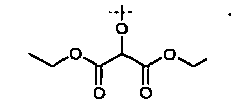 | 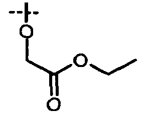 | 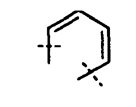 | 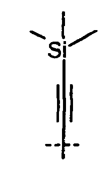 |
| 46) | 47) | 48) | 49) | 50) |
| 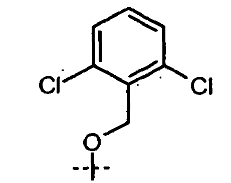 | 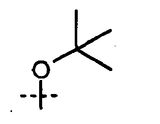 | 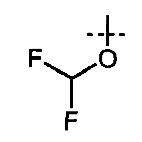 | 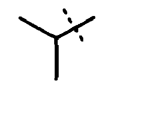 | 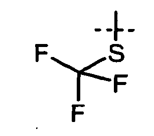 |
| 51) | 52) | 53) | 54) | 55) |
| 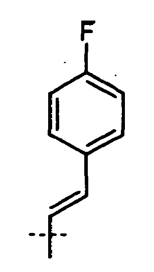 | 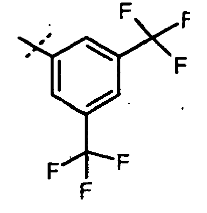 | 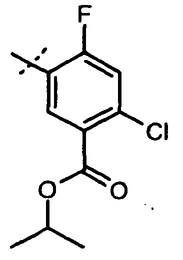 | 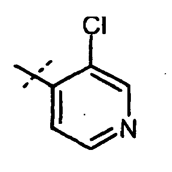 | 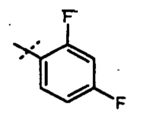 |
| 56) | 57) | 58) | 59) | 60) |
| 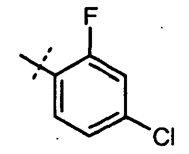 | 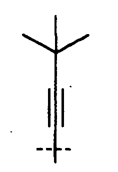 | 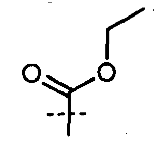 | 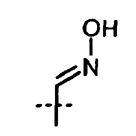 | 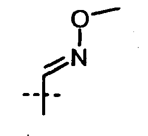 |
| 61) | 62) | 63) | 64) | 65) |
| 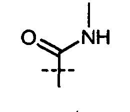 | 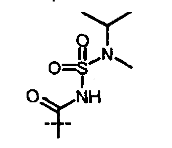 | 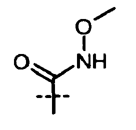 | 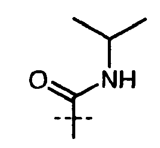 | 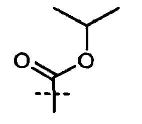 |
| 66) | 67) | 68) | 69) | 70) |
| 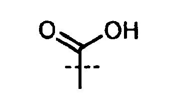 | 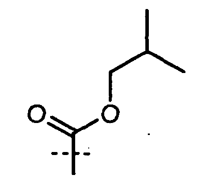 | 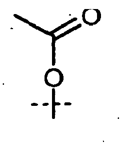 | 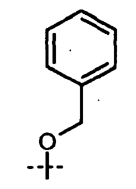 | 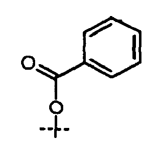 |
| 71) | 72) | 73) | 74) | 75) |
| 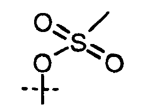 | 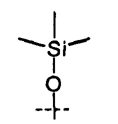 | 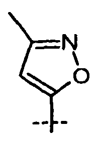 | 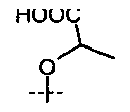 | 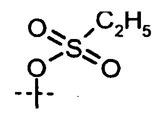 |
| 76) | 77) | 78) | 79) | 80) |

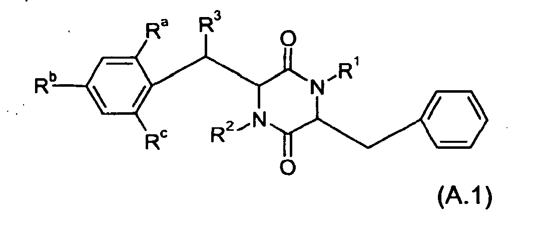

**Tabelle A.1**

| Nr. | R^{a} | R^{b} | R^{c} | R¹ | R² | R³ | RT HPLC/MS or m.p. | Diast. |
|---|---|---|---|---|---|---|---|---|
| | | | | | | | 3,187 min | |
| A.1.1 | NO₂ | CF₃ | H | CH₃ | CH₃ | H | m/z=435,9 | Diast. 2 |
| | | | | | | | [M+H]⁺ | |
| | | | | | | | 3,079 min | |
| A.1.2 | NO₂ | CF₃ | H | CH₃ | CH₃ | H | m/z=435,9 | Diast. 1 |
| | | | | | | | [M+H]⁺ | |
| | | | | | | | 2,702 min | |
| A.1.3 | F | F | H | CH₃ | CH₃ | H | m/z=359,1 | Diast. 1 |
| | | | | | | | [M+H]⁺ | |
| | | | | | | | 2,962 min | |
| A.1.4 | Cl | F | H | CH₃ | CH₃ | H | m/z=375,3 | Diast. 1 |
| | | | | | | | [M+H]⁺ | |
| | | | | | | | 2,737 min | |
| A.1.5 | F | F | F | CH₃ | CH₃ | H | m/z=377,2 | Diast. 1 |
| | | | | | | | [M+H]⁺ | |
| | | | | | | | 2,808 min | |
| A.1.6 | F | F | H | CH₃ | CH₃ | H | m/z=359,1 | Diast. 2 |
| | | | | | | | [M+H]+ | |
| | | | | | | | 3,042 min | |
| A.1.7 | Cl | F | H | CH₃ | CH₃ | H | m/z=375,4 | Diast. 2 |
| | | | | | | | [M+H]⁺ | |
| | | | | | | | 2,950 min | |
| A.1.8 | F | F | F | CH₃ | CH₃ | H | m/z=377,1 | Diast. 2 |
| | | | | | | | [M+H]⁺ | |
| | | | | | | | 2,808 min | |
| A.1.9 | NO₂ | F | H | CH₃ | CH₃ | H | m/z=386,0 | Diast. 2 |
| | | | | | | | [M+H]⁺ | |
| | | | | | | | 3,068 min | |
| A.1.10 | NO₂ | Cl | H | CH₃ | CH₃ | H | m/z=402,0 | Diast. 2 |
| | | | | | | | [M+H]⁺ | |
| | | | | | | | 2,716 min | |
| A.1.11 | NO₂ | F | H | CH₃ | CH₃ | H | m/z=386,0 | Diast. 1 |
| | | | | | | | [M+H]⁺ | |
| | | | | | | | 2,938 min | |
| A.1.12 | NO₂ | Cl | H | CH₃ | CH₃ | H | m/z=402,0 | Diast. 1 |
| | | | | | | | [M+H]⁺ | |
| | | | | | | | 2,324 min | |
| A.1.13 | NO₂ | 79) | H | CH₃ | CH₃ | H | m/z=455 | |
| | | | | | | | [M⁺] | |

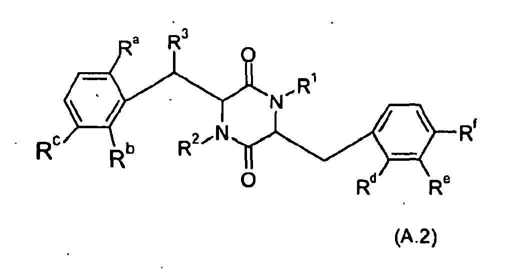

**Tabelle A.2**

| Nr. | R^{a} | R^{b} | R^{c} | R¹ | R² | R³ | R^{d} | R^{e} | R^{f} | RT HPLC/MS oder m.p. | Diast. |
|---|---|---|---|---|---|---|---|---|---|---|---|
| A.2.1 | NO₂ | H | H | CH₃ | CH₃ | 1) | H | H | H | 65°C | |
| A.2.2 | NO₂ | H | H | CH₃ | CH₃ | 2) | H | H | H | 130°C | |
| A.2.3 | NO₂ | H | H | CH₃ | CH₃ | 3) | H | H | H | 74°C | |
| A.2.4 | NO₂ | H | H | CH₃ | CH₃ | 4) | H | H | H | 207°C | |
| A.2.5 | NO₂ | H | H | CH₃ | CH₃ | 5) | H | H | H | 176°C | |
| A.2.6 | NO₂ | H | H | CH₃ | CH₃ | 6) | H | H | H | 117°C | |
| A.2.7 | NO₂ | H | H | CH₃ | CH₃ | 7) | H | H | H | m/z=472,1 [M+H]⁺ | |
| A.2.8 | NO₂ | H | H | CH₃ | CH₃ | 8) | H | H | H | 155°C | |
| A.2.9 | NO₂ | H | H | CH₃ | CH₃ | 9) | H | H | H | 201 °C | |
| | | | | | | | | | | 2,483 min | |
| A.2.10 | NO₂ | H | H | NH₂ | CH₃ | H | H | H | H | m/z=369,2 [M+H]⁺ | Diast. 1 |
| | | | | | | | | | | 177°C | |
| A.2.11 | NO₂ | H | H | CH₃ | CH₃ | 1) | H | H | H | 120°C | |
| A.2.12 | NO₂ | H | H | CH₃ | CH₃ | 3) | H | H | H | 124°C | |
| A.2.13 | NO₂ | H | H | CH₃ | CH₃ | 4) | H | H | H | 184°C | |
| A.2.14 | NO₂ | H | H | CH₃ | CH₃ | 5) | H | H | H | 118°C | |
| A.2.15 | NO₂ | H | H | CH₃ | CH₃ | 5) | H | H | H | 68°C | |
| A.2.16 | NO₂ | H | H | CH₃ | CH₃ | 4) | H | H | H | 162°C | |
| A.2.17 | NO₂ | H | H | CH₃ | CH₃ | 3) | H | H | H | 140°C | |
| A.2.18 | NO₂ | H | H | CH₃ | CH₃ | 1) | H | H | H | 139°C | |
| A.2.19 | NO₂ | H | H | CH₃ | CH₃ | 5) | H | H | H | 68°C | |
| A.2.20 | NO₂ | H | H | CH₃ | CH₃ | 4) | H | H | H | 162°C | |
| A.2.21 | NO₂ | H | H | CH₃ | CH₃ | 1) | H | H | H | 129°C | |
| A.2.22 | NO₂ | H | H | CH₃ | CH₃ | 10) | H | H | H | 3,378 min m/z=538,0 [M+H]⁺ | Diast. 2 |
| A.2.23 | NO₂ | H | H | CH₃ | CH₃ | 10) | H | H | H | 3,352 min m/z=538,0 [M+H]⁺ | Diast. 1 |
| A.2.24 | NO₂ | H | H | CH₃ | CH₃ | 10) | H | H | H | 3,354 min m/z=538,0 [M+H]⁺ | Diast. 3 |
| A.2.25 | NO₂ | H | H | H | CH₃ | H | H | H | H | 2,579 min m/z=354,0 [M+H]⁺ | Diast. 1 |
| A.2.26 | NO₂ | H | H | H | CH₃ | H | H | H | H | 2,651 min m/z=354,0 [M+H]⁺ | Diast. 2 |
| A.2.27 | F | H | H | CH₃ | CH₃ | H | H | H | H | 2,867 min m/z=341,1 [M+H]⁺ | |
| A.2.28 | Cl | H | H | CH₃ | CH₃ | H | H | H | H | 3,134 min m/z=357,2 [M+H]⁺ | |
| A.2.29 | Ph | H | H | CH₃ | CH₃ | H | H | H | H | 3,400 min m/z=399,1 [M+H]⁺ | Diast. 1 |
| A.2.30 | 11) | H | H | CH₃ | CH₃ | H | H | H | H | 2,873 min m/z=477,1 [M+H]⁺ | Diast. 2 |
| A.2.31 | Ph | H | H | CH₃ | CH₃ | H | H | H | H | 3,416 min m/z=399,1 [M+H]⁺ | Diast. 2 |
| A.2.32 | 11) | H | H | CH₃ | CH₃ | H | H | H | H | 2,823 min m/z=477,1 [M+H]⁺ | Diast. 1 |
| A.2.33 | NO₂ | H | H | CH₃ | CH₃ | H | H | H | H | 2,536 min m/z=368,0 [M+H]⁺ | Diast. 1 |
| A.2.34 | NO₂ | H | H | CH₃ | CH₃ | H | H | H | H | 2,640 min m/z=368,0 [M+H]⁺ | Diast. 2 |
| A.2.35 | NO₂ | F | H | CH₃ | CH₃ | H | H | H | H | 2,733 min m/z=386,1 [M+H]⁺ | Diast. 2 |
| A.2.36 | NO₂ | H | H | H | CH₃ | H | F | H | H | 2,496 min m/z=371,9 [M+H]⁺ | Diast. 2 |
| A.2.37 | NO₂ | H | H | CH₃ | CH₃ | H | F | H | H | 2,688 min m/z=386,0 [M+H]⁺ | |
| A.2.38 | NH₂ | H | H | CH₃ | CH₃ | H | H | H | H | 2,026 min m/z=338,0 [M+H]⁺ | |
| A.2.39 | F | H | H | H | CH₃ | H | CF₃ | H | H | 2,872 min m/z=394,9 [M+H]⁺ | Diast. 1 |
| A.2.40 | 12) | H | H | CH₃ | CH₃ | H | H | H | H | 3,186 min m/z=469,9 [M+H]⁺ | |
| A.2.41 | NO₂ | H | H | H | CH₃ | H | NO₂ | H | H | 2,479 min m/z=398,9 [M+H]⁺ | Diast. 2 |
| A.2.42 | F | H | H | CH₃ | CH₃ | H | CF₃ | H | H | 3,160 min m/z=409,4 [M+H]⁺ | |
| A.2.43 | NO₂ | H | H | H | CH₃ | H | H | F | H | 2,561 min m/z=371,9 [M+H]⁺ | Diast. 2 |
| A.2.44 | NO₂ | H | H | CH₃ | CH₃ | H | H | F | H | 2,729 min m/z=385,9 [M+H]⁺ | |
| | | | | | | | | | | 2,624 min | |
| A.2.45 | NO₂ | F | H | CH₃ | CH₃ | H | H | H | H | m/z=386,1 | Diast. 1 |
| | | | | | | | | | | [M+Na]⁺ | |
| A.2.46 | NO₂ | H | H | H | CH₃ | H | F | H | H | 2,468 min m/z=371,9 [M+H]⁺ | Diast. 1 |
| A.2.47 | NO₂ | H | CH₃O- | CH₃ | CH₂ | H | H | H | H | 2,571 min m/z=428,0 [M+H]⁺ | Diast. 1 |
| A.2.48 | NO₂ | H | H | CH₃ | CH₃ | H | F | H | H | 2,694 min m/z=385,9 [M+H]⁺ | |
| A.2.49 | F | H | H | H | CH₃ | H | CF₃ | H | H | 2,921 min m/z=394,9 [M+H]⁺ | Diast. 2 |
| A.2.50 | NO₂ | H | H | H | CH₃ | H | NO₂ | H | H | 2,464 min m/z=398,9 [M+H]⁺ | Diast. 1 |
| A.2.51 | F | H | H | CH₃ | CH | H | CF₃ | H | H | 3,071 min m/z=408,9 [M+H]⁺ | |
| A.2.52 | NO₂ | H | H | H | CH₃ | H | H | F | H | 2,509 min m/z=371,9 [M+H]⁺ | Diast. 1 |
| A.2.53 | NO₂ | H | H | CH₃ | CH₃ | H | H | F | H | 2,630 min m/z=385,9 [M+H]⁺ | |
| A.2.54 | 13) | H | H | CH₃ | CH₃ | H | H | H | H | 2,547 min m/z=381,0 [M+H]⁺ | Diast. 1 |
| A.2.55 | 13) | H | H | CH₃ | CH₃ | H | H | H | H | 2,769 min m/z=381,0 [M+H]⁺ | Diast. 2 |
| A.2.56 | 14) | H | H | CH₃ | CH₃ | H | H | H | H | m/z=449,1 [M+H]⁺ | Diast. 2 |
| A.2.57 | NO₂ | H | H | PhCH₂- | CH₃ | H | H | H | H | 3,398 min m/z=444,1 [M+H}⁺ | Diast. 1 |
| A.2.58 | NO₂ | H | H | 15) | CH₃ | H | H | H | H | 2,932 min m/z=426,1 [M+H]⁺ | Diast. 2 |
| A.2.59 | NO₂ | H | H | 16) | CH₃ | H | H | H | H | 3,361 min m/z=422,2 [M+H]⁺ | Diast. 1 |
| A.2.60 | NO₂ | H | H | 17) | CH₃ | H | H | H | H | 3,337 min m/z=454,1 [M+H]⁺ | Diast. 2 |
| A.2.61 | 14) | H | H | CH₃ | CH₃ | H | H | H | H | m/z=448,9 [M+H]⁺ | Diast. 1 |
| A.2.62 | NO₂ | H | H | PhCH₂- | CH₃ | H | H | H | H | 3,459 min m/z=444,1 [M+H]⁺ | Diast. 2 |
| A.2.63 | NO₂ | H | H | 15) | CH₃ | H | H | H | H | 2,928 min m/z=426,1 [M+H]⁺ | Diast. 1 |
| A.2.64 | NO₂ | H | H | 16) | CH₂ | H | H | H | H | 3,420 min m/z=422,1 [M+H]⁺ | Diast. 2 |
| A.2.65 | NO₂ | H | H | 17) | CH₃ | H | H | H | H | 3,285 min m/z=454,1 [M+H]⁺ | Diast. 1 |
| | | | | | | | | | | 3,528 min | |
| A.2.66 | NO₂ | H | H | 18) | CH₃ | H | H | H | H | m/z=490.1 | Diast. 2 |
| | | | | | | | | | | [M+Na]⁺ | |
| | | | | | | | | | | 3,458 min | |
| A.2.67 | NO₂ | H | H | 18) | CH₃ | H | H | H | H | m/z=490,1 | Diast. 1 |
| | | | | | | | | | | [M+Na]⁺ | |
| A.2.68 | NO₂ | H | H | 19) | CH₃ | H | H | H | H | 3,578 min m/z=502,5 [M+H]⁺ | Diast. 2 |
| A.2.69 | NO₂ | H | H | 20) | CH₃ | H | H | H | H | 2,634 min m/z=412,1 [M+H]⁺ | |
| A.2.70 | NO₂ | H | H | 19) | CH₃ | H | H | H | H | 3,547 min m/z=502,5 [M+H]⁺ | Diast. 1 |
| A.2.71 | NO₂ | H | H | 20) | CH₃ | H | H | H | H | 2,634 min m/z=412,1 [M+H]⁺ | |
| A.2.72 | NO₂ | H | H | 21) | CH₃ | H | H | H | H | 3,844 min m/z=526,5 [M+H]⁺ | Diast. 1 |
| A.2.73 | NO₂ | H | H | 22) | CH₃ | H | H | H | H | 3,516 min m/z=458,4 [M+H]⁺ | Diast. 2 |
| A.2.74 | NH₂ | H | H | CH₃ | CH₃ | H | H | H | H | 1,801 min m/z=338,2 [M+H]⁺ | Diast. 1 |
| A.2.75 | NO₂ | H | H | 22) | CH₃ | H | H | H | H | 3,511 min m/z=458,4 [M+H]⁺ | Diast. 1 |
| A.2.76 | NO₂ | H | H | 21) | CH₃ | H | H | H | H | 3,844 min m/z=526,5 [M+H]⁺ m/z=526.5[M+H]⁺ | Diast. 2 |
| A.2.77 | NO₂ | H | H | 23) | CH₃ | H | H | H | H | 3,252 min m/z=449,1 [M+H]⁺ | |
| A.2.78 | NO₂ | H | H | 24) | CH₃ | H | H | H | H | 3,411 min m/z=480,4 [M+H]⁺ | |
| | | | | | | | | | | 3,022 min | |
| A.2.79 | NO₂ | H | H | 25) | CH₃ | H | H | H | H | m/z=449,1 [M+H]⁺, | Diast. 1 |
| | | | | | | | | | | 137°C | |
| A.2.80 | NO₂ | H | H | 25) | CH₃ | H | H | H | H | 3,076 m/z=449,1 [M+H]⁺ | Diast. 2 |
| A.2.81 | CN | H | H | CH₃ | CH₃ | H | H | H | H | m/z=348,1 [M+H]⁺ | Diast. 1 |
| A.2.82 | CN | H | H | CH₃ | CH₃ | H | H | H | H | m/z=348,1 [M+H]⁺ | Diast. 2 |
| A.2.83 | NO₂ | H | H | H | CH₃ | H | Br | H | H | m/z=433,8 [M+H]⁺ | Diast. 1 |
| A.2.84 | NO₂ | H | H | H | CH₃ | H | H | Br | H | m/z=433,8 [M+H]⁺ | Diast. 1 |
| A.2.85 | NO₂ | H | H | H | CH₃ | H | Br | H | H | m/z=433,8 [M+H]⁺ | Diast. 2 |
| A.2.86 | NO₂ | H | H | H | CH₃ | H | H | Br | H | m/z=433,8 [M+H]⁺ | Diast. 2 |
| A.2.87 | 26) | H | H | CH₃ | CH₃ | H | H | H | H | 2,749 min m/z=459,1 [M+H]⁺ | |
| A.2.88 | NO₂ | H | H | 13) | CH₃ | H | H | H | H | 2,969 min m/z=412,1 [M+H]⁺ | |
| A.2.89 | NO₂ | H | H | HO-CH₂- | CH₃ | H | H | H | H | 2,565 min m/z=384,1 [M+H]⁺ | |
| | | | | | | | | | | 3,419 min | |
| A.2.90 | NO₂ | H | H | 27) | CH₃ | H | H | H | H | m/z=501,1 [M+H]⁺, | |
| | | | | | | | | | | 164°C | |
| A.2.91 | NO₂ | H | H | 28) | CH₃ | H | H | H | H | 3,011 min m/z=473,1 [M+H]⁺ | |
| A.2.92 | NO₂ | H | H | 29) | CH₃ | H | H | H | H | 3,562 min m/z=454,1 [M+H]⁺ | |
| A.2.93 | NO₂ | H | H | 30) | CH₃ | H | H | H | H | 3,115 min m/z=396,1 [M+H]⁺ | |
| A.2.94 | NO₂ | H | H | 31) | CH₃ | H | H | H | H | 3,728 min m/z=525,2 [M+H]⁺ | |
| A.2.95 | NO₂ | H | H | 13) | CH₃ | H | H | H | H | 2,998 min m/z=412,1 [M+H]⁺ | |
| A.2.96 | NO₂ | H | H | HO-CH₂- | CH₃ | H | H | H | H | 2,637 min m/z=384,4 [M+H]⁺ | |
| A.2.97 | I | H | H | CH₃ | CH₃ | H | H | H | H | 3,101 min m/z=449,0 [M+H]⁺ | |
| A.2.98 | B(OH)₂ | H | H | CH₃ | CH₃ | H | H | H | H | 2,260 min m/z=367,1 [M+H]⁺ | |
| | | | | | | | | | | 2,188 min | |
| A.2.99 | NH₂ | H | H | CH₃ | CH₃ | H | H | H | H | m/z=380,1 [M+H]⁺, | |
| | | | | | | | | | | 161°C | |
| | | | | | | | | | | 2,251 min | |
| A.2.100 | 32) | H | H | CH₃ | CH₃ | H | H | H | H | m/z=380,1 [M+H]⁺, | |
| | | | | | | | | | | 145°C | |
| | | | | | | | | | | 2,876 min | |
| A.2.101 | 33) | H | H | CH₃ | CH₃ | H | H | H | H | m/z=442,1 [M+H]⁺, | |
| | | | | | | | | | | 186°C | |
| | | | | | | | | | | 2,838 min | |
| A.2.102 | 34) | H | H | CH₃ | CH₃ | H | H | H | H | m/z=448,1 [M+H]⁺, | |
| | | | | | | | | | | 99°C | |
| A.2.103 | 35) | H | H | CH₃ | CH₃ | H | H | H | H | 2,549 min | |
| A.2.104 | 36) | H | H | CH₃ | CH₃ | H | H | H | H | 2,662 min m/z=494,0 [M+H]⁺ | |
| A.2.105 | 37) | H | H | CH₃ | CH₃ | H | H | H | H | 2,431 min m/z=416,1 [M+H]⁺ | |
| A.2.106 | 38) | H | H | CH₃ | CH₃ | H | H | H | H | 2,781 min m/z=464,1 [M+H]⁺ | |
| | | | | | | | | | | 3,053min | |
| A.2.107 | 39) | H | H | CH₃ | CH₃ | H | H | H | H | m/z=457,1 [M+H]⁺, | |
| | | | | | | | | | | 182°C | |
| A.2.108 | 40) | H | H | CH₃ | CH₃ | H | H | H | H | 2,573 min m/z=396,1 [M+H]⁺ | |
| A.2.109 | 41) | H | H | CH₃ | CH₃ | H | H | H | H | 3,227 min m/z=438,2 [M+H]⁺ | |
| A.2.110 | 42) | H | H | CH₃ | CH₃ | H | H | H | H | 2,310 min m/z=409,2 [M+H]⁺ | |
| A.2.111 | 43) | H | H | CH₃ | CH₃ | H | H | H | H | 2,842 min m/z=459,1 [M+H]⁺ | |
| A.2.112 | 44) | H | H | CH₃ | CH₃ | H | H | H | H | 2,988 min m/z=535,1 [M+H]⁺ | |
| | | | | | | | | | | 3,070 min | |
| A.2.113 | 45) | H | H | CH₃ | CH₃ | H | H | H | H | m/z=478,0 [M+H]⁺, | |
| | | | | | | | | | | 227°C | |
| A.2.114 | 46) | H | H | CH₃ | CH₃ | H | H | H | H | 3,297 min | |
| A.2.115 | NO₂ | H | H | N≡C- | CH₃ | H | H | H | H | 3,079 min m/z=401,0 [M+H]⁺ | |
| A.2.116 A | NO₂ | H | H | N≡C-CH₂- | CH₃ | H | H | H | H | 2,876 min m/z=393,1 [M+H]⁺ | |
| A.2.117 | NO₂ | H | H | 23) | CH₃ | H | H | H | H | 3,290 min m/z=449,2 [M+H]⁺ | |
| A.2.118 | NO₂ | H | H | 24) | CH₃ | H | H | H | H | 3,373 min m/z=480,0 [M+H]⁺ | |
| A.2.119 | HO- | H | H | CH₃ | CH₃ | H | H | H | H | 2,460 min m/z=339,1 [M+H]⁺ | |
| A.2.120 | 47) | H | H | CH₃ | CH₃ | H | H | H | H | 3,207 min m/z=497,1 [M+H]⁺ | |
| A.2.121 | 48) | H | H | CH₃ | CH₃ | H | H | H | H | 2,975 min m/z=425,2 [M+H]⁺ | |
| A.2.122 | NO₂ | H | H | CH₃ | CH₃ | H | Br | H | H | 2,979 min m/z=448,1 [M+H]⁺ | Diast. 1 |
| A.2.123 | NO₂ | H | H | CH₃ | CH₃ | H | H | Br | H | 3,055 min m/z=448,1 [M+H]⁺ | Diast. 2 |
| A.2.124 | 73) | H | H | CH₃ | CH₃ | H | H | H | H | 2,629 min m/z=381,0 [M+H]⁺ | |
| A.2.125 | HO- | H | H | CH₃ | CH₃ | H | H | H | H | 2,330 min m/z=339,1 [M+H]⁺ | |
| A.2.126 | NO₂ | H | H | CH₃ | CH₃ | H | Br | H | H | 2,984 min m/z=448,1 [M+H]⁺ | Diast. 2 |
| A.2.127 | NO₂ | H | H | CH₃ | CH₃ | H | H | Br | H | 2,989 min m/z=448,1 [M+H]⁺ | Diast. 1 |
| A.2.128 | NO₂ | H | H | H | CH₃ | H | 49) | 49) | H | 2,994 min m/z=404,1 [M+H]⁺ | Diast. 2 |
| A.2.129 | NO₂ | H | H | H | CH₃ | H | H | CN | H | 2,417 min m/z=379,1 [M+H]⁺ | Diast. 1 |
| A.2.130 | NO₂ | H | H | H | CH₃ | H | H | NO₂ | H | 2,541 min m/z=399,1 [M+H]⁺ | Diast. 1 |
| A.2.131 | NO₂ | H | H | H | CH₃ | H | H | H | H | 2,543 min m/z=399,1 [M+H]⁺ | Diast. 1 |
| A.2.132 | NO₂ | H | H | CH₃ | CH₃ | H | 26) | H | H | 2,779 min m/z=504,1 [M+H]⁺ | |
| A.2.133 | NO₂ | Br | H | CH₃ | CH₃ | H | H | H | H | 3,034 min m/z=446,1 [M+H]⁺ | Diast. 2 |
| A.2.134 | 50) | H | H | CH₃ | CH₃ | H | H | H | H | 3,886 min m/z=419,2 [M+H]⁺ | |
| A.2.135 | HC≡C- | H | H | CH₃ | CH₃ | H | H | H | H | 2,860 min m/z=347,0 [M+H]⁺ | |
| A.2.136 | NO₂ | Br | H | CH₃ | CH₃ | H | H | H | H | 2,901 min m/z=445,9 [M+H]⁺ | Diast. 1 |
| A.2.137 | NO₂ | H | H | H | CH₃ | H | 49) | 49) | H | 2,934 min m/z=404,1 [M+H]⁺ | Diast. 1 |
| A.2.138 | NO₂ | H | H | H | CH₃ | H | H | CN | H | 2,435 min m/z=379,1 [M+H]⁺ | Diast. 2 |
| A.2.139 | NO₂ | H | H | H | CH₃ | H | H | NO₂ | H | 2,569 min m/z=399,1 [M+H]⁺ | Diast. 2 |
| A.2.140 | NO₂ | H | H | H | CH₃ | H | H | H | NO₂ | 2,549 min m/z=399,1 [M+H]⁺ | Diast. 2 |
| A.2.141 | NO₂ | H | H | H | CH₃ | H | H | H | 51) | 3,473 min m/z=527,9 [M+H]⁺ | Diast. 2 |
| A.2.142 | NO₂ | H | H | H | CH₃ | H- | H | H | I | 2,951 min m/z=479,8 [M+H]⁺ | Diast. 1 |
| A.2.143 | NO₂ | H | H | H | CH₃ | H | H | H | F | 2,553 min m/z=372,0 [M+H]⁺ | Diast. 1 |
| | | | | | | | | | | 2,027 min | |
| A.2.144 | NO₂ | H | H | H | CH₃ | H | H | H | 52) | m/z=370,0 [M+H]⁺ | Diast. 2 |
| | | | | | | | | | | tert-butyl-Rest | |
| A.2.145 | NO₂ | H | H | H | CH₃ | H | H | CH₃ | H | 2,698 min m/z=368,0 [M+H]⁺ | Diast. 1 |
| A.2.146 | NO₂ | H | H | H | CH₃ | H | Cl | H | H | 2,758 min m/z=388,1 [M+H]⁺ | Diast. 2 |
| A.2.147 | NO₂ | H | H | H | CH₃ | H | H | 49) | 49) | 2,969 min m/z=404,2 [M+H]⁺ | Diast. 2 |
| A.2.148 | NO₂ | H | H | H | CH₃ | H | H | H | Cl | 2,812 min m/z=388,1 [M+H]⁺ | Diast. 1 |
| A.2.149 | NO₂ | H | H | H | CH₃ | H | H | H | CH₃ | 2,734 min m/z=368,2 [M+H]⁺ | Diast. 1 |
| A.2.150 | NO₂ | H | H | H | CH₃ | H | I | H | H | 2,736 min m/z=368,2 [M+H]⁺ | Diast. 2 |
| A.2.151 | 53) | H | H | CH₃ | CH₃ | H | H | H | H | 2,866 min m/z=389,1 [M+H]⁺ | Diast. 1 |
| A.2.152 | Cl | H | Cl | CH₃ | CH₃ | H | H | H | H | 3,168 min m/z=391,1 [M+H]⁺ | Diast. 1 |
| A.2.153 | Cl | Cl | H | CH₃ | CH₃ | H | H | H | H | 3,129 min m/z=391,4 [M+H]⁺ | Diast. 1 |
| A.2.154 | Cl | H | CF₃ | CH₃ | CH₃ | H | H | H | H | 3,308 min m/z=425,0 [M+H]⁺ | Diast. 1 |
| A.2.155 | F | H | Cl | CH₃ | CH₃ | H | H | H | H | 2,933 min m/z=375,1 [M+H]⁺ | Diast. 1 |
| A.2.156 | Br | H | F | CH₃ | CH₃ | H | H | H | H | 3,038 min m/z=421,1 [M+H]⁺ | Diast. 1 |
| A.2.157 | F | Cl | F | CH₃ | CH₃ | H | H | H | H | 2,965 min m/z=393,3 [M+H]⁺ | Diast. 1 |
| A.2.158 | 55) | H | H | CH₃ | CH₃ | H | H | H | H | 3,324 min m/z=423,2 [M+H]⁺ | Diast. 1 |
| A.2.159 | F | F | CH₃ | CH₃ | CH₃ | H | H | H | H | 2,859, min m/z=373,2 [M+H]⁺ | Diast. 1 |
| A.2.160 | F | H | CF₃ | CH₃ | CH₃ | H | H | H | H | 3,116 min m/z=409,2 [M+H]⁺ | Diast. 1 |
| A.2.161 | F | Cl | CH₃ | CH₃ | CH₃ | H | H | H | H | 3,137 min m/z=389,4 [M+H]⁺ | Diast. 1 |
| A.2.162 | F | F | H | CH₃ | CH₃ | H | H | H | H | 2,625 min m/z=359,2 [M+H]⁺ | Diast. 1 |
| A.2.163 | F | Cl | H | CH₃ | CH₃ | H | H | H | H | 2,959 min m/z=375,3 [M+H]⁺ | Diast. 1 |
| A.2.164 | I | H | H | CH₃ | CH₃ | H | H | H | H | 3,046 min m/z=448,9 [M+H]⁺ | Diast. 1 |
| A.2.165 | NO₂ | H | H | H | CH₃ | H | H | H | 51) | 3,366 min m/z=527,9 [M+H]⁺ | Diast. 1 |
| A.2.166 | NO₂ | H | H | H | CH₃ | H | H | H | I | 2,929 min m/z=479,8 [M+H]⁺ | Diast. 2 |
| A.2.167 | NO₂ | H | H | H | CH₃ | H | H | H | F | 2,585 min m/z=372,0 [M+H]⁺ | Diast. 2 |
| A.2.168 | NO₂ | H | H | H | CH₃ | H | H | H | 52) | 2,013 min m/z=370,0 [M+H]⁺ | Diast. 1 |
| A.2.169 | NO₂ | H | H | H | CH₃ | H | H | CH₃ | H | 2,724 min m/z=368.0 [M+H]⁺ | Diast. 2 |
| A.2.170 | NO₂ | H | H | H | CH₃ | H | Cl | H | H | 2,747 min m/z=388,1 [M+H]⁺ | Diast. 1 |
| A.2.171 | NO₂ | H | H | H | CH₃ | H | H | 49) | 49) | 2,941 min m/z=404,2 [M+H]⁺ | Diast. 1 |
| A.2.172 | NO₂ | H | H | H | CH₃ | H | H | H | Cl | 2,823 min m/z=388,1 [M+H]⁺ | Diast. 2 |
| A.2.173 | NO₂ | H | H | H | CH₃ | H | H | H | CH₃ | 2,759 min m/z=368,2 [M+H]⁺ | Diast. 2 |
| A.2.174 | NO₂ | H | H | H | CH₃ | H | I | H | H | 2,720 min m/z=368,2 [M+H]⁺ | Diast. 1 |
| A.2.175 | 53) | H | H | CH₃ | CH₃ | H | H | H | H | 2,931 min m/z=389,1 [M+H]⁺ | Diast. 2 |
| A.2.176 | F | H | H | CH₃ | CH₃ | H | H | H | H | 2,808 min m/z=359,1 [M+H]⁺ | Diast. 2 |
| A.2.177 | Cl | H | Cl | CH₃ | CH₃ | H | H | H | H | 3,251 min m/z=391,1 [M+H]⁺ | Diast. 2 |
| A.2.178 | Cl | Cl | H | CH₃ | CH₃ | H | H | H | H | 3,189 min m/z=391,4 [M+H]⁺ | Diast. 2 |
| A.2.179 | Cl | H | CF₃ | CH₃ | CH₃ | H | H | H | H | 3,376 min m/z=424,9 [M+H]⁺ | Diast. 2 |
| A.2.180 | F | H | Cl | CH₃ | CH₃ | H | H | H | H | 3,068 min m/z=375.1 [M+H]⁺ | Diast. 2 |
| A.2.181 | Br | H | F | CH₃ | CH₃ | H | H | H | H | 3,089 min m/z=421,1 [M+H]⁺ | Diast. 2 |
| A.2.182 | F | Cl | F | CH₃ | CH₃ | H | H | H | H | 3,062 min m/z=393,3 [M+H]⁺ | Diast. 2 |
| A.2.183 | 55) | H | H | CH₃ | CH₃ | H | H | H | H | 3,327 min m/z=432,1 [M+H]⁺ | Diast. 2 |
| A.2.184 | F | F | CH₃ | CH₃ | CH₃ | H | H | H | H | 3,056 min m/z=373,2 [M+H]⁺ | Diast. 2 |
| A.2.185 | F | H | CF₃ | CH₃ | CH₃ | H | H | H | H | 3,226 min m/z=409,2 [M+H]⁺ | Diast. 2 |
| A.2.186 | F | Cl | CH₃ | CH₃ | CH₃ | H | H | H | H | 3,240 min m/z=389,4 [M+H]⁺ | Diast. 2 |
| A.2.187 | F | F | H | CH₃ | CH₃ | H | H | H | H | 2,832 min m/z=359,2 [M+H]⁺ | Diast. 2 |
| A.2.188 | F | Cl | H | CH₃ | CH₃ | H | H | H | H | 3,042 min m/z=375,4 [M+H]⁺ | Diast. 2 |
| A.2.189 | I | H | H | CH₃ | CH₃ | H | H | H | H | 3,071 min m/z=448.9 [M+H]⁺ | Diast. 2 |
| A.2.190 | NO₂ | H | H | H | CH₃ | H | CF₃ | H | H | 2,814 min m/z=422,4 [M+H]⁺ | Diast. 1 |
| A.2.191 | NO₂ | H | H | H | CH₃ | H | CF₃ | H | H | 2,956 min m/z=422,3 [M+H]⁺ | Diast. 2 |
| | | | | | | | | | | 3,625 min | |
| A.2.192 | 56) | H | H | CH₃ | CH₃ | H | H | H | H | m/z=465,0 | |
| | | | | | | | | | | [M+Na]⁺ | |
| A.2.193 | PO(OH)₂ | H | H | CH₃ | CH₃ | H | H | H | H | 1,942 min m/z=403,0 [M+H]⁺ | |
| A.2.194 | 56) | H | H | CH₃ | CH₃ | H | H | H | H | 3,665 min m/z=443,0 [M+H]⁺ | |
| A.2.195 | NO₂ | H | H | H | CH₃ | H | CN | H | H | 2,475 min m/z=379,1 [M+H]⁺ | Diast. 2 |
| A.2.196 | NO₂ | H | H | H | CH₃ | H | CN | H | H | 2,346 min m/z=379,0 [M+H]⁺ | Diast. 1 |
| A.2.197 | NO₂ | H | H | H | CH₃ | H | 57) | H | H | 3,709 min m/z=566,2 [M+H]⁺ | Diast. 1 |
| A.2.198 | NO₂ | H | H | H | CH₃ | H | 58) | H | H | 3,597 min m/z=568.2 [M+H]⁺ | Diast. 1 |
| A.2.199 | NO₂ | H | H | H | CH₃ | H | 59) | H | H | 126°C | |
| A.2.200 | NO₂ | H | H | H | CH₃ | H | 60) | H | H | 3,145 min m/z=466,2 [M+H]⁺ | |
| A.2.201 | NO₂ | H | H | H | CH₃ | H | H | 60) | H | 3,215 min m/z=466,0 [M+H]⁺ | Diast. 1 |
| A.2.202 | NO₂ | H | H | H | CH₃ | H | H | 57) | H | 3,793 min m/z=566,2 [M+H]⁺ | Diast. 1 |
| A.2.203 | NO₂ | H | H | H | CH₃ | H | H | 61) | H | 2,433 min m/z=481.9 [M+H]⁺ | Diast. 1 |
| | | | | | | | | | | 3,692 min | |
| A.2.204 | NO₂ | H | H | H | CH₃ | H | H | 58) | H | m/z=590,2 | Diast.1 |
| | | | | | | | | | | [M+Na]⁺ | |
| A.2.205 | N02 | H | H | H | CH₃ | H | H | 59) | H | 2,503 min m/z=465,2 [M+H]⁺ | Diast..2 |
| A.2.206 | 59) | H | H | CH₃ | CH₃ | H | H | H | H | 2,883 min m/z=434,1 [M+H]⁺ | |
| A.2.207 | 61) | H | H | CH₃ | CH₃ | H | H | H | H | 3,499 min m/z=451,2 [M+H]⁺ | |
| A.2.208 | 58) | H | H | CH₃ | CH₃ | H | H | H | H | 3,813 min m/z=537,2 [M+H]⁺ | |
| A.2.209 | NO₂ | H | H | H | CH₃ | H | 57) | H | H | 3,777 min m/z=565,9 [M+H]⁺ | Diast. 2 |
| A.2.210 | NO₂ | H | H | H | CH₃ | H | 60) | H | H | 121 °C | |
| A.2.211 | NO₂ | H | H | H | CH₃ | H | H | 60) | H | 3,229 min m/z=466,0 [M+H]⁺ | Diast..2 |
| A.2.212 | NO₂ | H | H | H | CH₃ | H | H | 57) | H | 3,853 min m/z=566,2 [M+H]⁺ | Diast. 2 |
| A.2.213 | NO₂ | H | H | H | CH₃ | H | H | 61) | H | 2,485 min m/z=482,2 [M+H]⁺ | Diast..2 |
| | | | | | | | | | | 3,697 min | |
| A.2.214 | NO₂ | H | H | H | CH₃ | H | H | 58) | H | m/z=590,1 | Diast.2 |
| | | | | | | | | | | [M+Na]⁺ | |
| A.2.215 | NO₂ | H | H | H | CH₃ | H | H | 59) | H | 2,406 min m/z=465,2 [M+H]⁺ | Diast. 1 |
| A.2.216 | 60) | H | H | CH₃ | CH₃ | H | H | H | H | 3,352 min m/z=435,2 [M+H]⁺ | Diast. 2 |
| A.2.217 | 61) | H | H | CH₃ | CH₃ | H | H | H | H | 3,576 min m/z=451.2[M+H]⁺ | Diast. 2 |
| A.2.218 | 58) | H | H | CH₃ | CH₃ | H | H | H | H | 3,864 min m/z=537,2 [M+H]⁺ | Diast..2 |
| A.2.219 | 62) | H | H | CH₃ | CH₃ | H | H | H | H | 3,695 min m/z=403,1 [M+H]⁺ | |
| A.2.220 | 1 | H | H | CH₃ | CH₃ | H | H | H | H | 3,014 min m/z=448,9 [M+H]⁺ | Diast. 1 |
| A.2.221 | I | H | H | CH₃ | CH₃ | H | H | H | H | 3,041 min m/z=448,9 [M+H]⁺ | Diast. 2 |
| A.2.222 | NO₂ | H | Cl | CH₃ | CH₃ | H | H | H | H | 3,035 min m/z=402,1 [M+H]⁺ | Diast. 2 |
| A.2.223 | 63) | H | H | CH₃ | CH₃ | H | H | H | H | 3,009 min m/z=395,1 [M+H]⁺ | Diast. 2 |
| A.2.224 | NO₂ | H₂C=CH | H | CH₃ | CH₃ | H | H | H | H | 2,981 min m/z=394,0 [M+H]⁺ | |
| A.2.225 | NO₂ | H | H | CH₃ | CH₃ | H | CN | H | H | 2,592 min m/z=393,2 [M+H]⁺ | Diast. 2 |
| A.2.226 | NO₂ | H | Cl | CH₃ | CH₃ | H | H | H | H | 2,919 min m/z=402,1 [M+H]⁺ | Diast. 1 |
| A.2.227 | 63) | H | H | CH₃ | CH₃ | H | H | H | H | 2,864 min m/z=395,2 [M+H]⁺ | Diast. 1 |
| A.2.228 | NO₂ | H | H | CH₃ | CH₃ | H | CN | H | H | 2,525 min m/z=393,2 [M+H]⁺, 184°C | Diast. 1 |
| A.2.229 | NO₂ | H | H | H₂N- | CH₃ | H | H | H | H | 2,599 min m/z=369,2 [M+H]⁺, 115°C | Diast. 2 |
| A.2.230 | NO₂ | Cl | 63) | CH₃ | CH₃ | H | H | H | H | 3,095 min m/z=474,1 [M+H]⁺ | Diast. 1 |
| A.2.231 | NO₂ | Cl | 13) | CH₃ | CH₃ | H | H | H | H | 2,882 min m/z=460,1 [M+H]⁺ | Diast. 1 |
| A.2.232 | NO₂ | H | H | CH₃ | CH₃ | H | C(O)NH₂ | H | H | 2,051 min m/z=41 1,0 [M+H]⁺ | |
| A.2.233 | NO₂ | H | H | CH₃ | CH₃ | H | COOH | H | H | 2,401 min m/z=412,0 [M+H]⁺ | |
| A.2.234 | NO₂ | Cl | 63) | CH₃ | CH₃ | H | H | H | H | 3,172 min m/z=474,1 [M+H]⁺ | Diast. 2 |
| A.2.235 | NO₂ | Cl | 13) | CH₃ | CH₃ | H | H | H | H | 2,970 min m/z=460,0 [M+H]⁺ | Diast. 2 |
| A.2.236 | C(O)H | H | H | CH₃ | CH₂ | H | H | H | H | 2,317 min m/z=351,0 [M+H]⁺ | Diast. 1 |
| A.2.237 | 64) | H | H | CH₃ | CH₃ | H | H | H | H | 2,184 min m/z=366,1 [M+H]⁺ | Diast. 1 |
| A.2.238 | 65) | H | H | CH₃ | CH₃ | H | H | H | H | 2,774 min m/z=380,1 [M+H]⁺ | Diast. 1 |
| A.2.239 | C(O)H | H | H | CH₃ | CH₃ | H | H | H | H | 2,564 min m/z=351,2 [M+H]⁺ | Diast. 2 |
| A.2.240 | 64) | H | H | CH₃ | CH₃ | H | H | H | H | 2,473 min m/z=366,1 [M+H]⁺ | Diast. 2 |
| A.2.241 | 65) | H | H | CH₃ | CH₃ | H | H | H | H | 2,943 min m/z=380,1 [M+H]⁺ | Diast. 2 |
| | | | | | | | | | | 1,828 min | |
| A.2.242 | 66) | H | H | CH₃ | CH₃ | H | H | H | H | m/z=336,0 1,920 | |
| | | | | | | | | | | min m/z=336,0 | |
| | | | | | | | | | | [M+H]⁺ | |
| A.2.243 | 67) | H | H | CH₃ | CH₃ | H | H | H | H | 2,864 min m/z=501,0 [M+H]⁺ | |
| A.2.244 | 68) | H | H | CH₃ | CH₃ | H | H | H | H | 2,152 min m/z=396,0 [M+H]⁺ | |
| A.2.245 | 69) | H | H | CH₃ | CH₃ | H | H | H | H | 2,512 min m/z=408,0 [M+H]⁺ | |
| A.2.246 | 70) | H | H | CH₃ | CH₃ | H | H | H | H | 3,307 min m/z=409,5 [M+H]⁺ | |
| A.2.247 | 71) | H | H | CH₃ | CH₃ | H | H | H | H | 2,402 min m/z=367,0 [M+H]⁺ | |
| A.2.248 | 72) | H | H | CH₃ | CH₃ | H | H | H | H | 3,528 min m/z=423,1 [M+H]⁺ | |
| A.2.249 | NO₂ | H | H | CH₃ | CH₃ | 73) | H | H | H | 2,778 min m/z=426,1 [M+H]⁺ | |
| A.2.250 | NO₂ | H | H | CH₃ | CH₃ | 73) | H | H | H | 2,828 min m/z=426,4 [M+H]⁺ | |
| A.2.251 | NO₂ | H | H | CH₃ | CH₃ | 73) | H | H | H | 2.851 min m/z=426,4 [M+H]⁺ | |
| A.2.252 | NO₂ | H | H | CH₃ | CH₃ | 73) | H | H | H | 2,827 min m/z=426,4 [M+H]⁺ | |
| A.2.253 | NO₂ | H | H | CH₃ | CH₃ | 74) | H | H | H | 3,426 min m/z=474,2 [M+H]⁺ | |
| A.2.254 | NO₂ | H | H | CH₃ | CH₃ | 74) | H | H | H | 3,481 min m/z=474,5 [M+H]⁺ | |
| A.2.255 | NO₂ | H | H | CH₃ | CH₃ | 74) | H | H | H | 3,483 min m/z=474,5 [M+H]⁺ | |
| A.2.256 | NO₂ | H | H | CH₃ | CH₃ | 74) | H | H | H | 3,479 min m/z=474,5 [M+H]⁺ | |
| A.2.257 | 75) | NO₂ | H | CH₃ | CH₃ | H | H | H | H | 181°C | |
| A.2.258 | 75) | NO₂ | H | CH₃ | CH₃ | H | H | H | H | 73°C | |
| A.2.259 | 75) | NO₂ | H | CH₃ | CH₃ | H | H | H | H | 108°C | |
| A.2.260 | 75) | NO₂ | H | CH₃ | CH₃ | H | H | H | H | 108°C | |
| A.2.261 | NO₂ | H | H | CH₃ | CH₃ | HO- | H | H | H | 2,548 min m/z=384,4 [M+H]⁺ | Diast. 2 |
| A.2.262 | NO₂ | H | H | CH₃ | CH₃ | HO- | H | H | H | 2,483 min m/z=384,4 [M+H]⁺ | Diast. 1 |
| A.2.263 | NO₂ | H | H | CH₃ | CH₃ | HO- | H | H | H | 2,548 min m/z=384,4 [M+H]⁺ | Diast. 3 |
| A.2.264 | NO₂ | H | H | CH₃ | CH₃ | HO- | H | H | H | 2,484 min m/z=384,4 [M+H]⁺ | Diast. 4 |
| A.2.265 | NO₂ | H | H | CH₃ | CH₃ | 76) | H | H | H | 2,687min m/z=462,0 [M+H]⁺ | Diast. 3 |
| A.2.266 | NO₂ | H | H | CH₃ | CH₃ | 76) | H | H | H | 2,700 min m/z=461,9 [M+H]⁺ | Diast. 4 |
| A.2.267 | NO₂ | H | H | CH₃ | CH₃ | 76) | H | H | H | 2,740 min m/z=462,1 [M+H]⁺ | Diast. 1 |
| A.2.268 | NO₂ | H | H | CH₃ | CH₃ | 76) | H | H | H | 2,754 min m/z=462,2 [M+H]⁺ | Diast. 2 |
| | | | | | | | | | | 2,475 min | |
| A.2.269 | NO₂ | H | H | H | CH₃ | 76) | H | H | H | m/z=447,9 2,593 | |
| | | | | | | | | | | min m/z=447,9 | |
| | | | | | | | | | | [M+H]⁺ | |
| A.2.270 | NO₂ | H | H | CH₃ | CH₃ | 77) | H | H | H | 109°C | Diast. 1 |
| A.2.271 | NO₂ | H | H | CH₃ | CH₃ | 77) | H | H | H | 109°C | Diast. 2 |
| A.2.272 | NO₂ | H | H | CH₃ | CH₃ | 77) | H | H | H | 144°C | Diast. 3 |
| A.2.273 | 78) | H | H | CH₃ | CH₃ | H | H | H | H | m/z=404,05 [M]⁺ | |
| A.2.274 | CH=CH₂ | H | H | CH₃ | CH₃ | H | H | H | H | 2,980 m/z=349,1 [M+H]⁺ | |
| A.2.275 | NO₂ | H | H | CH₃ | CH₃ | 80) | H | H | H | 145°C | |
| A.2.276 | Br | F | F | CH₃ | CH₃ | 76) | H | H | H | 3,030 m/z=531,0 [M]⁺ | |

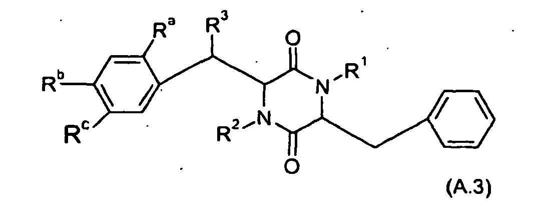

**Tabelle A.3**

| | | | | | | | RT | |
|---|---|---|---|---|---|---|---|---|
| Nr. | R^{a} | R^{b} | R^{c} | R¹ | R² | R³ | HPLC/MS oder | Diast. |
| | | | | | | | m.p. | |
| A.3.1 | NO₂ | CH₃O- | CH₃O- | CH₃ | CH₃ | H | 2,668 min m/z=428,0 [M+H]⁺ | Diast. 2 |
| A.3.2 | NO₂ | CH₃O- | CH₃O- | CH₃ | CH₃ | H | 2,571 min m/z=428,0 [M+H]⁺ | Diast. 1 |

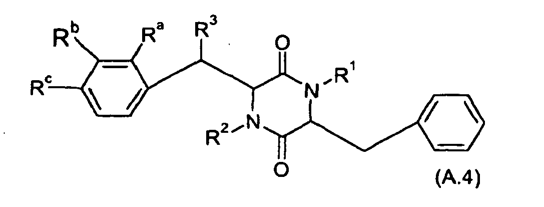

**Tabelle A.4**

| Nr. | R^{a} | R^{b} | R^{c} | R¹ | R² | R³ | RT HPLC/MS oder m.p. | Diast. |
|---|---|---|---|---|---|---|---|---|
| A.4.1 | Cl | CH₃ | H₃CS- | CH₃ | CH₃ | H | 3,354 min m/z=417,0 [M+H]⁺ | Diast. 1 |
| A.4.2 | Cl | CH₃ | H₃CS- | CH₃ | CH₃ | H | 3,397 min m/z=417,0 [M+H]⁺ | Diast. 2 |
| A.4.3 | Cl | 54) | H | CH₃ | CH₃ | H | 3,513 min m/z=399,2 [M+H]⁺ | Diast. 1 |
| A.4.4 | Cl | CF₃ | H | CH₃ | CH₃ | H | 3,281 min m/z=425,4 [M+H]⁺ | |
| A.4.5 | Cl | CH₃ | H₃CS- | CH₃ | CH₃ | H | 3,389 min m/z=417,2 [M+H]⁺ | Diast. 1 |
| A.4.6 | F | Cl | H | CH₃ | CH₃ | H | 2,927 min m/z=375,4 [M+H]⁺ | Diast. 1 |
| A.4.7 | F | F | H | CH₃ | CH₃ | H | 2,739 min m/z=359,4 [M+H]⁺ | Diast. 1 |
| A.4.8 | F | CF₃ | H | CH₃ | CH₃ | H | 3,096 min m/z=409,2 [M+H]⁺ | Diast. 1 |
| A.4.9 | Cl | Cl | H₃CO- | CH₃ | CH₃ | H | 3,088 min m/z=421,4 [M+H]⁺ | Diast. 1 |
| A.4.10 | F | F | F | CH₃ | CH₃ | H | 2,872 min m/z=377,2 [M+H]⁺ | Diast. 1 |
| A.4.11 | Cl | 54) | H | CH₃ | CH₃ | H | 3,557 min m/z=399,2 [M+H]⁺ | Diast. 2 |
| A.4.12 | Cl | CH₃ | H₃CS- | CH₃ | CH₃ | H | 3,428 min m/z=417,1 [M+H]⁺ | Diast. 2 Diast. 2 |
| A.4.13 | F | Cl | H | CH₃ | CH₃ | H | 3,052 min m/z=375,3 [M+H]* | Diast. 2 |
| A.4.14 | F | F | H | CH₃ | CH₃ | H | 2,965 min m/z=359,4 [M+H]⁺ | Diast. 2 |
| A.4.15 | F | CF₃ | H | CH₃ | CH₃ | H | 3,209 min m/z=409,2 [M+H]⁺ | Diast. 2 |
| A.4.16 | Cl | Cl | H₃CO- | CH₃ | CH₃ | H | 3,102 min m/z=421,1 [M+H]⁺ | Diast. 2 |
| A.4.17 | F | F | F | CH₃ | CH₃ | H | 2,999 min m/z=377,1 [M+H]⁺ | Diast. 2 |
| A.4.18 | Cl | Cl | H₃CO- | CH₃ | CH₃ | H | 3,138 min m/z=421,3 [M+H]⁺ | |
| A.4.19 | NO₂ | 13) | H | CH₃ | CH₃ | H | 2,764 min m/z=426,0 [M+H]⁺ | Diast. 2 |
| A.4.20 | NO₂ | 13) | H | CH₃ | CH₃ | H | 2,754 min m/z=426,0 [M+H]⁺ | Diast. 1 |

| | | | | | | | | |
|---|---|---|---|---|---|---|---|---|
| In analoger Weise wurden weitere Verbindungen der Formel I hergestellt, die in der nachfolgenden Tabelle A.5 aufgelistet sind. | | | | | | | | |

**Tabelle A.5**

| Nr. | Name | RT | Diast. |
|---|---|---|---|
| | | HPLC/MS oder | |
| | | m.p. | |
| A.5.1 | 1-Methyl-6-(2-nitro-benzyl)-3-thiophen-2-ylmethyl-piperazin-2,5-dion | 2,474 min m/z=360,3 [M+H]⁺ | Diast. 1 |
| A.5.2 | 1,4-Dimethyl-3-(2-nitro-benzyl)-6-thiophen-2-ylmethyl-piperazin-2,5-dion | 2,564 min m/z=373,9 [M+H]⁺ | Diast. 1 |
| A.5.3 | 1-Methyl-6-(2-nitro-benzyl)-3-thiophen-2-ylmethyl-piperazin-2,5-dion | 2,374 min m/z=360,3 [M+H]⁺ | Diast. 2 |
| A.5.4 | 1,4-Dimethyl-3-(2-nitro-benzyl)-6-thiophen-2-ylmethyl-piperazin-2,5-dion | 2,418 min m/z=373,9 [M+H]⁺ | Diast. 2 |
| A.5.5 | 1,4-Dimethyl-3,6-bis-(2-nitro-benzyl)-piperazin-2,5-dion | 2,666 min m/z=412,9 [M+H]⁺ | |
| A.5.6 | 3-Benzyl-1,4-dimethyl-6-(2-nitro-benzyl)-piperazin-2,5-dion | 2,868 min m/z=382,1 3,192 min m/z=382,1 [M+H]⁺ | |
| A.5.7 | 1-Methyl-6-(2-nitro-benzyl)-3-tetrazol-2-ylmethyl-piperazin-2,5-dion | 1,753 min m/z=346,1 [M+H]⁺ | Diast. 1 |
| A.5.8 | 1-Methyl-6-(2-nitro-benzyl)-3-tetrazol-2-ylmethyl-piperazin-2,5-dion | 1,829 min m/z=346,1 [M+H]⁺ | Diast. 2 |
| A.5.9 | 1-Methyl-6-(2-nitro-benzyl)-3-[1,2,4]triazol-1-ylmethyl-piperazin-2,5-dion | 1,669 min m/z=345,1 [M+H]⁺ | Diast. 1 |
| A.5.10 | N-{2-[2-(5-Benzyl-1,4-dimethyl-3,6-dioxopiperazin-2-yl)-acetyl]-3-nitro-phenyl}-formamid | 2,504 min m/z=461,1 [M+Na]⁺ | |
| A.5.11 | 1-Methyl-6-(2-nitro-benzyl)-3-[1,2,4]triazol-1-ylmethyl-piperazin-2,5-dion | 1,671 min m/z=345,1 [M+H]⁺ | Diast. 2 |
| A.5.12 | 3-Benzyl-1,3-dimethyl-6-(2=nitro-benzyl)-piperazin-2,5-dion | 2,652 min m/z=368,0 [M+H]⁺ | Diast. 1 |
| A.5.13 | 3-Benzyl-1,3-dimethyl-6-(2-nitro-benzyl)-piperazin-2,5-dion | 2,696 min m/z=368,0 [M+H]⁺ | Diast. 2 |
| A.5.14 | 1-Methyl-6-(2-nitro-benzyl)-3-pyridin-3-ylmethyl-piperazin-2,5-dion | 1,555 min m/z=355,0 [M+H]⁺ | Diast. 1 |
| A.5.15 | 1-Methyl-6-(2-nitro-benzyl)-3-pyridin-3-ylmethyl-piperazin-2,5-dion | 1,580 min m/z=355,0 [M+H]⁺ | Diast. 2 |
| A.5.16 | Methansulfonsäure-(5-benzyl-1,4-dimethyl-3,6-dioxopiperazin-2-yl)-(2-brompyridin-3-yl)-methyl ester | 2,530 min m/z = 498 [M+H]⁺ | |
| A.5.17 | Methansulfonsäure-[1,4-dimethyl-3,6-dioxo-5-(thiophen-2-ylmethl)piperazin-2-yl]-(2-nitrophenyl)-methylester | 2,575 min m/z = 468,1 [M+H]⁺ | |

Die Produkte wurden durch HPLC/MS (High Performance Liquid Chromatographie kombiniert mit Massen-Spektrometrie), durch NMR oder durch ihren Schmelzpunkt charakterisiert.
HPLC-Säule: RP-18 Säule (Chromolith Speed ROD von Merck KgaA, Deutschland) Eluent: Acetonitril + 0.1% Trifuoroessigsäure (TFA)/ Wasser + 0.1% TFA in einem Gradient von 5:95 bis 95:5 in 5 Minuten bei 40°C.
MS: Quadrupol Elektrospray Ionisation, 80 V (positiv Modus)

Die Verbindungen I und deren landwirtschaftlich brauchbaren Salze eignen sich - sowohl als Isomerengemische als auch in Form der reinen isomeren - als Herbizide. Sie eignen sich als solche oder als entsprechend formuliertes Mittel. Die herbiziden Mittel, die die Verbindung I oder Ia enthalten, bekämpfen Pflanzenwuchs auf Nichtkulturflächen sehr gut, besonders bei hohen Aufwandmengen. In Kulturen wie Weizen, Reis, Mais, Soja und Baumwolle wirken sie gegen Unkräuter und Schadgräser, ohne die Kulturpflanzen nennenswert zu schädigen. Dieser Effekt tritt vor allem bei niedrigen Aufwandmengen auf.

In Abhängigkeit von der jeweiligen Applikationsmethode können die Verbindungen I oder Ia bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:
Allium cepa, Ananas comosus, Arachis hypogaea, Asparagus officinalis, Avena sativa, Beta vulgaris spec. altissima, Beta vulgaris spec. rapa, Brassica napus var. napus, Brassica napus var. napobrassica, Brassica rapa var. silvestris, Brassica oleracea, Brassica nigra, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa, Phaseolus lunatus, Phaseolus vulgaris, Picea abies, Pinus spec., Pistacia vera, Pisum sativum, Prunus avium, Prunus persica, Pyrus communis, Prunus armeniaca, Prunus cerasus, Prunus dulcis und prunus domestica, Ribes sylvestre, Ricinus communis, Saccharum officinarum, Secale cereale, Sinapis alba, Solanum tuberosum, Sorghum bicolor (s. vulgare), Theobroma cacao, Trifolium pratense, Triticale, Triticum aestivum, Triticum durum, Vicia faba, Vitis vinifera, Zea mays.

Darüber hinaus können die Verbindungen I auch in Kulturen, die durch Züchtung einschließlich gentechnischer Methoden gegen die Wirkung von Herbiziden tolerant sind, verwandt werden.

Darüber hinaus können die Verbindungen I auch in Kulturen, die durch Züchtung einschließlich gentechnischer Methoden gegen Insekten oder Pilzbefall tolerant sind, verwendet werden.

Des Weiteren wurde gefunden, dass die Verbindungen der Formel I auch zur Defoliation und/oder Desikkation von Pflanzenteilen geeignet ist, wofür Kulturpflanzen wie Baumwolle, Kartoffel, Raps, Sonnenblume, Sojabohne oder Ackerbohnen, insbesondere Baumwolle, in Betracht kommen. Diesbezüglich wurden Mittel zur Desikkation und /oder Defoliation von Pflanzen, Verfahren zur Herstellung dieser Mittel und Verfahren zur Desikkation und/oder Defoliation von Pflanzen mit der Verbindungen der Formel I gefunden.

Als Desikkantien eignet sich die Verbindungen der Formel I insbesondere zur Austrocknung der oberirdischen Teile von Kulturpflanzen wie Kartoffel, Raps, Sonnenblume und Sojabohne aber auch Getreide. Damit wird ein vollständig mechanisches Beernten dieser wichtigen Kulturpflanzen ermöglicht.

Von wirtschaftlichem Interesse ist ferner die Ernteerleichterung, die durch das zeitlich konzentrierte Abfallen oder Vermindern der Haftfestigkeit am Baum bei Zitrusfrüchten, Oliven oder bei anderen Arten und Sorten von Kern-, Stein- und Schalenobst ermöglicht wird. Derselbe Mechanismus, d.h., die Förderung der Ausbildung von Trenngewebe zwischen Frucht- oder Blatt- und Sprossteil der Pflanzen ist auch für ein gut kontrollierbares Entblättern von Nutzpflanzen, insbesondere Baumwolle, wesentlich.

Außerdem führt die Verkürzung des Zeitintervalls, in dem die einzelnen Baumwollpflanzen reif werden, zu einer erhöhten Qualität der Faser nach der Ernte.

Die Verbindungen I bzw. die sie enthaltenden herbiziden Mittel können beispielsweise in Form von direkt versprühbaren wässrigen Lösungen, Pulvern, Suspensionen, auch hochprozentigen wässrigen, öligen oder sonstigen Suspensionen oder Dispersionen, Emulsionen, Öldispersionen, Pasten, Stäubemittel, Streumitteln oder Granulaten durch Versprühen, Vernebeln, Verstäuben, Verstreuen, Gießen oder Behandlung des Saatgutes bzw. Mischen mit dem Saatgut angewendet werden. Die Anwendungsformen richten sich nach den Verwendungszwecken; sie sollten in jedem Fall möglichst die feinste Verteilung der erfindungsgemäßen Wirkstoffe gewährleisten.

Die herbiziden Mittel enthalten eine herbizid wirksame Menge mindestens einer Verbindung der Formel I oder eines landwirtschaftlich brauchbaren Salzes von I und für die Formulierung von Pflanzenschutzmitteln übliche Hilfsstoffe.

Beispiele für die Formulierung von Pflanzenschutzmitteln übliche Hilfsmittel sind inerte Hilfsstoffe, feste Trägerstoffe, oberflächenaktive Stoffe (wie Dispergiermittel Schutzkolloide, Emulgatoren, Netzmittel und Haftmittel), organische und anorganische Verdicker, Bakterizide, Frostschutzmittel, Entschäumer ggf. Farbstoffe und für Saatgutformulierungen Kleber.

Beispiele für Verdicker (d.h. Verbindungen, die der Formulierung ein modifiziertes Fließverhalten verleihen, d.h. hohe Viskosität im Ruhezustand und niedrige Viskosität im bewegten Zustand) sind Polysaccharide wie Xanthan Gum (Kelzan® der Fa. Kelco), Rhodopol® 23 (Rhone Poulenc) oder Veegum® (Firma R.T. Vanderbilt) sowie organische und anorganische Schichtmineralienwie Attaclay® (Firma Engelhardt).

Beispiele für Antischaummittel sind Silikonemulsionen (wie z.Bsp. Silikon® SRE, Firma Wacker oder Rhodorsil® der Firma Rhodia ), langkettige Alkohole, Fettsäuren, Salze von Fettsäuren, fluororganische Verbindungen und deren Gemische.

Bakterizide können zur Stabilisierung der wäßrigen Herbizid-Formulierung zugesetzt werden. Beispiele für Bakterizide sind Bakterizide basierend auf Diclorophen und Benzylalkoholhemiformal (Proxel® der Fa. ICI oder Acticide® RS der Fa. Thor Chemie und Kathon® MK der Firma Rohm & Haas) sowie Isothiazolinonderivaten wie Alkylisothiazolinonen und Benzisothiazolinonen (Acticide MBS der Fa. Thor Chemie)

Beispiele für Frostschutzmittel sind Ethylenglycol, Propylenglycol, Harnstoff oder Glycerin.

Beispiele für Farbmittel sind sowohl in Wasser wenig lösliche Pigmente als auch in Wasser lösliche Farbstoffe. Als Beispiele genannt seien die unter den Bezeichnungen Rhodamin B, C.I. Pigment Red 112 und C.I. Solvent Red 1 bekannten Farbstoffe, sowie pigment blue 15:4, pigment blue 15:3, pigment blue 15:2, pigment blue 15:1, pigment blue 80, pigment yellow 1, pigment yellow 13, pigment red 112, pigment red 48:2, pigment red 48:1, pigment red 57:1, pigment red 53:1, pigment orange 43, pigment orange 34, pigment orange 5, pigment green 36, pigment green 7, pigment white 6, pigment brown 25, basic violet 10, basic violet 49, acid red 51, acid red 52, acid red 14, acid blue 9, acid yellow 23, basic red 10, basic red 108.

Beispiele für Kleber sind Polyvinylpyrrolidon, Polyvinylacetat, Polyvinylalkohol und Tylose.

Als inerte Zusatzstoffe kommen beispielsweise in Betracht:
Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Paraffin, Tetrahydronaphthalin, alkylierte Naphthaline oder deren Derivate, alkylierte Benzole oder deren Derivate, Alkohole wie Methanol, Ethanol, Propanol, Butanol, Cyclohexanol, Ketone wie Cyclohexanon oder stark polare Lösungsmittel, z. B. Amine wie N-Methylpyrrolidon oder Wasser.

Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.
Als oberflächenaktive Stoffe (Adjuvantien, Netz-, Haft-, Dispergier- sowie Emulgiermittel) kommen die Alkali-, Erdalkali-, Ammoniumsalze von aromatischen Sulfonsäuren, z.B. Ligninsulfonsäuren (z.B. Borrespers-Typen, Borregaard), Phenolsulfonsäuren, Naphthalinsulfonsäuren (Morwet-Typen, Akzo Nobel) und Dibutylnaphthalinsulfonsäure (Nekal-Typen, BASF AG), sowie von Fettsäuren, Alkyl- und Alkylarylsulfonaten, Alkyl-, Laurylether- und Fettalkoholsulfaten, sowie Salze sulfatierter Hexa-, Hepta- und Octadecanole sowie von Fettalkoholglykolethem, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylenalkylether, Laurylalkoholpolyglykoletheracetat, Sorbitester, Lignin-Sulfitablaugen sowie Proteine, denaturierte Proteine, Polysaccharide (z.B. Methylcellulose), hydrophob modifizierte Stärken, Polyvinylalkohol (Mowiol typen Clariant), Polycarboxylate (BASF AG, Sokalan-Typen), Polyalkoxylate, Polyvinylamin (BASF AG, Lupamin-Typen), Polyethylenimin (BASF AG, Lupasol-Typen), Polyvinylpyrrolidon und deren Copolymere in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden.

Wässrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die Verbindungen der Formel I oder Ia als solche oder in einem Öl oder Lösungsmittel gelöst; mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Die Konzentrationen der Verbindungen der Formel I in den anwendungsfertigen Zubereitungen können in weiten Bereichen variiert werden. Die Formulierungen enthalten im Allgemeinen 0,001 bis 98 Gew.-%, vorzugsweise 0,01 bis 95 Gew.-%, mindestens eines Wirkstoffs. Die Wirkstoffe werden dabei in einer Reinheit von 90% bis 100%, vorzugsweise 95% bis 100% (nach NMR-Spektrum) eingesetzt.

Die erfindungsgemäßen Verbindungen I können beispielsweise wie folgt formuliert werden:

### 1. Produkte zur Verdünnung in Wasser

### A Wasserlösliche Konzentrate

10 Gew.-Teile Wirkstoff werden mit 90 Gew.-Teilen Wasser oder einem wasserlöslichen Lösungsmittel gelöst. Alternativ werden Netzmittel oder andere Hilfsmittel zugefügt. Bei der Verdünnung in Wasser löst sich der Wirkstoff. Man erhält auf diese Weise eine Formulierung mit 10 Gew.-% Wirkstoffgehalt.

### B Dispergierbare Konzentrate

20 Gew.-Teile Wirkstoff werden in 70 Gew.-Teilen Cyclohexanon unter Zusatz von 10 Gew.-Teilen eines Dispergiermittels z.B. Polyvinylpyrrolidon gelöst. Bei Verdünnung in Wasser ergibt sich eine Dispersion. Der Wirkstoffgehalt beträgt 20 Gew.-%

### C Emulgierbare Konzentrate

15 Gew.-Teile Wirkstoff werden in 75 Gew.-Teilen eines organisches Lösungsmittel (z.B. Alkylaromaten)-unter Zusatz von Ca-Dodecylbenzolsulfonat und Ricinusölethoxylat (jeweils 5 Gew.-Teile) gelöst. Bei der Verdünnung in Wasser ergibt sich eine Emulsion. Die Formulierung hat 15 Gew.-% Wirkstoffgehalt.

### D Emulsionen

25 Gew.-Teile Wirkstoff werden in 35 Gew.-Teilen eines organisches Lösungsmittel (z.B. Alkylaromaten) unter Zusatz von Ca-Dodecylbenzolsulfonat und Ricinusölethoxylat (jeweils 5 Gew.-Teile) gelöst. Diese Mischung wird mittels einer Emulgiermaschine (z.B. Ultraturax) in 30 Gew.Teile Wasser gegeben und zu einer homogenen Emulsion gebracht. Bei der Verdünnung in Wasser ergibt sich eine Emulsion. Die Formulierung hat einen Wirkstoffgehalt von 25 Gew.-%.

### E Suspensionen

20 Gew.-Teile Wirkstoff werden unter Zusatz von 10 Gew.-Teilen Dispergier- und Netzmitteln und 70 Gew.-Teilen Wasser oder einem organischen Lösungsmittel in einer Rührwerkskugelmühle zu einer feinen Wirkstoffsuspension zerkleinert. Bei der Verdünnung in Wasser ergibt sich eine stabile Suspension des Wirkstoffs. Der Wirkstoffgehalt in der Formulierung beträgt 20 Gew.-%.

### F Wasserdispergierbare und wasserlösliche Granulate

50 Gew.-Teile Wirkstoff werden unter Zusatz von 50 Gew-Teilen Dispergier- und Netzmitteln fein gemahlen und mittels technischer Geräte (z.B. Extrusion, Sprühturm, Wirbelschicht) als wasserdispergierbare oder wasserlösliche Granulate hergestellt. Bei der Verdünnung in Wasser ergibt sich eine stabile Dispersion oder Lösung des Wirkstoffs. Die Formulierung hat einen Wirkstoffgehalt von 50 Gew.-%.

### G Wasserdispergierbare und wasserlösliche Pulver

75 Gew.-Teile Wirkstoff werden unter Zusatz von 25 Gew.-Teilen Dispergier- und Netzmitteln sowie Kieselsäuregel in einer Rotor-Strator Mühle vermahlen. Bei der Verdünnung in Wasser ergibt sich eine stabile Dispersion oder Lösung des Wirkstoffs. Der Wirkstoffgehalt der Formulierung beträgt 75 Gew.-%.

### H Gelformulierungen

In einer Kugelmühle werden 20 Gew.-Teile Wirkstoff, 10 Gew.-Teile Dispergiermittel, 1 Gew.-Teil Geliermittel und 70 Gew.-Teile Wasser oder eines organischen Lösungsmittels zu einer feinen Suspension vermahlen. Bei der Verdünnung mit Wasser ergibt sich eine stabile Suspension mit 20 Ges.-% Wirkstoffgehalt.

### 2. Produkte für die Direktapplikation

### I Stäube

5 Gew.-Teile Wirkstoff werden fein gemahlen und mit 95 Gew.-Teilen feinteiligem Kaolin innig vermischt. Man erhält dadurch ein Stäubemittel mit 5 Gew.-% Wirkstoffgehalt.

### J Granulate (GR, FG, GG, MG)

0,5 Gew-Teile Wirkstoff werden fein gemahlen und mit 99,5 Gewichtsteilen Trägerstoffe verbunden. Gängige Verfahren sind dabei die Extrusion, die Sprühtrocknung oder die Wirbelschicht. Man erhält dadurch ein Granulat für die Direktapplikation mit 0,5 Gew.-% Wirkstoffgehalt.

### K ULV- Lösungen (UL)

10 Gew.-Teile Wirkstoff werden in 90 Gew.-Teilen eines organischen Lösungsmittel z.B. Xylol gelöst. Dadurch erhält man ein Produkt für die Direktapplikation mit 10 Gew.-% Wirkstoffgehalt.

Im Folgenden sind konkrete Formulierungen angegeben:
- i: 20 Gewichtsteile der Verbindung der Formel I werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew. % des Wirkstoffs enthält.
- II: 20 Gewichtsteile der Verbindung der Formel I werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.-% des Wirkstoffs enthält.
- III: 20 Gewichtsteile der Verbindung der Formel I werden in einer Mischung gelöst, die aus 25 Gewichtsteilen Cyclohexanon, 65 Gewichststeilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilern der Lösung in 100 000 Gewichtsteilen Wasser erhält man eine wässrige Dispersion, die 0,02 Gew.-% des Wirkstoffs enthält.
- IV: 20 Gewichtsteile der Verbindung der Formel I werden mit 3 Gewichtsteilen des Natriumsalzes der Diisobutylnaphthalinsulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.-% des Wirkstoffs enthält.
- V: 3 Gewichtsteile der Verbindung der Formel I werden mit 97 Gewichtsteilen feinteiligem Kaolin vermischt. Man erhält auf diese Weise ein Stäubemittel, das 3 Gew.-% des Wirkstoffs enthält.
- VI: 20 Gewichtsteile der Verbindung der Formel I werden mit 2 Gewichtsteilen Calciumsalz der Dodecylbenzolsulfonsäure, 8 Gewichtsteilen Fettalkoholpolyglykolether, 2 Gewichtsteilen Natriumsalz eines Phenol-Harnstoff-Formaldehyd-Kondesates und 68 Gewichtsteilen eines paraffinischen Mineralöls innig vermischt. Man erhält eine stabile ölige Dispersion.
- VII: 1 Gewichtsteil der Verbindung der Formel I wird in einer Mischung gelöst, die aus 70 Gewichtsteilen Cyclohexanon, 20 Gewichtsteilen ethoxyliertem Isooctylphenol und 10 Gewichtsteilen ethoxyliertem Rizinusöl besteht. Man erhält ein stabiles Emulsionskonzentrat.
- VIII: 1 Gewichtsteil der Verbindung der Formel I wird in einer Mischung gelöst, die aus 80 Gewichtsteilen Cyclohexanon und 20 Gewichtsteilen Wettol ® EM 31 (nicht ionischer Emulgator auf der Basis von ethoxyliertem Ricinusöl). Man erhält ein stabiles Emulsionskonzentrat.

Die Applikation der Verbindungen I oder der sie enthaltenden herbiziden Mittel kann im Vorauflauf-, im Nachauflaufverfahren oder zusammen mit dem Saatgut einer Kulturpflanze erfolgen. Es besteht auch die Möglichkeit, die herbiziden Mittel bzw. Wirkstoffe dadurch zu applizieren, dass mit den herbiziden Mitteln bzw. Wirkstoffen vorbehandeltes Saatgut einer Kulturpflanze ausgebracht wird. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

In einer weiteren Ausführungsform kann die Applikation der Verbindungen der Formel I bzw. der herbiziden Mittel durch Behandlung von Saatgut erfolgen.

Die Behandlung von Saatgut umfasst im Wesentlichen alle dem Fachmann geläufigen Techniken (seed dressing, seed coating, seed dusting, seed soaking, seed film coating, seed multilayer coating, seed encrusting, seed dripping, und seed pelleting" ) basierend auf den erfindungsgemäßen Verbindungen der Formel I bzw. daraus hergestellten Mitteln. Hierbei können die herbiziden Mittel verdünnt oder unverdünnt aufgetragen werden.

Der Begriff Saatgut umfasst Saatgut aller Arten, wie z.B. Körner, Samen, Früchte, Knollen, Stecklinge und ähnliche Formen. Bevorzugt beschreibt der Begriff Saatgut hier Körner und Samen.

Als Saatgut kann Saatgut der oben erwähnten Nutzpflanzen aber auch das Saatgut transgener oder durch herkömmliche Züchtungsmethoden erhaltener Pflanzen eingesetzt werden.

Die Aufwandmengen an Wirkstoff betragen je nach Bekämpfungsziel, Jahreszeit, Zielpflanzen und Wachstumsstadium 0.001 bis 3.0, vorzugsweise 0.01 bis 1.0 kg/ha aktive Substanz (a. S.). Zur Saatgutbehandlung werden die Verbindungen I üblicherweise in Mengen von 0,001 bis 10kg pro 100kg Saatgut eingesetzt.

Zur Verbreiterung des Wirkungsspektrums und zur Erzielung synergistischer Effekte können die Verbindungen der Formel I mit zahlreichen Vertretern anderer herbizider oder wachstumsregulierender Wirkstoffgruppen gemischt und gemeinsam ausgebracht werden. Beispielsweise kommen als Mischungspartner 1,2,4-Thiadiazole, 1,3,4-Thiadiazole, Amide, Aminophosphorsäure und deren Derivate, Aminotriazole, Anilide, (Het)-Aryloxyalkansäure und deren Derivate, Benzoesäure und deren Derivate, Benzothiadiazinone, 2-Aroyl-1,3-cyclohexandione, 2-Hetaroyl-1,3-cyclohexandione, Hetaryl-Aryl-Ketone, Benzylisoxazolidinone, Meta-CF₃-phenylderivate, Carbamate, Chinolincarbonsäure und deren Derivate, Chloracetanilide, Cyclohexenonoximether -Derivate, Diazine, Dichlorpropionsäure und deren Derivate, Dihydrobenzofurane, Dihydrofuran-3-one, Dinitroaniline, Dinitrophenole, Diphenylether, Dipyridyle, Halogencarbonsäuren und deren Derivate, Harnstoffe, 3-Phenyluracile, Imidazole, Imidazolinone, N-Phenyl-3,4,5,6-tetrahydrophthalimide, Oxadiazole, Oxirane, Phenole, Aryloxy- oder Heteroaryloxyphenoxypropionsäureester, Phenylessigsäure und deren Derivate, Phenylpropionsäure und deren Derivate, Pyrazole, Phenylpyrazole, Pyridazine, Pyridincarbonsäure und deren Derivate, Pyrimidylether, Sulfonamide, Sulfonylharnstoffe, Triazine, Triazinone, Triazolinone, Triazolcarboxamide, Uracile, Phenylpyrazoline und Isoxazoline und deren Derivate in Betracht.

Außerdem kann es von Nutzen sein, die Verbindungen I allein oder in Kombination mit anderen Herbiziden oder auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch weitere Additive wie nichtphytotoxische Öle und Ölkonzentrate zugesetzt werden.

### Teil B

### Anwendungsbeispiele

Die herbizide Wirkung der Verbindungen der Formel I ließ sich durch Gewächshausversuche zeigen:
Als Kulturgefäße dienten Plastiktöpfe mit lehmigem Sand mit etwa 3,0% Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren: Diese Abdeckung bewirkte ein gleichmäßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer Wuchshöhe von 3 bis 15 cm angezogen und dann mit den in Wasser suspendierten oder emulgierten Wirkstoffen behandelt. Die Testpflanzen wurden dafür entweder direkt gesät und in den gleichen Gefäßen aufgezogen oder sie wurden erst als Keimpflanzen getrennt angezogen und einige Tage vor der Behandlung in die Versuchsgefäße verpflanzt.
Die Pflanzen wurden artenspezifisch bei Temperaturen von 10 - 25°C bzw. 20 - 35°C. gehalten. Die Versuchsperiode erstreckte sich über 2 bis 4 Wochen. Während dieser

Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf. Eine gute herbizide Aktivität ist bei Werten von wenigstens 70 und eine sehr gute herbizide Aktivität ist bei Werten von wenigstens 85 gegeben.

Die in den Gewächshausversuchen verwendeten Pflanzen setzten sich aus folgenden Arten zusammen:

| Lateinischer Name | Deutscher Name | Englischer Name |
|---|---|---|
| Amaranthus retroflexus (AMARE) | Fuchsschwanz | pig weed |
| Chenopodium album (CHEAL) | Weißer Gänsefuß | lambsquaters |
| Setaria viridis (SETVI) | Grüne Borstenhirse | green foxtail |
| Setaria faberi (SETFA) | Fabers Borstendhirse | giant foxtail |
| Echinochloa crus galli (ECHCG) | Hühnerhirse | barnyard grass |
| Alopecurus myosuroides (ALOMY) | Ackerfuchsschwanzgras | black grass |
| Avena fatua (AVEFA) | Flughafer | wild oat |
| Lolium multiflorum (LOLMU) | Italienisches Raygras | raygrass |
| Apera spica-venti (APESV) | Gemeiner Windhalm | windgrass |

Die Verbindungen A.1.10, A.1.11, A. 2.81, A.2.97, A.2.135, A.2.136, A2.222, A.2.223, A.2.224, A.2.226, und A.2.267 zeigen eine sehr gute herbizide Wirkung im Nachauflaufverfahren.

Bei einer Aufwandmenge von 1 kg/ha wiesen die Verbindungen A.1.10, A.1.11, A.2.135, A.2.222, A.2.223 und A.2.224 im Nachauflauf gegen CHEAL eine sehr gute herbiziden Wirkung auf.

Bei einer Aufwandmenge von 1 kg/ha wiesen die Verbindungen A.1.10. A.1.11, A.2.135, A.2.222, A.2.223, A.2.224, A.2.226 und A.2.267 im Nachauflauf gegen AMARE eine sehr gute herbizide Wirkung auf.

Bei einer Aufwandmenge von 1 kg/ha wiesen die Verbindungen A.1.10, A.1.11, A.2.97, A.2.136, A.2.222, A.2.223 und A.2.226 im Nachauflauf gegen SETVI eine sehr gute herbizide Wirkung auf.

Bei einer Aufwandmenge von 1 kg/ha wiesen die Verbindungen A.2.97, A.2.136 und A.2.224 im Nachauflauf gegen LOLMU eine sehr gute herbizide Wirkung auf.

Bei einer Aufwandmenge von 3 kg/ha wies die Verbindung A.2.81 im Nachauflauf gegen ECHCG eine sehr gute herbizide Wirkung auf. Bei einer Aufwandmenge von 1 kg/ha wiesen die Verbindungen A.2.226 und A.2.267 im Nachauflauf gegen ECHCG eine sehr gute herbizide Wirkung auf.

Bei einer Aufwandmenge von 3 kg/ha wies die Verbindung A.2.105 im Nachauflauf gegen ALOMY und AVEFA eine sehr gute herbizide Wirkung auf.

Bei einer Aufwandmenge von 3 kg/ha wiesen die Verbindungen A.2.64, A.2.65, A.2.133, A.2.135, A.2.251, A.2.255, A.2.265, A.2.267, A.2.273, A.2.274 und A.2.275 im Nachauflauf gegen SETFA eine gute bis sehr gute herbizide Wirkung auf. Bei einer Aufwandmenge von 1 kg/ha wies die Verbindung A.2.224 im Nachauflauf gegen SETFA eine gute bis sehr gute herbizide Wirkung auf.

Bei einer Aufwandmenge von 3 kg/ha wiesen die Verbindungen A.2.64, A.2.105, A.2.133, A.2.135, A.2.265, A.2.267, A.2.273, A.2.274 und A.2.275 im Vorauflauf gegen ECHCG eine gute bis sehr gute herbizide Wirkung auf.

Bei einer Aufwandmenge von 3 kg/ha wiesen die Verbindungen A.1.13, A.5.16, A.2.135, A.2.265, A.2.267, A.2.271, A.2.273 und A.2.276 im Vorauflauf gegen SETIT eine gute bis sehr gute herbizide Wirkung auf.

Bei einer Aufwandmenge von 1 kg/ha wiesen die Verbindungen A.2.97, A.2.136, A.2.224 und A.2.267 im Vorauflauf gegen APESV eine sehr gute herbizide Wirkung auf.

Bei einer Aufwandmenge von 3kg/ha wies die Verbindung A.2.81 im Vorauflauf gegen SETFA eine sehr gute herbizide Wirkung auf.

Bei einer Aufwandmenge von 0,5 kg/ha wies die Verbindung A.5.17 im Vorauflauf gegen APESV eine gute und gegen AMARE eine sehr gute herbizide Wirkung auf.

## Patentansprüche

1. Verwendung von Piperazinverbindungen der Formel I 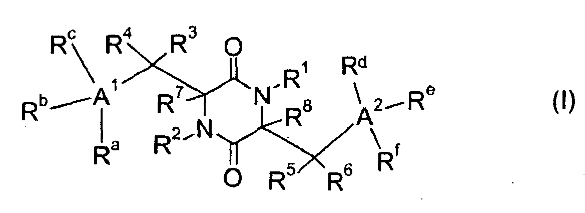 oder der landwirtschaftlich brauchbaren Salze von Piperazinverbindungen der Formel I als Herbizide, wobei in Formel I die Variablen die folgenden Bedeutungen haben:
R¹ und R² unabhängig voneinander
Cyano, C₁-C₆-Alkyl, C₃-C₆-Cycloalkyl, C₃-C₆-Alkenyl, C₃-C₆-Cycloalkenyl, C₃-C₆-Alkinyl, C₃-C₆-Cycloalkinyl, Phenyl, Phenyl-(C₁-C₆)-Alkyl, Heterocyclyl, Heterocyclyl-(C₁-C₆)-Alkyl; Phenyl-[C₁-C₆-alkoxycarbonyl]-(C₁-C₆)-alkyl oder Phenylheterocyclyl-(C₁-C₆)-alkyl; oder
COR²¹, wobei
R²¹ Wasserstoff, C₁-C₆-Alkyl, C₃-C₆-Cycloalkyl, C₂-C₆-Alkenyl, C₃-C₆-Cycloalkenyl, C₂-C₆-Alkinyl, C₃-C₆-Cycloalkinyl, Hydroxy, C₁-C₆-Alkoxy, C₃-C₆-Alkeriyloxy, C₃-C₆-Alkinyloxy, Amino, C₁-C₆-Alkylamino, [Di-(C₁-C₆)-alkyl]amino, C₃-C₆-Alkenylamino, C₃-C₆-Alkinylamino, C₁-C₆-Alkylsulfonylamino, N-(C₂-C₆-Alkenyl)-N-(C₁-C₆-alkyl)-amino, N-(C₂-C₆-Alkinyl)-N-(C₁-C₆-alkyl)-amino, N-(C₁-C₆-Alkoxy)-N-(C₁-C₆-alkyl)-amino, N-(C₂-C₆-Alkenyl)-N-(C₁-C₆-alkoxy)-amino, N-(C₂-C₆-Alkinyl)-N-(C₁-C₆-alkoxy)-amino, Phenyl, Phenylamino, Phenoxy, Naphthyl oder Heterocyclyl bedeutet; oder
NR²²R²³, wobei
R²² und R²³ unabhängig voneinander Wasserstoff, C₁-C₆-Alkyl, C₃-C₆-Cycloalkyl, C₃-C₆-Alkenyl, C₃-C₆-Cycloalkenyl, C₃-C₆-Alkinyl, C₃-C₆-Cycloalkinyl oder C₁-C₆-Alkylcarbonyl bedeuten; oder
OR²⁴, wobei
R²⁴ C₁-C₆-Alkyl, C₃-C₆-Cycloalkyl, C₃-C₆-Alkenyl, C₃-C₆-Cycloalkenyl, C₃-C₆-Alkinyl, C₃-C₆-Cycloalkinyl, Phenyl oder Phenyl-(C₁-C₆)-Alkyl bedeutet; oder
SO₂R²⁵, wobei
R²⁵ C₁-C₆-Alkyl oder Phenyl bedeutet;
wobei die genannten aliphatischen, cyclischen oder aromatischen Teile der Substituenten von R¹ und R² partiell oder vollständig halogeniert sein können und/oder eine bis drei der folgenden Gruppen tragen können: Cyano, Hydroxy, C₁-C₄-Alkyl, C₁-C₄-Halogenalkyl, C₃-C₆-Cycloalkyl, C₁-C₄-Alkoxy, C₁-C₄-Alkylthio, [Di-(C₁-C₄)-alkyl]-amino, C₁-C₄-Alkylcarbonyl, Hydroxycarbonyl, C₁-C₄-Alkoxycarbonyl, Aminocarbonyl, C₁-C₄-Alkylaminocarbonyl, [Di-(C₁-C₄)-alkyl]aminocarbonyl oder C₁-C₄-Alkylcarbonyloxy;
und wobei R¹ zusätzlich Wasserstoff bedeuten kann;
R³ ein Rest R²⁶, OR²⁷, SR²⁸, NR²⁹R³⁰ oder N(OR³¹)R³², wobei
R²⁶, R²⁷, R²⁸, R²⁹ und R³² unabhängig voneinander Wasserstoff, C₁-C₆-Alkyl, C₃-C₆-Cycloalkyl, C₃-C₆-Alkenyl, C₃-C₆-Alkinyl, C₃-C₆-Halogenalkinyl, Formyl, C₁-C₆-Alkylcarbonyl, C₃-C₆-Cycloalkylcarbonyl, C₂-C₆-Alkenylcarbonyl, C₂-C₆-Alkinylcarbonyl, C₁-C₆-Alkoxy-(C₁-C₆)-alkyl, C₁-C₆-Alkoxycarbonyl, C₂-C₆-Alkenyloxycarbonyl, C₃-C₆-Alkinyloxycarbonyl, C₁-C₆-Alkylaminocarbonyl, C₃-C₆-Alkenylaminocarbonyl, C₃-C₆-Alkinylaminocarbonyl, C₁-C₆-Alkylsulfonylaminocarbonyl, C₁-C₆-alkylaminocarbonyl, [Di-(C₁-C₆)-alkylamino]carbonyl, N-(C₃-C₆-Alkenyl)-N-(C₁-C₆-alkyl)-aminocarbonyl, N-(C₃-C₆-Alkinyl)-N-(C₁-C₆-alkyl)-aminocarbonyl, N-(C₁-C₆-Alkoxy)-N-(C₁-C₆-alkyl)-amino-carbonyl, N-(C₃-C₆-Alkenyl)-N-(C₁-C₆-alkoxy)-aminocarbonyl, N-(C₃-C₆-Alkinyl)-N-(C₁-C₆-alkoxy)-aminocarbonyl, Di-(C₁-C₆-alkyl)-aminothiocarbonyl, C₁-C₆-Alkylcarbonyl-C₁-C₆-alkyl, C₁-C₆-Alkoxyimino-C₁-C₆-alkyl, N-(C₁-C₆-Alkylamino)-imino-C₁-C₆-alkyl, N-(Di-C₁-C₆-alkylamino)-imino-C₁-C₆-alkyl oder [Tri-(C₁-C₄)-alkyl]silyl, wobei die genannten aliphatischen oder isocyclischen Teile der Substituenten partiell oder vollständig halogeniert sein können und/oder eine bis drei der folgenden Gruppen tragen können: Cyano, Hydroxy, C₁-C₄-Alkyl, C₁-C₄-Halogenalkyl, C₃-C₆-Cycloalkyl, C₁-C₄-Alkoxy, C₁-C₄-Alkylthio, [Di-(C₁-C₄)-alkyl]-amino, C₁-C₄-Alkylcarbonyl, Hydroxycarbonyl, C₁-C₄-Alkoxycarbonyl, Aminocarbonyl, C₁-C₄-Alkylaminocarbonyl, [Di-(C₁-C₄)-alkyl]aminocarbonyl oder C₁-C₄-Alkylcarbonyloxy; oder
Phenyl, Phenyl-C₁-C₆-alkyl, Phenylcarbonyl, Phenylcarbonyl-C₁-C₆-alkyl, Phenoxycarbonyl, Phenylaminocarbonyl, Phenylsulfonylaminocarbonyl, N-(C₁-C₆-Alkyl)-N-(phenyl)-aminocarbonyl, Phenyl-C₁-C₆-alkylcarbonyl, Heterocyclyl, Heterocyclyl-C₁-C₆-alkyl, Heterocyclylcarbonyl, Heterocyclylcarbonyl-C₁-C₆-alkyl, Heterocyclyloxycarbonyl, Heterocyclylaminocarbonyl, Heterocyclylsulfonylaminocarbonyl, N-(C₁-C₆-Alkyl)-N-(heterocyclyl)-aminocarbonyl, oder Heterocyclyl-C₁-C₆-alkylcarbonyl, wobei die Phenyl- oder Heterocyclyl-Teile der Substituenten partiell oder vollständig halogeniert sein können und/oder eine bis drei der folgenden Gruppen tragen können: Nitro, Cyano, C₁-C₄-Alkyl, C₁-C₄-Halogenalkyl, C₁-C₄-Alkoxy oder C₁-C₄-Halogenalkoxy; oder
S(O)ₙR³³ bedeuten, wobei
n 1 oder 2 bedeutet; und
R³³ C₁-C₆-Alkyl, C₁-C₆-Halogenalkyl oder Phenyl bedeutet, und wobei der Phenylsubstituent partiell oder vollständig halogeniert sein kann und/oder eine bis drei der folgenden Gruppen tragen kann: Nitro, Cyano, C₁-C₄-Alkyl, C₁-C₄-Halogenalkyl, C₁-C₄-Alkoxy oder C₁-C₄-Halogenalkoxy; und
R³⁰ und R³¹ unabhängig voneinander Wasserstoff, C₁-C₆-Alkyl, C₃-C₆-Cycloalkyl, C₂-C₆-Alkenyl oder C₂-C₆-Alkinyl, wobei aliphatische oder isocyclische Teile der Substituenten partiell oder vollständig halogeniert sein können und/oder eine bis drei der folgenden Gruppen tragen können: Cyano, Hydroxy, C₁-C₄-Alkyl, C₁-C₄-Halogenalkyl, C₃-C₆-Cycloalkyl, C₁-C₄-Alkoxy, C₁-C₄-Alkylthio, [Di-(C₁-C₄)-alkyl]-amino, C₁-C₄-Alkylcarbonyl, Hydroxycarbonyl, C₁-C₄-Alkoxycarbonyl, Aminocarbonyl, C₁-C₄-Alkylaminocarbonyl, [Di-(C₁-C₄)-alkyl]aminocarbonyl oder C₁-C₄-Alkylcarbonyloxy,
Phenyl, Phenyl-C₁-C₆-alkyl, Heterocyclyl oder Heterocyclyl-C₁-C₆-alkyl bedeuten, wobei die Phenyl- oder Heterocyclyl-Teile der Substituenten partiell oder vollständig halogeniert sein können und/oder eine bis drei der folgenden Gruppen tragen können: Nitro, Cyano, C₁-C₄-Alkyl, C₁-C₄-Halogenalkyl, C₁-C₄-Alkoxy oder C₁-C₄-Halogenalkoxy;
R⁴, R⁵, R⁶ unabhängig voneinander Wasserstoff, C₁-C₆-Alkyl oder C₁-C₆-Alkoxy, wobei die genannten aliphatischen Teile der Substituenten von R⁴, R⁵ oder R⁶ partiell oder vollständig halogeniert sein können und/oder eine bis drei der folgenden Gruppen tragen können: Cyano, Hydroxy, C₁-C₄-Alkyl, C₁-C₄-Halogenalkyl, C₃-C₆-Cycloalkyl, C₁-C₄-Alkoxy, C₁-C₄-Alkylthio, [Di-(C₁-C₄)-alkylj-amino, C₁-C₄-Alkylcarbonyl, Hydroxycarbonyl, C₁-C₄-Alkoxycarbonyl, Aminocarbonyl, C₁-C₄-Alkylaminocarbonyl, [Di-(C₁-C₄)-alkyl]aminocarbonyl oder C₁-C₄-Alkylcarbonyloxy;
R³ und R⁴ auch gemeinsam eine Ketogruppe bedeuten können;
R⁷, R⁸ unabhängig voneinander Wasserstoff, Hydroxy, C₁-C₆-Alkyl, das partiell oder vollständig halogeniert sein kann und/oder eine bis drei der folgenden Gruppen tragen kann: Cyano, Hydroxy, C₁-C₄-Alkyl, C₁-C₄-Halogenalkyl, C₃-C₆-Cycloalkyl, C₁-C₄-Alkoxy, C₁-C₄-Alkylthio, [Di-(C₁-C₄)-alkyl]-amino, C₁-C₄-Alkylcarbonyl, Hydroxycarbonyl, C₁-C₄-Alkoxycarbonyl, Aminocarbonyl, C₁-C₄-Alkylaminocarbonyl, [Di-(C₁-C₄)-alkyl]aminocarbonyl oder C₁-C₄-Alkylcarbonyloxy;
A¹, A² unabhängig voneinander Aryl oder Heteroaryl, mit der Ausnahme von Indolyl, bedeuten, wobei R^{a} in ortho-Position zur Verknüpfungsstelle von A¹ an ein C-Atom oder N-Atom von A¹ gebunden ist und wobei R^{a} eine der im Folgenden angegebenen Bedeutungen aufweist:
R^{a} Halogen, Cyano, Nitro, C₁-C₆-Alkyl, C₃-C₆-Cycloalkyl, C₂-C₆-Alkenyl, C₃-C₆-Cycloalkenyl, C₄-C₁₀-Alkadienyl, C₂-C₆-Alkinyl, [Tri-(C₁-C₆)-alkylsilyl]-(C₂-C₆)-alkinyl, C₃-C₆-Cycloalkinyl, C₁-C₆-Alkylthio, C₁-C₆-Alkylsulfinyl, Aryl, Phenyl-(C₁-C₆)-Alkyl, Phenyl-(C₂-C₆)-Alkenyl, Phenylsulfonyl-(C₁-C₆)-Alkyl, Heterocyclyl, Heterocydyl-(C₁-C₆)-Alkyl oder Phenyl-[C₁-C₆-alkoxycarbonyl]-(C₁-C₆)-alkyl,
Z¹P(O)(OR⁹)₂, Z²B(OR¹⁰)₂, wobei
R⁹ und R¹⁰ jeweils Wasserstoff oder C₁-C₆-Alkyl bedeuten und R¹⁰ in Z²B(OR¹⁰)₂ zusammen eine C₂-C₄-Alkylenkette bilden können; oder
Z³COR¹¹, wobei
R¹¹ Wasserstoff, C₁-C₆-Alkyl, C₃-C₆-Cycloalkyl, C₂-C₆-Alkenyl, C₃-C₆-Cycloalkenyl, C₂-C₆-Alkinyl, C₃-C₆-Cycloalkinyl, Hydroxy, C₁-C₆-Alkoxy, C₃-C₆-Alkenyloxy, C₃-C₆-Alkinyloxy, Amino, C₁-C₆-Alkylamino, [Di-(C₁-C₆)-alkyl]amino, C₁-C₆-Alkoxyamino, [Di-(C₁-C₆)-Alkoxy]amino, C₁-C₆-Alkylsulfonylamino, C₁-C₆-Alkylaminosulfonylamino, [Di-(C₁-C₆)-alkylamino]sulfonylamino, C₃-C₆-Alkenylamino, C₃-C₆-Alkinylamino, N-(C₂-C₆-Alkenyl)-N-(C₁-C₆-alkyl)-amino, N-(C₂-C₆-Alkinyl)-N-(C₁-C₆-alkyl)-amino, N-(C₁-C₆-Alkoxy)-N-(C₁-C₆-alkyl)-amino, N-(C₂-C₆-Alkenyl)-N-(C₁-C₆-alkoxy)-amino, N-(C₂-C₆-Alkinyl)-N-(C₁-C₆-alkoxy)-amino, Phenyl, Phenoxy, Phenylamino, Naphthyl oder Heterocyclyl bedeutet; oder
Z⁴NR¹²R¹³, wobei
R¹² und R¹³ unabhängig voneinander Wasserstoff, C₁-C₆-Alkyl, C₃-C₆-Cycloalkyl, C₃-C₆-Alkenyl, C₃-C₆-Cycloalkenyl, C₃-C₆-Alkinyl, C₃-C₆-Cycloalkinyl, C₁-C₆-Alkylcarbonyl, C₃-C₆-Cycloalkylcarbonyl, [Di-(C₁-C₆)-alkylamino]carbonyl, C₁-C₆-Alkoxycarbonyl, C₁-C₆-Alkoxycarbonyl-(C₁-C₆)-alkyl, C₁-C₆-Alkylsulfonyl, C₁-C₆-Alkylaminosulfonyl, [Di-(C₁-C₆)-alkylamino]sulfonyl, Phenylcarbonyl, Phenylaminocarbonyl, Phenylsulfonyl, Phenylsulfonylaminocarbonyl oder Heterocyclylcarbonyl bedeuten; oder
Z⁵CH=N-O-R¹⁴, wobei R¹⁴ Wasserstoff oder C₁-C₆-Alkyl bedeutet; oder
Z⁶OR¹⁵, wobei
R₁₅ Wasserstoff, C₁-C₆-Alkyl, C₃-C₆-Cycloalkyl, C₃-C₆-Alkenyl, C₃-C₆-Cycloalkenyl, C₃-C₆-Alkinyl, C₃-C₆-Cycloalkinyl, C₁-C₆-Alkylcarbonyl, C₁-C₆-Atkoxycarbonyl-(C₁-C₆)-alkyl, [Di-(C₁-C₆)-Alkoxycarbonyl]-(C₁-C₆)-alkyl, Phenyl oder Phenyl-(C₁-C₆)-Alkyl bedeutet; oder
Z⁷SO₂R¹⁶, wobei R¹⁶ C₁-C₆-Alkyl oder Phenyl bedeutet; und wobei
Z¹, Z², Z³, Z⁴, Z⁵, Z⁶, Z⁷ unabhängig voneinander eine Bindung, -CH₂-, -CH₂-CH₂-, -O-CH(R¹⁷)-, -S-CH(R¹⁸)-, -S(O)-CH(R¹⁹)- oder -SO₂CH(R²⁰)-bedeutet, und wobei R¹⁷, R¹⁸, R¹⁹ und R²⁰ unabhängig voneinander Wasserstoff oder C₁-C₆-Alkyl bedeuten; und
wobei die genannten aliphatischen, cyclischen oder aromatischen Teile des Substituenten R^{a} partiell oder vollständig halogeniert sein können und/oder eine bis drei der folgenden Gruppen tragen können: Cyano, Hydroxy, C₁-C₄-Alkyl, C₁-C₄-Halogenalkyl, C₃-C₆-Cycloalkyl, C₁-C₄-Alkoxy, C₁-C₄-Alkylthio, [Di-(C₁-C₄)-alkyl]-amino, C₁-C₄-Alkylcarbonyl, Hydroxycarbonyl, C₁-C₄-Alkoxycarbonyl, Aminocarbonyl, C₁-C₄-Alkylaminocarbonyl, [Di-(C₁- C₄)-alkyl]aminocarbonyl oder C₁-C₄-Alkylcarbonyloxy; und
R^{b}, R^{c}, R^{d}, R^{e} und R^{f} jeweils unabhängig voneinander für Wasserstoff stehen oder eine der für R^{a} angegebenen Bedeutungen aufweisen; und
worin zwei an benachbarte Ringatome von A¹ gebundene Reste R^{a}, R^{b} oder R^{c} oder zwei an benachbarte Ringatome von A² gebundene Reste R^{d}, R^{e} oder R^{f} auch für lineares C₃-C₆-Alkylen stehen können, das teilweise oder vollständig halogeniert sein kann und das eine bis drei der folgenden Gruppen tragen kann: Cyano, Hydroxy, C₁-C₄-Alkyl, C₁-C₄-Halogenalkyl, C₃-C₆-Cycloalkyl, C₁-C₄-Alkoxy, C₁-C₄-Alkylthio, [Di-(C₁-C₄)-alkyl]-amino, C₁-C₄-Alkylcarbonyl, Hydroxycarbonyl, C₁-C₄-Alkoxycarbonyl, Aminocarbonyl, C₁-C₄-Alkylaminocarbonyl, [Di-(C₁-C₄)-alkyl]aminocarbonyl oder C₁-C₄-Alkylcarbonyloxy, worin eine CH₂-Gruppe in C₃-C₆-Alkylen durch eine Carbonylgruppe, Thiocarbonylgruppe oder Sulfonylgruppe ersetzt sein kann und worin eine oder zwei nicht benachbarte CH₂-Gruppen in C₃-C₆-Alkylen jeweils durch Sauerstoff, Schwefel oder eine Gruppe NR³⁴ ersetzt sein können, wobei R³⁴ eine für R¹² angegebenen Bedeutungen aufweist.

2. Verwendung von Piperazinverbindungen der Formel I nach Anspruch 1, worin A¹ und A² unabhängig voneinander ausgewählt sind aus der Gruppe Phenyl, Naphthyl, Furyl, Thienyl, Pyrrolyl, Pyrazolyl, Thiazolyl, Isothiazolyl, Oxazolyl, Isoxazolyl, Triazolyl, Tetrazolyl, Pyridinyl, Pyridazinyl, Pyrimidinyl, Pyrazinyl, Triazinyl und Tetrazinyl.

3. Verwendung von Piperazinverbindungen der Formel I nach einem der vorhergehenden Ansprüche, worin A¹ und A² unabhängig voneinander ausgewählt sind aus der Gruppe Phenyl, Furyl, Thienyl, Triazolyl, Tetrazolyl und Pyridinyl.

4. Verwendung von Piperazinverbindungen der Formel I nach einem der vorhergehenden Ansprüche, worin A¹ für Phenyl oder Pyridinyl steht.

5. Verwendung von Piperazinverbindungen der Formel I nach einem der Ansprüche 1 bis 4, worin A² für Phenyl oder Thienyl steht.

6. Verwendung von Piperazinverbindungen der Formel I nach nach einem der vorhergehenden Ansprüche, worin:
R^{a} ausgewählt ist unter Halogen, Cyano, Nitro, C₂-C₆-Alkenyl, C₂-C₆-Alkinyl, Z¹P(O)(OR⁹)₂, Z³COR¹¹, Z⁴NR¹²R¹³, Z⁵CH=N-O-R¹⁴, Z⁶OR¹⁵, Z⁷SO₂R¹⁶, C₁-C₆-Alkylthio, C₁-C₆-Alkylsulfinyl, Aryl und Heterocyclyl, wobei
Z¹ eine Bindung oder CH₂ bedeutet und R⁹ und R¹⁰ jeweils Wasserstoff oder C₁-C₆-Alkyl bedeuten;
Z³ eine Bindung bedeutet und R¹¹ Wasserstoff, C₁-C₆-Alkyl, Hydroxy, C₁-C₆-Alkoxy, C₃-C₆-Alkenyloxy, C₃-C₆-Alkinyloxy, Amino, C₁-C₆-Alkylamino, [Di-(C₁-C₆)-alkyl]amino, C₁-C₆-Alkoxyamino, N-C₁-C₆-Alkoxy-N-C₁-C₆-alkylamino, C₁-C₆-Alkyisulfonylamino, C₁-C₆-Alkylaminosulfonylamino, [Di-(C₁-C₆)-alkylamino]sulfonylamino, Phenyl, Phenoxy, Phenylamino, Naphthyl oder Heterocyclyl bedeutet; oder
Z⁴ eine Bindung oder CH₂ bedeutet und R¹² und R¹³ unabhängig voneinander Wasserstoff, C₁-C₆-Alkyl, C₃-C₆-Cycloalkyl, C₃-C₆-Alkenyl, C₃-C₆-Alkinyl, C₁-C₆-Alkylcarbonyl, [Di-(C₁-C₆)-alkylamino]carbonyl, C₁-C₆-Alkoxycarbonyl, C₁-C₆-Alkylsulfonyl, Phenylcarbonyl, Phenylsulfonyl, oder Heterocyclylcarbonyl bedeuten; oder
Z⁵ eine Bindung oder CH₂ bedeutet und R¹⁴ Wasserstoff oder C₁-C₆-Alkyl bedeutet; oder
Z⁶ eine Bindung oder CH₂ bedeutet und R¹⁵, Wasserstoff, C₁-C₆-Alkyl, C₃-C₆-Cycloalkyl, C₃-C₆-Alkenyl, C₃-C₆-Alkinyl, C₁-C₆-Alkylcarbonyl, C₁-C₆-Alkoxycarbonyl-(C₁-C₆)-alkyl, Phenyl oder Phenyl-(C₁-C₆)-Alkyl bedeutet; oder
Z⁷ eine Bindung bedeutet und R¹⁶ C₁-C₆-Alkyl oder Phenyl;
und wobei
R^{b}, R^{c}, R^{d}, R^{e} und R^{f} unabhängig voneinander für Wasserstoff stehen, eine der für R^{a} genannten Bedeutungen haben sowie zusätzlich stehen können für:
C₁-C₆-Alkyl, C₃-C₆-Cycloalkyl, [Tri-(C₁-C₆)-alkylsilyl]-(C₂-C₆)-alkinyl;
und wobei die genannten aliphatischen, cyclischen oder aromatischen Teile der Substituenten R^{a}, R^{b}, R^{c}, R^{d}, R^{e} und R^{f} partiell oder vollständig halogeniert sein können.

7. Verwendung von Piperazinverbindungen der Formel I nach einem der vorhergehenden Ansprüche, worin
R¹ Wasserstoff oder C₁-C₆-Alkyl; und
R² C₁-C₆-Alkyl;
bedeuten, wobei C₁-C₆-Alkyl in R¹ und R² partiell oder vollständig halogeniert sein kann.

8. Verwendung von Piperazinverbindungen der Formel I nach einem der vorhergehenden Ansprüche, worin
R³ für R²⁶ oder OR²⁷ steht, wobei
R²⁶ und R²⁷ unabhängig voneinander Wasserstoff, C₁-C₆-Alkyl C₁-C₆-Alkylcarbonyl, Phenyl-C₁-C₆-alkyl oder Phenylcarbonyl, wobei die genannten aliphatischen oder aromatischen Teile der Substituenten partiell oder vollständig halogeniert sein können; oder eine Gruppe SO₂R³¹ bedeuten, wobei
R³¹ C₁-C₆-Alkyl oder Phenyl bedeutet, und wobei der Phenylsubstituent partiell oder vollständig halogeniert sein kann und/oder eine bis drei C₁-C₆-Alkyl-Gruppen tragen kann.

9. Verwendung von Piperazinverbindungen der Formel I nach einem der vorhergehenden Ansprüche, worin R⁴, R⁵, R⁶, R⁷ und R⁸ für Wasserstoff stehen.

10. Verwendung von Piperazinverbindungen der Formel I nach einem der vorhergehenden Ansprüche, bei denen die beiden Chiralitätszentren im Piperazinring die (S,S)-Konfiguration aufweisen.

11. Mittel, enthaltend eine herbizid wirksame Menge mindestens einer Piperazinverbindung der Formel I oder eines landwirtschaftlich brauchbaren Salzes von I nach einem der Ansprüche 1 bis 10 und für die Formulierung von Pflanzenschutzmitteln übliche Hilfsmittel.

12. Verfahren zur Herstellung von Mitteln gemäß Anspruch 11, **dadurch gekennzeichnet, dass** man eine herbizid wirksame Menge mindestens einer Piperazinverbindung der Formel I oder eines landwirtschaftlich brauchbaren Salzes von I. nach einem der Ansprüche 1 bis 10 und für die Formulierung von Pflanzenschutzmitteln übliche Hilfsmittel mischt.

13. Verfahren zur Bekämpfung von unerwünschtem Pflanzenwuchs, **dadurch gekennzeichnet, dass** man eine herbizid wirksame Menge mindestens einer Piperazinverbindung der Formel I oder eines landwirtschaftlich brauchbaren Salzes von I nach einem der Ansprüche 1 bis 10 auf Pflanzen, deren Samen und/oder deren Lebensraum einwirken läßt.

14. Piperazinverbindungen der allgemeinen Formel I gemäß einem der vorhergehenden Ansprüche,
ausgenommen Verbindungen der Formel I, worin A¹ und A² Phenyl bedeuten, R¹ Methyl, R² Wasserstoff oder Methyl, R³, R⁴, R⁵, R⁶, R⁷, R⁸ Wasserstoff bedeuten, die Substituenten R^{a} und R^{b} jeweils für Hydroxy stehen, die in den beiden ortho-Position des Phenylrings A¹ angeordnet sind, und R^{c}, R^{d}, R^{e} und R^{f} Wasserstoff bedeuten,
weiterhin ausgenommen Verbindungen der Formel I, worin A¹ für Phenyl steht und A² 4-Imidazolyl bedeutet oder A¹ für 4-Imidazolyl steht und A² Phenyl bedeutet,
weiterhin ausgenommen Verbindungen der Formel I, worin A¹ für Phenyl steht, R^{a} Chlor bedeutet, R^{b} und R^{c} für Wasserstoff stehen, die Gruppe A²(R^{d}R^{e}R^{f}) für o-Chlorphenyl steht, R¹ und R² Methylcarbonyl bedeuten und R³ bis R⁸ jeweils Wasserstoff bedeuten.

15. Dipeptide der Formel II, 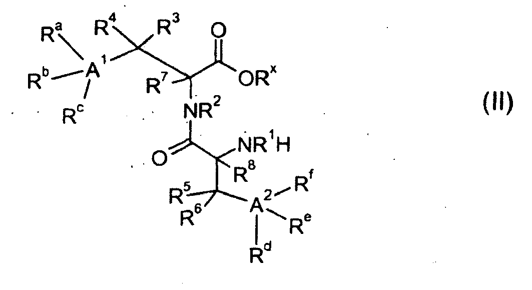 worin die Variablen A¹, A², R¹ - R⁸, R^{a}, R^{b}, R^{c}, R^{d}, R^{e} und R^{f} die für Formel I angegebene Bedeutung aufweisen und worin OR^{x} für eine über ein Sauerstoffatom gebundene Abgangsgruppe steht.

16. Dipeptide der Formel VI, 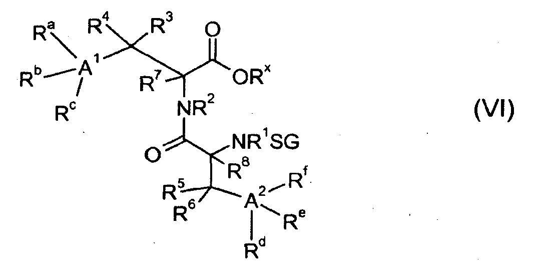 worin die Variablen A¹, A², R¹ - R⁸, R^{a}, R^{b}, R^{c}, R^{d}, R^{e} und R^{f} die für Formel I angegebene Bedeutung aufweisen und SG eine Schutzgruppe bedeutet und worin OR^{x} für eine über ein Sauerstoffatom gebundene Abgangsgruppe steht.

## Claims

1. The use of piperazine compounds of the formula I 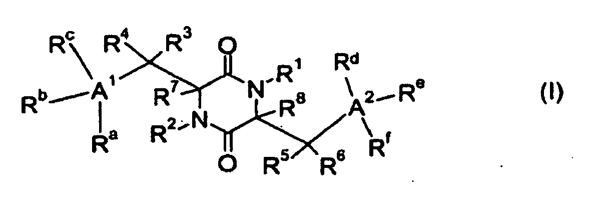 or of the agriculturally useful salts of piperazine compounds of the formula I as herbicides, where in formula I the variables are as defined below:
R¹ and R² independently of one another are:
cyano, C₁-C₆-alkyl, C₃-C₆-cycloalkyl, C₃-C₆-alkenyl, C₃-C₆-cycloalkenyl, C₃-C₆-alkynyl, C₃-C₆-cycloalkynyl, phenyl, phenyl-(C₁-C₆)-alkyl, heterocyclyl, heterocyclyl-(C₁-C₆)-alkyl; phenyl-[C₁-C₆-alkoxycarbonyl]-(C₁-C₆)-alkyl or phenylheterocyclyl-(C₁-C₆)-alkyl; or
COR²¹, where
R²¹ is hydrogen, C₁-C₆-alkyl, C₃-C₆-cycloalkyl, C₂-C₆-alkenyl, C₃-C₆-cycloalkenyl, C₂-C₆-alkynyl, C₃-C₆-cycloalkynyl, hydroxyl, C₁-C₆-alkoxy, C₃-C₆-alkenyloxy, C₃-C₆-alkynyloxy, amino, C₁-C₆-alkylamino, [di-(C₁-C₆)-alkyl]amino, C₃-C₆-alkenylamino, C₃-C₆-alkynylamino, C₁-C₆-alkylsulfonylamino, N-(C₂-C₆-alkenyl)-N-(C₁-C₆-alkyl)amino, N-(C₂-C₆-alkynyl)-N-(C₁-C₆-alkyl)amino, N-(C₁-C₆-alkoxy)-N-(C₁-C₆-alkyl)amino, N-(C₂-C₆-alkenyl)-N-(C₁-C₆-alkoxy)amino, N-(C₂-C₆-alkynyl)-N-(C₁-C₆-alkoxy)amino, phenyl, phenylamino, phenoxy, naphthyl or heterocyclyl; or
NR²²R²³, where
R²² and R²³ independently of one another are hydrogen, C₁-C₆-alkyl, C₃-C₆-cycloalkyl, C₃-C₆-alkenyl, C₃-C₆-cycloalkenyl, C₃-C₆-alkynyl, C₃-C₆-cycloalkynyl or C₁-C₆-alkylcarbonyl; or
OR²⁴, where
R²⁴ is C₁-C₆-alkyl, C₃-C₆-cycloalkyl, C₃-C₆-alkenyl, C₃-C₆-cycloalkenyl, C₃-C₆-alkynyl, C₃-C₆-cycloalkynyl, phenyl or phenyl-(C₁-C₆)-alkyl; or
SO₂R²⁵, where
R²⁵ is C₁-C₆-alkyl or phenyl;
where the abovementioned aliphatic, cyclic or aromatic moieties of the substituents of R¹ and R² may be partially or fully halogenated and/or may carry one to three of the following groups: cyano, hydroxyl, C₁-C₄-alkyl, C₁-C₄-haloalkyl, C₃-C₆-cycloalkyl, C₁-C₄-alkoxy, C₁-C₄-alkylthio, [di-(C₁-C₄)-alkyl]amino, C₁-C₄-alkylcarbonyl, hydroxycarbonyl, C₁-C₄-alkoxycarbonyl, aminocarbonyl, C₁-C₄-alkylaminocarbonyl, [di-(C₁-C₄)-alkyl]aminocarbonyl or C₁-C₄-alkylcarbonyloxy;
and where R¹ may additionally be hydrogen;
R³ is a radical R²⁶, OR²⁷, SR²⁸, NR²⁹R³⁰ or N(OR³¹)R³², where
R²⁶, R²⁷, R²⁸, R²⁹ and R³² independently of one another are hydrogen, C₁-C₆-alkyl, C₃-C₆-cycloalkyl, C₃-C₆-alkenyl, C₃-C₆-alkynyl, C₃-C₆-haloalkynyl, formyl, C₁-C₆-alkylcarbonyl, C₃-C₆-cycloalkylcarbonyl, C₂-C₆-alkenylcarbonyl, C₂-C₆-alkynylcarbonyl, C₁-C₆-alkoxy-(C₁-C₆)-alkyl, C₁-C₆-alkoxycarbonyl, C₂-C₆-alkenyloxycarbonyl, C₃-C₆-alkynyloxycarbonyl, C₁-C₆-alkylaminocarbonyl, C₃-C₆-alkenylaminocarbonyl, C₃-C₆-alkynylaminocarbonyl, C₁-C₆-alkylsulfonylaminocarbonyl, C₁-C₆-alkylaminocarbonyl, [di-(C₁-C₆)-alkylamino] carbonyl, N-(C₃-C₆-alkenyl)-N-(C₁-C₆-alkyl)aminocarbonyl, N-(C₃-C₆-alkynyl)-N-(C₁-C₆-alkyl)aminocarbonyl, N-(C₁-C₆-alkoxy)-N-(C₁-C₆-alkyl)aminocarbonyl, N-(C₃-C₆-alkenyl)-N-(C₁-C₆-alkoxy)aminocarbonyl, N-(C₃-C₆-alkynyl)-N-(C₁-C₆-alkoxy)-aminocarbonyl, di-(C₁-C₆-alkyl)-aminothiocarbonyl, C₁-C₆-alkylcarbonyl-C₁-C₆-alkyl, C₁-C₆-alkoxyimino-C₁-C₆-alkyl, N-(C₁-C₆-alkylamino)-imino-C₁-C₆-alkyl, N- (di-C₁-C₆-alkylamino)-imino-C₁-C₆-alkyl or [tri-(C₁-C₄)-alkyl]silyl, where the abovementioned aliphatic or isocyclic moieties of the substituents may be partially or fully halogenated and/or may carry one to three of the following groups: cyano, hydroxyl, C₁-C₄-alkyl, C₁-C₄-haloalkyl, C₃-C₆-cycloalkyl, C₁-C₄-alkoxy, C₁-C₄-alkylthio, [di-(C₁-C₄-)alkyl]amino, C₁-C₄-alkylcarbonyl, hydroxycarbonyl, C₁-C₄-alkoxycarbonyl, aminocarbonyl, C₁-C₄-alkylaminocarbonyl, [di-(C₁-C₄-)alkyl]aminocarbonyl or C₁-C₄-alkylcarbonyloxy; or
phenyl, phenyl-C₁-C₆-alkyl, phenylcarbonyl, phenylcarbonyl-C₁-C₆-alkyl, phenoxycarbonyl, phenylaminocarbonyl, phenylsulfonylaminocarbonyl, N-(C₁-C₆-alkyl)-N-(phenyl)aminocarbonyl, phenyl-C₁-C₆-alkylcarbonyl, heterocyclyl, heterocyclyl-C₁-C₆-alkyl, heterocyclylcarbonyl, heterocyclylcarbonyl-C₁-C₆-alkyl, hetercyclyloxycarbonyl, heterocyclylaminocarbonyl, heterocyclylsulfonylaminocarbonyl, N-(C₁-C₆-alkyl)-N-(heterocyclyl)-aminocarbonyl or heterocyclyl-C₁-C₆-alkylcarbonyl, where the phenyl or heterocyclyl moieties of the substituents may be partially or fully halogenated and/or may carry one to three of the following groups: nitro, cyano, C₁-C₄-alkyl, C₁-C₄-haloalkyl, C₁-C₄-alkoxy or C₁-C₄-haloalkoxy; or
S(O)ₙR³³, where
n is 1 or 2;
R³³ is C₁-C₆-alkyl, C₁-C₆-haloalkyl or phenyl, where the phenyl substituent may be partially or fully halogenated and/or may carry one to three of the following groups: nitro, cyano, C₁-C₄-alkyl, C₁-C₄-haloalkyl, C₁-C₄-alkoxy or C₁-C₄-haloalkoxy; and
R³⁰ and R³¹ independently of one another are hydrogen, C₁-C₆-alkyl, C₃-C₆-cycloalkyl , C₂-C₆-alkenyl or C₂-C₆-alkynyl, where aliphatic or isocyclic moieties of the substituents may be partially or fully halogenated and/or may carry one to three of the following groups: cyano, hydroxyl, C₁-C₄-alkyl, C₁-C₄-haloalkyl, C₃-C₆-cycloalkyl, C₁-C₄-alkoxy, C₁-C₄-alkylthio, [di(C₁-C₄-)alkyl]amino, C₁-C₄-alkylcarbonyl, hydroxycarbonyl, C₁-C₄-alkoxycarbonyl, aminocarbonyl, C₁-C₄-alkylaminocarbonyl, [di(C₁-C₄-)alkyl]aminocarbonyl or C₁-C₄-alkylcarbonyloxy,
phenyl, phenyl-C₁-C₆-alkyl, heterocyclyl or heterocyclyl-C₁-C₆-alkyl, where the phenyl or the heterocyclyl moieties of the substituents may be partially or fully halogenated and/or may carry one to three of the following groups: nitro, cyano, C₁-C₄-alkyl, C₁-C₄-haloalkyl, C₁-C₄-alkoxy or C₁-C₄-haloalkoxy;
R⁴, R⁵, R⁶ independently of one another are hydrogen, C₁-C₆-alkyl or C₁-C₆-alkoxy, where the abovementioned aliphatic moieties of the substituents of R⁴, R⁵ or R⁶ may be partially or fully halogenated and/or may carry one to three of the following groups: cyano, hydroxyl, C₁-C₄-alkyl, C₁-C₄-haloalkyl, C₃-C₆-cycloalkyl, C₁-C₄-alkoxy, C₁-C₄-alkylthio, [di-(C₁-C₄)-alkyl]-amino, C₁-C₄-alkylcarbonyl, hydroxycarbonyl, C₁-C₄-alkoxycarbonyl, aminocarbonyl, C₁-C₄-alkylaminocarbonyl, [di-(C₁-C₄)-alkyl]aminocarbonyl or C₁-C₄-alkylcarbonyloxy;
R³ and R⁴ together may also be a keto group;
R⁷, R⁸ independently of one another are hydrogen, hydroxyl, C₁-C₆-alkyl which may be partially or fully halogenated and/or may carry one to three of the following groups: cyano, hydroxyl, C₁-C₄-alkyl, C₁-C₄-haloalkyl, C₃-C₆-cycloalkyl, C₁-C₄-alkoxy, C₁-C₄-alkylthio, [di-(C₁-C₄)-alkyl]amino, C₁-C₄-alkylcarbonyl, hydroxycarbonyl, C₁-C₄-alkoxycarbonyl, aminocarbonyl, C₁-C₄-alkylaminocarbonyl, [di-(C₁-C₄-alkyl]aminocarbonyl or C₁-C₄-alkylcarbonyloxy;
A¹, A² independently of one another are aryl or heteroaryl except for indolyl, where R^{a} is attached in the ortho position to the point of attachment of A¹ to a carbon atom or nitrogen atom of A¹ and where R^{a} has one of the meanings given below:
R^{a} is halogen, cyano, nitro, C₁-C₆-alkyl, C₃-C₆-cycloalkyl, C₂-C₆-alkenyl, C₃-C₆-cycloalkenyl, C₄-C₁₀-alkadienyl, C₂-C₆-alkynyl, [tri-(C₁-C₆)-alkylsilyl]-(C₂-C₆)-alkynyl, C₃-C₆-cycloalkynyl, C₁-C₆-alkylthio, C₁-C₆-alkylsulfinyl, aryl, phenyl-(C₁-C₆)-alkyl, phenyl-(C₂-C₆)-alkenyl, phenylsulfonyl-(C₁-C₆)-alkyl, heterocyclyl, heterocyclyl-(C₁-C₆)-alkyl or phenyl-[C₁-C₆-alkoxycarbonyl]-(C₁-C₆)-alkyl,
Z¹P(O)(OR⁹)₂, Z²B(OR¹⁰)₂, where
R⁹ and R¹⁰ are each hydrogen or C₁-C₆-alkyl and the radicals R¹⁰ in Z²B(OR¹⁰)₂ together may form a C₂-C₄-alkylene chain; or
Z³COR¹¹, where
R¹¹ is hydrogen, C₁-C₆-alkyl, C₃-C₆-cycloalkyl, C₂-C₆-alkenyl, C₃-C₆-cycloalkenyl, C₂-C₆-alkynyl, C₃-C₆-cycloalkynyl, hydroxyl, C₁-C₆-alkoxy, C₃-C₆-alkenyloxy, C₃-C₆-alkynyloxy, amino, C₁-C₆-alkylamino, [di-(C₁-C₆)-alkyl]amino, C₁-C₆-alkoxyamino, [di-(C₁-C₆)-alkoxy]amino, C₁-C₆-alkylsulfonylamino, C₁-C₆-alkylaminosulfonylamino, [di-(C₁-C₆)-alkylamino]sulfonylamino, C₃-C₆-alkenylamino, C₃-C₆-alkynylamino, N-(C₂-C₆-alkenyl)-N-(C₁-C₆-alkyl)amino, N-(C₂-C₆-alkynyl)-N-(C₁-C₆-alkyl)amino, N-(C₁-C₆-alkoxy)-N-(C₁-C₆-alkyl)amino, N-(C₂-C₆-alkenyl)-N-(C₁-C₆-alkoxy)amino, N-(C₂-C₆-alkynyl)-N-(C₁-C₆-alkoxy)amino, phenyl, phenoxy, phenylamino, naphthyl or heterocyclyl; or
Z⁴NR¹²R¹³, where
R¹² and R¹³ independently of one another are hydrogen, C₁-C₆-alkyl, C₃-C₆-cycloalkyl, C₃-C₆-alkenyl, C₃-C₆-cycloalkenyl, C₃-C₆-alkynyl, C₃-C₆-cycloalkynyl, C₁-C₆-alkylcarbonyl, C₃-C₆-cycloalkylcarbonyl, [di-(C₁-C₆)-alkylamino]carbonyl, C₁-C₆-alkoxycarbonyl, C₁-C₆-alkoxycarbonyl-(C₁-C₆) alkyl, C₁-C₆-alkylsulfonyl, C₁-C₆-alkylaminosulfonyl, [di-(C₁-C₆)-alkylamino]sulfonyl, phenylcarbonyl, phenylaminocarbonyl, phenylsulfonyl, phenylsulfonylaminocarbonyl or heterocyclylcarbonyl; or
Z⁵CH=N-O-R¹⁴, where R¹⁴ is hydrogen or C₁-C₆-alkyl; or
Z⁶OR¹⁵, where
R¹⁵ is hydrogen, C₁-C₆-alkyl, C₃-C₆-cycloalkyl, C₃-C₆-alkenyl, C₃-C₆-cycloalkenyl, C₃-C₆-alkynyl, C₃-C₆-cycloalkynyl, C₁-C₆-alkylcarbonyl, C₁-C₆-alkoxycarbonyl-(C₁-C₆)-alkyl, [di-(C₁-C₆)-alkoxycarbonyl]-(C₁-C₆)-alkyl, phenyl or phenyl-(C₁-C₆)-alkyl; or
Z⁷SO₂R¹⁶, where R¹⁶ is C₁-C₆-alkyl or phenyl; and where
Z¹, Z², Z³, Z⁴, Z⁵, Z⁶, Z⁷ independently of one another are a bond, -CH₂-, -CH₂-CH₂-, -O-CH(R¹⁷)--S-CH(R¹⁸)-, -S(O)-CH(R¹⁹)- or -SO₂CH(R²⁰)-, and where R¹⁷, R¹⁸, R¹⁹ and R²⁰ independently of one another are hydrogen or C₁-C₆-alkyl; and
where the abovementioned aliphatic, cyclic or aromatic moieties of the substituent R^{a} may be partially or fully halogenated and/or may carry one to three of the following groups: cyano, hydroxyl, C₁-C₄-alkyl, C₁-C₄-haloalkyl, C₃-C₆-cycloalkyl, C₁-C₄-alkoxy, C₁-C₄-alkylthio, [di(C₁-C₄)-alkyl]amino, C₁-C₄-alkylcarbonyl, hydroxycarbonyl, C₁-C₄-alkoxycarbonyl, aminocarbonyl, C₁-C₄-alkylaminocarbonyl, [di(C₁-C₄)-alkyl]aminocarbonyl or C₁-C₄-alkylcarbonyloxy; and
R^{b}, R^{c}, R^{d}, R^{e} and R^{f} are each independently of one another hydrogen or have one of the meanings given for R^{a}; and
where two radicals R^{a}, R^{b} or R^{c} attached to adjacent ring atoms of A¹ or two radicals R^{d}, R^{e} or R^{f} attached to adjacent ring atoms of A² may also be straight-chain C₃-C₆-alkylene which may be partially or fully halogenated and which may carry one to three of the following groups: cyano, hydroxyl, C₁-C₄-alkyl, C₁-C₄-haloalkyl, C₃-C₆-cycloalkyl, C₁-C₄-alkoxy, C₁-C₄-alkylthio, [di-(C₁-C₄)-alkyl]amino, C₁-C₄-alkylcarbonyl, hydroxycarbonyl, C₁-C₄-alkoxycarbonyl, aminocarbonyl, C₁-C₄-alkylaminocarbonyl, [di-(C₁-C₄)-alkyl]aminocarbonyl or C₁-C₄-alkylcarbonyloxy, where one CH₂ group in C₃-C₆-alkylene may be replaced by a carbonyl group, thiocarbonyl group or sulfonyl group and in which one or two non-adjacent CH₂ groups in C₃-C₆-alkylene may in each case be replaced by oxygen, sulfur or a group NR³⁴, where R³⁴ has one of the meanings given for R¹².

2. The use of piperazine compounds of the formula I according to claim 1 in which A¹ and A² independently of one another are selected from the group consisting of phenyl, naphthyl, furyl, thienyl, pyrrolyl, pyrazolyl, thiazolyl, isothiazolyl, oxazolyl, isoxazolyl, triazolyl, tetrazolyl, pyridinyl, pyridazinyl, pyrimidinyl, pyrazinyl, triazinyl and tetrazinyl.

3. The use of piperazine compounds of the formula I according to any of the preceding claims in which A¹ and A² independently of one another are selected from the group consisting of phenyl, furyl, thienyl, triazolyl, tetrazolyl and pyridinyl.

4. The use of piperazine compounds of the formula I according to any of the preceding claims in which A¹ is phenyl or pyridinyl.

5. The use of piperazine compounds of the formula I according to any of claims 1 to 4 in which A² is phenyl or thienyl.

6. The use of piperazine compounds of the formula I according to any of the preceding claims in which:
R^{a} is selected from the group consisting of halogen, cyano, nitro, C₂-C₆-alkenyl, C₂-C₆-alkynyl, Z¹P(O)(OR⁹)₂, Z³COR¹¹, Z⁴NR¹²R¹³, Z⁵CH=N-O-R¹⁴, Z⁶OR¹⁵, Z⁷SO₂R₁₆, C₁-C₆-alkylthio, C₁-C₆-alkylsulfinyl, aryl and heterocyclyl, where
Z¹ is a bond or CH₂ and R⁹ and R¹⁰ are each hydrogen or C₁-C₆-alkyl;
Z³ is a bond and R¹¹ is hydrogen, C₁-C₆-alkyl, hydroxyl, C₁-C₆-alkoxy, C₃-C₆-alkenyloxy, C₃-C₆-alkynyloxy, amino, C₁-C₆-alkylamino, [di-(C₁-C₆)-alkyl]amino, C₁-C₆-alkoxyamino, N-C₁-C₆-alkoxy-N-C₁-C₆-alkylamino, C₁-C₆-alkylsulfonylamino, C₁-C₆-alkylaminosulfonylamino, [di-(C₁-C₆)-alkylamino]sulfonylamino, phenyl, phenoxy, phenylamino, naphthyl or heterocyclyl; or
Z⁴ is a bond or CH₂ and R¹² and R¹³ independently of one another are hydrogen, C₁-C₆-alkyl, C₃-C₆-cycloalkyl, C₃-C₆-alkenyl, C₃-C₆-alkynyl, C₁-C₆-alkylcarbonyl, [di-(C₁-C₆)-alkylamino]carbonyl, C₁-C₆-alkoxycarbonyl, C₁-C₆-alkylsulfonyl, phenylcarbonyl, phenylsulfonyl, or heterocyclylcarbonyl; or
Z⁵ is a bond or CH₂ and R¹⁴ is hydrogen or C₁-C₆-alkyl; or
Z⁶ is a bond or CH₂ and R¹⁵ is hydrogen, C₁-C₆-alkyl, C₃-C₆-cycloalkyl, C₃-C₆-alkenyl, C₃-C₆-alkynyl, C₁-C₆-alkylcarbonyl, C₁-C₆-alkoxycarbonyl-(C₁-C₆)-alkyl, phenyl or phenyl-(C₁-C₆)-alkyl; or
Z⁷ is a bond and R¹⁶ is C₁-C₆-alkyl or phenyl;
and where
R^{b}, R^{c}, R^{d}, R^{e} and R^{f} independently of one another are hydrogen, have one of the meanings mentioned for R^{a}, and may additionally be: C₁-C₆-alkyl, C₃-C₆-cycloalkyl, [tri-(C₁-C₆)-alkylsilyl]-(C₂-C₆)-alkynyl;
and where the abovementioned aliphatic, cyclic or aromatic moieties of the substituents R^{a}, R^{b}, R^{c}, R^{d}, R^{e} and R^{f} may be partially or fully halogenated.

7. The use of piperazine compounds of the formula I according to any of the preceding claims in which
R¹ is hydrogen or C₁-C₆-alkyl; and
R² is C₁-C₆-alkyl;
where C₁-C₆-alkyl in R¹ and R² may be partially or fully halogenated.

8. The use of piperazine compounds of the formula I according to any of the preceding claims in which
R³ is R²⁶ or OR²⁷, where
R²⁶ and R²⁷ independently of one another are hydrogen, C₁-C₆-alkyl, C₁-C₆-alkylcarbonyl, phenyl-C₁-C₆-alkyl or phenylcarbonyl, where the abovementioned aliphatic or aromatic moieties of the substituents may be partially or fully halogenated; or a group SO₂R³¹ where
R³¹ is C₁-C₆-alkyl or phenyl, where the phenyl substituent may be partially or fully halogenated and/or may carry one to three C₁-C₆-alkyl groups.

9. The use of piperazine compounds of the formula I according to any of the preceding claims in which R⁴, R⁵, R⁶, R⁷ and R⁸ are hydrogen.

10. The use of piperazine compounds of the formula I according to any of the preceding claims in which the two centers of chirality in the piperazine ring have the (S,S)-configuration.

11. A composition comprising a herbicidally effective amount of at least one piperazine compound of the formula I or an agriculturally useful salt of I according to any of claims 1 to 10 and auxiliaries customary for formulating crop protection agents.

12. A method for preparing compositions according to claim 11 which comprises mixing a herbicidally effective amount of at least one piperazine compound of the formula I or an agriculturally useful salt of I according to any of claims 1 to 10 and auxiliaries customary for formulating crop protection agents.

13. A method for controlling unwanted vegetation which comprises allowing a herbicidally effective amount of at least one piperazine compound of the formula I or an agriculturally useful salt of I according to any of claims 1 to 10 to act on plants, their seeds and/or their habitat.

14. A piperazine compound of the general formula I according to any of the preceding claims,
except for compounds of the formula I in which A¹ and A² are phenyl, R¹ is methyl, R² is hydrogen or methyl, R³, R⁴, R^{5,} R⁶, R⁷, R⁸ are hydrogen, the substituents R^{a} and R^{b} are each hydroxyl which are located in the two ortho positions of the phenyl ring A¹, and R^{c}, R^{d}, R^{e} and R^{f} are hydrogen,
further except for compounds of the formula I in which A¹ is phenyl and A² is 4-imidazolyl or A¹ is 4-imidazolyl and A² is phenyl,
furthermore except for compounds of the formula I in which A¹ is phenyl, R^{a} is chlorine, R^{b} and R^{c} are hydrogen, the group A²(R^{d}R^{e}R^{f}) is o-chlorophenyl, R¹ and R² are methylcarbonyl and R³ to R⁸ are each hydrogen.

15. A dipeptide of the formula II 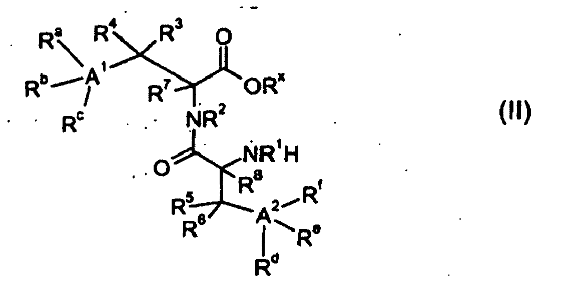 in which the variables A¹, A², R¹ - R⁸, R^{a}, R^{b}, R^{c}, R^{d}, R^{e} and R^{f} have the meaning given for formula I and in which OR^{x} is a leaving group attached via an oxygen atom.

16. A dipeptide of the formula VI 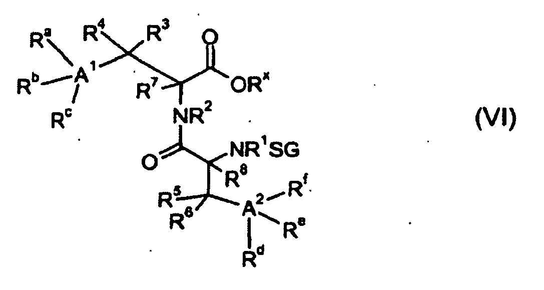 in which the variables A¹, A², R¹ - R⁸, R^{a}, R^{b}, R^{c}, R^{d}, R^{e} and R^{f} have the meaning given for the formula I and SG is a protective group and in which OR^{x} is a leaving group attached via an oxygen atom.

## Revendications

1. Utilisation de composés de type pipérazine de formule I 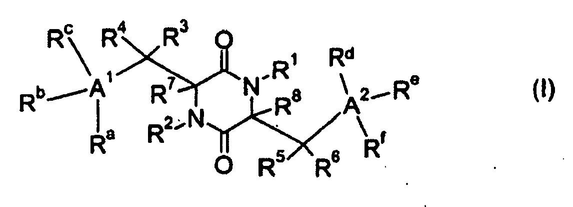 ou des sels utilisables en agriculture des composés de type pipérazine de formule I, en tant qu'herbicides, les variables dans la formule I ayant les significations suivantes :
R¹ et R² représentent, indépendamment l'un de l'autre,
un groupe cyano, alkyle en C₁-C₆, cycloalkyle en C₃-C₆, alcényle en C₃-C₆, cycloalcényle en C₃-C₆, alcynyle en C₃-C₆, cycloalcynyle en C₃-C₆, phényle, phényl-alkyle(C₁-C₆), hétérocyclyle, hétérocyclyl-alkyle(C₁-C₆) ; phényl-[alcoxy(C₁-C₆)-carbonyl]-alkyle(C₁-C₆) ou phénylhétérocyclyl-alkyle(C₁-C₆) ; ou
COR²¹, où
R²¹ représente un atome d'hydrogène, un groupe alkyle en C₁-C₆, cycloalkyle en C₃-C_{6,} alcényle en C₂-C₆, cycloalcényle en C₃-C₆, alcynyle en C₂-C₆, cycloalcynyle en C₃-C₆, hydroxy, alcoxy en C₁-C₆, alcényl(C₃-C₆)oxy, alcynyl(C₃-C₆)oxy, amino, alkyl(C₁-C₆)amino, [dialkyl(C₁-C₆)]amino, alcényl (C₃-C₆) amino, alcynyl (C₃-C₆) amino, alkyl-(C₁-C₆)sulfonylamino, N-[alcényl(C₂-C₆)]-N-[alkyl(C₁-C₆)]-amino, N-[alcynyl(C₂-C₆)]-N-[alkyl(C₁-C₆)]-amino, N-[alcoxy(C₁-C₆)]-N-[alkyl(C₁-C₆)]-amino, N-[alcényl(C₂-C₆)]-N-[alcoxy(C₁-C₆)]-amino, N-[alcynyl(C₂-C₆)]-N-[alcoxy(C₁-C₆)]-amino, phényle, phénylamino, phénoxy, naphtyle ou hétérocyclyle ; ou
NR²²R²³, où
R²² et R²³ représentent, indépendamment l'un de l'autre, un atome d'hydrogène, un groupe alkyle en C₁-C₆, cycloalkyle en C₃-C₆, alcényle en C₃-C₆, cycloalcényle en C₃-C₆, alcynyle en C₃-C₆, cycloalcynyle en C₃-C₆ ou alkyl(C₁-C₆)carbonyle; ou
OR²⁴, où
R²⁴ représente un groupe alkyle en C₁-C₆, cycloalkyle en C₃-C₆, alcényle en C₃-C₆, cycloalcényle en C₃-C_{6,} alcynyle en C₃-C_{6,} cycloalcynyle en C₃-C₆, phényle ou phénylalkyle(C₁-C₆) ; ou
SO₂R²⁵, où
R²⁵ représente un groupe alkyle en C₁-C₆ ou phényle ; les fragments aliphatiques, cycliques ou aromatiques nommés des substituants de R¹ et R² pouvant être partiellement ou totalement halogénés et/ou pouvant porter un à trois des groupes suivants : cyano, hydroxy, alkyle en C₁-C₄, halogénoalkyle en C₁-C₄, cycloalkyle en C₃-C₆, alcoxy en C₁-C₄, alkyl(C₁-C₄)thio, [dialkyl(C₁-C₄)] -amino, alkyl(C₁-C₄)carbonyle, hydroxycarbonyle, alcoxy(C₁-C₄) carbonyle, aminocarbonyle, alkyl(C₁-C₄)amino-carbonyle, [dialkyl(C₁-C₄)]amino-carbonyle ou alkyl(C₁-C₄)carbonyloxy;
et R¹ pouvant représenter en outre un atome d'hydrogène ;
R³ représente un radical R²⁶, OR²⁷, SR²⁸, NR²⁹R³⁰ ou N(OR³¹)R³², où
R²⁶, R²⁷, R²⁸, R²⁹ et R³² représentent, indépendamment les uns des autres, un atome d'hydrogène, un groupe alkyle en C₁-C₆, cycloalkyle en C₃-C₆, alcényle en C₃-C₆, alcynyle en C₃-C₆, halogénoalcynyle en C₃-C₆, formyle, alkyl (C₁-C₆)carbonyle, cycloalkyl(C₃-C₆)carbonyle, alcényl(C₂-C₆)carbonyle, alcynyl(C₂-C₆) carbonyle, alcoxy(C₁-C₆)alkyle(C₁-C₆), alcoxy(C₁-C₆)carbonyle, alcényl(C₂-C₆)oxycarbonyle, alcynyl(C₃-C₆)oxycarbonyle, alkyl(C₁-C₆)aminocarbonyle, alcényl(C₃-C₆)aminocarbonyle, alcynyl(C₃-C₆)aminocarbonyle, alkyl(C₁-C₆)sulfonylaminocarbonyle, alkyl(C₁-C₆)aminocarbonyle, [di-alkyl(C₁-C₆)amino] carbonyle, N-[alcényl(C₃-C₆)]-N-[alkyl(C₁-C₆)aminocarbonyle, N-[alcynyl(C₃-C₆)]-N-[alkyl(C₁-C₆)]aminocarbonyle, N-[alcoxy(C₁-C₆)]-N-[alkyl(C₁-C₆)]-aminocarbonyle, N-[alcényl(C₃-C₆)]-N-[alcoxy(C₁-C₆)]-aminocarbonyle, N-[alcynyl(C₃-C₆)]-N-[alcoxy(C₁-C₆)]aminocarbonyle, di-[alkyl(C₁-C₆)]aminothiocarbonyle, alkyl(C₁-C₆)carbonylalkyle(C₁-C₆), alcoxy(C₁-C₆)iminoalkyle(C₁-C₆), N-[alkyl(C₁-C₆)amino]-iminoalkyle(C₁-C₆), N-[di-alkyl(C₁-C₆)amino]-iminoalkyle(C₁-C₆) ou [tri-alkyl(C₁-C₄)]silyle, les fragments aliphatiques ou isocycliques nommés des substituants pouvant être partiellement ou totalement halogénés et/ou pouvant porter un à trois des groupes suivants : cyano, hydroxy, alkyle en C₁-C₄, halogénoalkyle en C₁-C₄, cycloalkyle en C₃-C₆, alcoxy en C₁-C₄, alkyl(C₁-C₄)thio, [dialkyl(C₁-C₄)]-amino, alkyl(C₁-C₄)carbonyle, hydroxycarbonyle, alcoxy(C₁-C₄) carbonyle, aminocarbonyle, alkyl(C₁-C₄)amino-carbonyle, [dialkyl(C₁-C₄)]amino-carbonyle ou alkyl(C₁-C₄)carbonyloxy ; ou
un groupe phényle, phénylalkyle(C₁-C₆), phénylcarbonyle, phénylcarbonylalkyle(C₁-C₆), phénoxycarbonyle, phénylaminocarbonyle, phénylsulfonylaminocarbonyle, N-[alkyl(C₁-C₆)]-N-(phényl)-aminocarbonyle, phénylalkyl(C₁-C₆)carbonyle, hétérocyclyle, hétérocyclylalkyle(C₁-C₆), hétérocyclylcarbonyle, hétérocyclylcarbonylalkyle(C₁-C₆), hétérocyclyloxycarbonyle, hétérocyclylaminocarbonyle, hétérocyclylsulfonylaminocarbonyle, N-[alkyl(C₁-C₆)]-N-(hétérocyclyl)-aminocarbonyle ou hétérocyclylalkyl(C₁-C₆)carbonyle, les fragments phényle ou hétérocyclyle des substituants pouvant être partiellement ou totalement halogénés et/ou porter un à trois des groupes suivants : nitro, cyano, alkyle en C₁-C₄, halogénoalkyle en C₁-C₄, alcoxy en C₁-C₄ ou halogénoalcoxy en C₁-C₄ ; ou
S(O)ₙR³³, où
n vaut 1 ou 2 ; et
R³³ représente un groupe alkyle en C₁-C₆, halogéno alkyle en C₁-C₆ ou phényle, et le substituant phényle pouvant être partiellement ou totalement halogéné et/ou pouvant porter un à trois des groupes suivants : nitro, cyano, alkyle en C₁-C₄, halogénoalkyle en C₁-C₄, alcoxy en C₁-C₄ ou halogénoalcoxy en C₁-C₄ ; et
R³⁰ et R³¹ représentent, indépendamment l'un de l'autre, un atome d'hydrogène, un groupe alkyle en C₁-C₆, cycloalkyle en C₃-C₆, alcényle en C₂-C₆ ou alcynyle en C₂-C₆, les fragments aliphatiques ou isocycliques des substituants pouvant être partiellement ou totalement halogénés et/ou pouvant porter un à trois des groupes suivants : cyano, hydroxy, alkyle en C₁-C_{4,} halogénoalkyle en C₁-C_{4,} cycloalkyle en C₃-C_{6,} alcoxy en C₁-C₄, alkyl(C₁-C₄)thio, [dialkyl(C₁-C₄)]-amino, alkyl(C₁-C₄)carbonyle, hydroxycarbonyle, alcoxy(C₁-C₄) carbonyle, aminocarbonyle, alkyl(C₁-C₄)amino-carbonyle, [dialkyl(C₁-C₄)]-amino-carbonyle ou alkyl(C₁-C₄)carbonyloxy,
phényle, phénylalkyle(C₁-C₆), hétérocyclyle ou hétérocyclylalkyle(C₁-C₆), les fragments phényle ou hétérocyclyle des substituants pouvant être partiellement ou totalement halogénés et/ou pouvant porter un à trois des groupes suivants : nitro, cyano, alkyle en C₁-C₄, halogénoalkyle en C₁-C₄, alcoxy en C₁-C₄ ou halogénoalcoxy en C₁-C₄ ;
R⁴, R⁵, R⁶ représentent, chacun indépendamment, un atome d'hydrogène, un groupe alkyle en C₁-C₆ ou alcoxy en C₁-C₆, les fragments aliphatiques nommés des substituants de R⁴, R⁵ ou R⁶ pouvant être partiellement ou totalement halogénés et/ou pouvant porter un à trois des groupes suivants : cyano, hydroxy, alkyle en C₁-C₄, halogénoalkyle en C₁-C₄, cycloalkyle en C₃-C₆, alcoxy en C₁-C₄, alkyl(C₁-C₄)thio, [dialkyl(C₁-C₄)]-amino, alkyl(C₁-C₄)carbonyle, hydroxycarbonyle, alcoxy(C₁-C₄) carbonyle, aminocarbonyle, alkyl(C₁-C₄)amino-carbonyle, [dialkyl(C₁-C₄)]amino-carbonyle ou alkyl (C₁-C₄) carbonyloxy ;
R³ et R⁴ peuvent également représenter conjointement un groupe céto ;
R⁷, R⁸ représentent, indépendamment l'un de l'autre, un atome d'hydrogène, un groupe hydroxy, alkyle en C₁-C₆, qui peut être partiellement ou totalement halogéné et/ou peut porter un à trois des groupes suivantes : cyano, hydroxy, alkyle en C₁-C₄, halogénoalkyle en C₁-C₄, cycloalkyle en C₃-C₆, alcoxy en C₁-C₄, alkyl(C₁-C₄)thio, [dialkyl(C₁-C₄)]-amino, alkyl(C₁-C₄)carbonyle, hydroxycarbonyle, alcoxy(C₁-C₄)carbonyle, aminocarbonyle, alkyl(C₁-C₄)amino-carbonyle, [dialkyl(C₁-C₄)]amino-carbonyle ou alkyl(C₁-C₄)carbonyloxy;
A¹, A² représentent, indépendamment l'un de l'autre, un groupe aryle ou hétéroaryle, à l'exception d'un groupe indolyle, R^{a} étant lié à un atome de carbone ou un atome d'azote de A¹ en position ortho par rapport au point de fixation de A¹ et R^{a} présentant l'une des significations indiquées ci-dessous :
R^{a} représente un atome d'halogène, un groupe cyano, nitro, alkyle en C₁-C₆, cycloalkyle en C₃-C₆, alcényle en C₂-C₆, cycloalcényle en C₃-C_{6,} alcadiényle en C₄-C₁₀, alcynyle en C₂-C₆, [trialkyl(C₁-C₆)silyl]-alcynyle(C₂-C₆), cycloalcynyle en C₃-C₆, alkyl(C₁-C₆)thio, alkyl(C₁-C₆)sulfinyle, aryle, phénylalkyle(C₁-C₆), phényl-alcényle(C₂-C₆), phénylsulfonyl-alkyle(C₁-C₆), hétérocyclyle, hétérocyclyl-alkyle(C₁-C₆) ou phényl-[alcoxy(C₁-C₆)carbonyl]-alkyle(C₁-C₆),
Z¹P(O)(OR⁹)₂, Z²B(OR¹⁰)₂,
R⁹ et R¹⁰ représentant chacun un atome d'hydrogène ou un groupe alkyle en C₁-C₆ et les radicaux R¹⁰ dans Z²B(OR¹⁰)₂ pouvant former ensemble une chaîne alkylène en C₂-C₄ ; ou
Z³COR¹¹,
R¹¹ représentant un atome d'hydrogène, un groupe alkyle en C₁-C₆, cycloalkyle en C₃-C₆, alcényle en C₂-C₆, cycloalcényle en C₃-C₆, alcynyle en C₂-C₆, cycloalcynyle en C₃-C_{6,} hydroxy, alcoxy en C₁-C₆, alcényl(C₃-C₆)oxy, alcynyl(C₃-C₆)oxy, amino, alkyl(C₁-C₆)amino, [dialkyl(C₁-C₆)]amino, alcoxy(C₁-C₆)amino, [dialcoxy(C₁-C₆)]amino, alkyl(C₁-C₆)-sulfonylamino, alkyl(C₁-C₆)aminosulfonylamino, [dialkyl(C₁-C₆)amino]-sulfonylamino, alcényl(C₃-C₆)amino, alcynyl(C₃-C₆)amino, N-[alcényl(C₂-C₆)]-N-[alkyl(C₁-C₆)]-amino, N-[alcynyl(C₂-C₆)]-N-[alkyl(C₁-C₆)]-amino, N-[alcoxy(C₁-C₆)]-N-[alkyl(C₁-C₆)]-amino, N-[alcényl-(C₂-C₆)]-N-[alcoxy(C₁-C₆)]-amino, N-[alcynyl(C₂-C₆)]-N-[alcoxy(C₁-C₆)]-amino, phényle, phénoxy, phénylamino, naphtyle ou hétérocyclyle ; ou
Z⁴NR¹²R¹³,
R¹² et R¹³ représentant, indépendamment l'un de l'autre, un atome d'hydrogène, un groupe alkyle en C₁-C₆, cycloalkyle en C₃-C₆, alcényle en C₃-C₆, cycloalcényle en C₃-C₆, alcynyle en C₃-C₆, cycloalcynyle en C₃-C₆, alkyl(C₁-C₆)carbonyle, cycloalkyl-(C₃-C₆) carbonyle, [dialkyl(C₁-C₆)amino]-carbonyle, alcoxy(C₁-C₆)carbonyle, alcoxy(C₁-C₆)carbonyl-alkyle(C₁-C₆), alkyl(C₁-C₆) sulfonyle, alkyl(C₁-C₆)aminosulfonyle, [dialkyl(C₁-C₆)amino]-sulfonyle, phénylcarbonyle, phénylaminocarbonyle, phénylsulfonyle, phénylsulfonylaminocarbonyle ou hétérocyclylcarbonyle ; ou
Z⁵CH=N-O-R¹⁴, R¹⁴ représentant un atome d'hydrogène ou un groupe alkyle en C₁-C₆ ; ou
Z⁶OR¹⁵,
R¹⁵ représentant un atome d'hydrogène, un groupe alkyle en C₁-C₆, cycloalkyle en C₃-C₆, alcényle en C₃-C₆, cycloalcényle en C₃-C₆, alcynyle en C₃-C₆, cycloalcynyle en C₃-C₆, alkyl (C₁-C₆) carbonyle, alcoxy(C₁-C₆)carbonyl-alkyle (C₁-C₆), [dialcoxy(C₁-C₆)carbonyl]-alkyle(C₁-C₆), phényle ou phényl-alkyle(C₁-C₆) ; ou
Z⁷SO₂R¹⁶, R¹⁶ représentant un groupe alkyle en C₁-C₆ ou phényle ; et
Z¹, Z², Z³, Z⁴, Z⁵, Z⁶, Z⁷ représentant, indépendamment les uns des autres, une liaison, -CH₂-, -CH₂-CH₂-, -O-CH(R¹⁷)-, -S-CH(R¹⁸)-, -S(O)-CH(R¹⁹)- ou -SO₂CH(R²⁰)-, et R¹⁷, R¹⁸, R¹⁹ et R²⁰ représentant, indépendamment les uns des autres, un atome d'hydrogène ou un groupe alkyle en C₁-C₆ ;
les fragments aliphatiques, cycliques ou aromatiques nommés du substituant R^{a} pouvant être partiellement ou totalement halogénés et/ou pouvant porter un à trois des groupes suivants : cyano, hydroxy, alkyle en C₁-C₄, halogénoalkyle en C₁-C_{4,} cycloalkyle en C₃-C₆, alcoxy en C₁-C₄, alkyl(C₁-C₄)thio, [dialkyl(C₁-C₄)-amino, alkyl(C₁-C₄)carbonyle, hydroxycarbonyle, alcoxy(C₁-C₄)carbonyle, aminocarbonyle, alkyl(C₁-C₄)amino-carbonyle, [dialkyl(C₁-C₄)]amino-carbonyle ou alkyl(C₁-C₄)carbonyloxy ; et
R^{b}, R^{c}, R^{d}, R^{e} et R^{f}, indépendamment les uns des autres, représentent chacun un atome d'hydrogène ou ont chacun l'une des significations indiquées pour R^{a} ; et
dans laquelle deux radicaux R^{a}, R^{b} ou R^{c} liés à des atomes contigus formant le cycle de A¹ ou deux radicaux R^{d}, R^{e} ou R^{f} liés à des atomes contigus formant le cycle de A² peuvent également représenter un groupe alkylène linéaire en C₃-C₆ qui peut être partiellement ou totalement halogéné et qui peut porter un à trois des groupes suivants : cyano, hydroxy, alkyle en C₁-C₄, halogénoalkyle en C₁-C₄, cycloalkyle en C₃-C₆, alcoxy en C₁-C₄, alkyl(C₁-C₄)thio, [dialkyl(C₁-C₄)]-amino, alkyl(C₁-C₄)carbonyle, hydroxycarbonyle, alcoxy(C₁-C₄)carbonyle, aminocarbonyle, alkyl(C₁-C₄)amino-carbonyle, [dialkyl(C₁-C₄)] aminocarbonyle ou alkyl(C₁-C₄)carbonyloxy, un groupe CH₂ dans le groupe alkylène en C₃-C₆ pouvant être remplacé par un groupe carbonyle, un groupe thiocarbonyle ou un groupe sulfonyle et un ou deux groupes CH₂ non contigus dans le groupe alkylène en C₃-C₆ pouvant être remplacés chacun par un atome d'oxygène ou de soufre ou par un groupe NR³⁴, R³⁴ ayant l'une des significations données pour R¹².

2. Utilisation des composés de type pipérazine de formule I selon la revendication 1, dans lesquels A¹ et A² sont choisis, indépendamment l'un de l'autre, dans l'ensemble constitué par les groupes phényle, naphtyle, furyle, thiényle, pyrrolyle, pyrazolyle, thiazolyle, isothiazolyle, oxazolyle, isoxazolyle, triazolyle, tétrazolyle, pyridinyle, pyridazinyle, pyrimidinyle, pyrazinyle, triazinyle et tétrazinyle.

3. Utilisation des composés de type pipérazine de formule I selon l'une quelconque des revendications précédentes, dans lesquels A¹ et A² sont choisis, indépendamment l'un de l'autre, dans l'ensemble constitué par les groupes phényle, furyle, thiényle, triazolyle, tétrazolyle et pyridinyle.

4. Utilisation des composés de type pipérazine de formule I selon l'une quelconque des revendications précédentes, dans lesquels A¹ représente le groupe phényle ou pyridinyle.

5. Utilisation des composés de type pipérazine de formule I selon l'une quelconque des revendications 1 à 4, dans lesquels A² représente le groupe phényle ou thiényle.

6. Utilisation des composés de type pipérazine de formule I selon l'une quelconque des revendications précédentes, dans lesquels
R^{a} est choisi parmi un atome d'halogène, un groupe cyano, nitro, alcényle en C₂-C₆, alcynyle en C₂-C₆, Z¹P(O)(OR⁹)₂, Z³COR¹¹, Z⁴NR¹²R¹³, Z⁵CH-N-O-R¹⁴, Z⁶OR¹⁵, Z⁷SO₂R¹⁶, alkyl(C₁-C₆)thio, alkyl(C₁-C₆)sulfinyle, aryle et hétérocyclyle,
Z¹ représentant une liaison ou CH₂ et R⁹ et R¹⁰ représentant chacun un atome d'hydrogène ou un groupe alkyle en C₁-C₆ ;
Z³ représentant une liaison et R¹¹ représentant un atome d'hydrogène, un groupe alkyle en C₁-C₆, hydroxy, alcoxy en C₁-C₆, alcényl(C₃-C₆)oxy, alcynyl(C₃-C₆)oxy, amino, alkyl(C₁-C₆)amino, [dialkyl(C₁-C₆)]amino, alcoxy(C₁-C₆)amino, N-alcoxy-(C₁-C₆)-N-alkyl(C₁-C₆)amino, alkyl(C₁-C₆)sulfonylamino, alkyl-(C₁-C₆)aminosulfonylamino, [dialkyl(C₁-C₆)-amino]sulfonylamino, phényle, phénoxy, phénylamino, naphtyle ou hétérocyclyle ; ou
Z⁴ représentant une liaison ou CH₂ et R¹² et R¹³ représentant, indépendamment l'un de l'autre, un atome d'hydrogène ou un groupe alkyle en C₁-C₆, cycloalkyle en C₃-C₆, alcényle en C₃-C₆, alcynyle en C₃-C₆, alkyl(C₁-C₆)carbonyle, [dialkyl(C₁-C₆)amino]carbonyle, alcoxy(C₁-C₆)carbonyle, alkyl(C₁-C₆)sulfonyle, phénylcarbonyle, phénylsulfonyle ou hétérocyclylcarbonyle ; ou
Z⁵ représentant une liaison ou CH₂ et R¹⁴ représentant un atome d'hydrogène ou un groupe alkyle en C₁-C₆ ; ou
Z⁶ représentant une liaison ou CH₂ et R¹⁵ représentant un atome d'hydrogène ou un groupe alkyle en C₁-C₆, cycloalkyle en C₃-C₆, alcényle en C₃-C₆, alcynyle en C₃-C₆, alkyl(C₁-C₆)carbonyle, alcoxy(C₁-C₆)carbonylalkyle(C₁-C₆), phényle ou phényl-alkyle(C₁-C₆); ou
Z⁷ représentant une liaison et R¹⁶ représentant un groupe alkyle en C₁-C₆ ou phényle ;
et
R^{b}, R^{c}, R^{d}, R^{e} et R^{f}, indépendamment les uns des autres, représentant un atome d'hydrogène, ayant l'une des significations indiquées pour R^{a} et pouvant en outre représenter :
un groupe alkyle en C₁-C₆, cycloalkyle en C₃-C₆, [tri-alkyl (C₁-C₆) silyl] alcynyle (C₂-C₆) ;
et les fragments aliphatiques, cycliques ou aromatiques nommés des substituants R^{a}, R^{b}, R^{c}, R^{d}, R^{e} et R^{f} pouvant être partiellement ou totalement halogénés.

7. Utilisation des composés de type pipérazine de formule I selon l'une quelconque des revendications précédentes, dans lesquels
R¹ représente un atome d'hydrogène ou un groupe alkyle en C₁-C₆ ;
R² représente un groupe alkyle en C₁-C₆ ;
les groupes alkyle en C₁-C₆ dans R¹ et R² pouvant être partiellement ou totalement halogénés.

8. Utilisation des composés de type pipérazine de formule I selon l'une quelconque des revendications précédentes, dans lesquels
R³ représente un groupe R²⁶ ou OR²⁷, où
R²⁶ et R²⁷ représentent, indépendamment l'un de l'autre, un atome d'hydrogène, un groupe alkyle en C₁-C₆, alkyl(C₁-C₆)carbonyle, phénylalkyle(C₁-C₆) ou phénylcarbonyle, les fragments aliphatiques ou aromatiques nommés des substituants pouvant être partiellement ou totalement halogénés ; ou un groupe SO₂R³¹, où
R³¹ représente un groupe alkyle en C₁-C₆ ou phényle, et le substituant phényle pouvant être partiellement ou totalement halogéné et/ou pouvant porter un à trois groupes alkyle en C₁-C₆.

9. Utilisation des composés de type pipérazine de formule I selon l'une quelconque des revendications précédentes, dans lesquels R⁴, R⁵, R⁶, R⁷ et R⁸ représentent un atome d'hydrogène.

10. Utilisation des composés de type pipérazine de formule I selon l'une quelconque des revendications précédentes, dans lesquels les deux centres de chiralité dans le cycle pipérazine présentent la configuration (S, S).

11. Composition contenant une quantité, efficace en tant qu'herbicide, d'au moins un composé pipérazine de formule I ou d'un sel de I utilisable en agriculture, selon l'une quelconque des revendications 1 à 10, et des adjuvants usuels pour la formulation de produits phytosanitaires.

12. Procédé de préparation de compositions selon la revendication 11, **caractérisé en ce qu'**une quantité à activité herbicide d'un composé de type pipérazine de formule I ou d'un sel de I utilisable en agriculture, selon l'une quelconque des revendications 1 à 10, et des adjuvants usuels pour la formulation de produits phytosanitaires sont mélangés.

13. Procédé pour la lutte contre la croissance de plantes indésirables, **caractérisé en ce qu'**on laisse agir sur des plantes, leurs semences et/ou leur habitat une quantité, efficace en tant qu'herbicide, d'au moins un composé de type pipérazine de formule I ou d'un sel de I utilisable en agriculture, selon l'une quelconque des revendications 1 à 10.

14. Composés de type pipérazine de formule générale I selon l'une quelconque des revendications précédentes,
à l'exclusion des composés de formule I dans lesquels A¹ et A² représentent un groupe phényle, R¹ représente un groupe méthyle, R² représente un atome d'hydrogène ou un groupe méthyle, R³, R⁴, R⁵, R⁶, R⁷ et R⁸ représentent un atome d'hydrogène, les substituants R^{a} et R^{b} représentent chacun un groupe hydroxy, et sont situés aux deux positions ortho du noyau phényle A¹ et R^{c}, R^{d}, R^{e} et R^{f} représentent un atome d'hydrogène, et en outre à l'exception des composés de formule I, dans lesquels A¹ représente un groupe phényle et A² représente un groupe 4-imidazolyle, ou A¹ représente un groupe 4-imidazolyle et A² représente un groupe phényle,
et en outre à l'exception des composés de formule I, dans lesquels A¹ représente un groupe phényle, R^{a} représente un groupe chloro, R^{b} et R^{c} représentent un atome d'hydrogène, le groupe A²(R^{d}R^{e}R^{f}) représente un groupe o-chlorophényle, R¹ et R² représentent un groupe méthylcarbonyle et R³ à R⁸ représentent chacun un atome d'hydrogène.

15. Dipeptides de formule II, 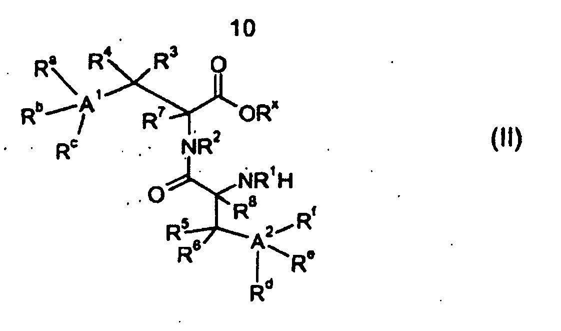 dans lesquels les variables A¹, A², R¹ - R⁸, R^{a}, R^{b}, R^{c}, R^{d}, R^{e} et R^{f} présentent la signification indiquée pour la formule I et dans lesquels OR^{x} représente un groupe partant lié par l'intermédiaire d'un atome d'oxygène.

16. Dipeptides de formule VI, 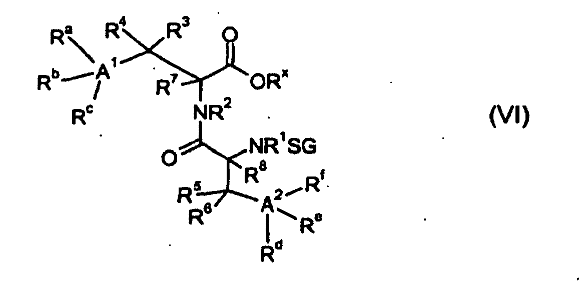 dans lesquels les variables A¹, A², R¹ - R⁸, R^{a}, R^{b}, R^{c}, R^{d}, R^{e} et R^{f} présentent la signification indiquée pour la formule I et SG représente un groupe protecteur et dans lesquels OR^{x} représente un groupe partant lié par l'intermédiaire d'un atome d'oxygène.
